(19) Europäisches Patentamt / European Patent Office / Office européen des brevets

(11) **EP 4 541 787 A1**

(12) **EUROPEAN PATENT APPLICATION**
published in accordance with Art. 153(4) EPC


(43) Date of publication:
**23.04.2025 Bulletin 2025/17**

(21) Application number: **23846695.7**

(22) Date of filing: **28.07.2023**

(51) International Patent Classification (IPC):
***C07C 269/06*** *(2006.01)* ***C07C 271/22*** *(2006.01)*
***C07D 263/04*** *(2006.01)* ***C07D 263/06*** *(2006.01)*
***C07D 263/16*** *(2006.01)*

(52) Cooperative Patent Classification (CPC):
**C07C 269/06; C07C 271/22; C07D 263/04; C07D 263/06; C07D 263/16**

(86) International application number:
**PCT/JP2023/027836**

(87) International publication number:
**WO 2024/024965 (01.02.2024 Gazette 2024/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.07.2022 JP 2022121484**



(71) Applicant: **CHUGAI SEIYAKU KABUSHIKI KAISHA**
**Tokyo 115-8543 (JP)**

(72) Inventor: **NOGI, Keisuke**
**Tokyo 115-8543 (JP)**

(74) Representative: **Vossius & Partner**
**Patentanwälte Rechtsanwälte mbB**
**Siebertstrasse 3**
**81675 München (DE)**


(54) **METHOD FOR PRODUCING O-SUBSTITUTED SERINE DERIVATIVE**


(57) An object is to provide a method for producing an O-substituted serine derivative, in which the O-substituted serine derivative can be obtained in a small number of steps. The present inventors have intensively studied the reductive hydrogenation of cyclic N,O-acetals and found a short-step method for producing an O-substituted serine derivative with excellent positional selectivity and chemical yield while maintaining the optical purity.


EP 4 541 787 A1

## Description

### Technical Field

The present invention relates to an O-substituted serine derivative useful as a pharmaceutical intermediate and a cyclic N,O-acetal useful for production thereof and methods for producing them.

### Background Art

In access to tough targets, such as the inhibition of protein-protein interactions, middle size molecules (having molecular weights of 500 to 2000) may be more superior to small molecules. Middle size molecules may also be superior to antibodies in that they can migrate into the cells. Among the biologically active middle size molecules, peptide pharmaceuticals are high valuable molecular species and 40 or more kinds of them are already on the market (Non Patent Literature 1). Representative examples of peptide pharmaceuticals include cyclosporine A and polymyxin B. From these structures, it can be seen that they are peptide compounds containing several non-natural amino acids. Non-natural amino acids are amino acids that are not naturally encoded on mRNA. It is very interesting that non-natural amino acids are included in the naturally-derived cyclosporine A and polymyxin B, and also that these non-natural structural sites interact with the sites of action in vivo to express pharmacological activities. Known examples of the non-natural amino acids interacting with a site of action in vivo include a study of the interaction of the O-substituted serine site of lacosamide with a sodium channel is known (Non Patent Literature 2).

Of the methods for producing O-substituted serine derivatives, the following methods are known as methods for producing O-alkyl-substituted serine derivatives.

1. A method for producing an O-substituted serine derivative from serine and alkyl halide in the presence of a base using the Williamson ether synthesis method, or an improved method thereof (Non Patent Literature 3).
2. A synthesis method for producing an O-substituted serine derivative from serine and trichloroacetimidate in the presence of an acid catalyst using Schmidt Glycosylation method (Non Patent Literature 4).
3. A synthesis method for producing an O-substituted serine derivative from serine and allyl carbonate in the presence of a palladium catalyst (Non Patent Literature 5).

These are methods of introducing an alkyl group directly to serine.

4. A synthesis method of reacting an aziridine compound derived from serine with an alcohol in the presence of a Lewis acid or a Brønsted acid catalyst (Patent Literatures 1 and 2).
5. A method of reacting a cyclic sulfamidate derived from serine with an alcohol (Patent Literature 3).

These are methods for producing an O-alkyl-substituted serine derivative via an intermediate derived from serine.

### Citation List

#### Patent Literature

Patent Literature 1: Japanese Unexamined Patent Publication No. S57-159747
Patent Literature 2: International Publication No. WO2010/053050
Patent Literature 3: International Publication No. WO2020/095983

#### Non Patent Literature

Non Patent Literature 1: Future Med. Chem., 2009, 1, 1289-1310
Non Patent Literature 2: J. Med. Chem., 2010, 53(15), 5716-5726
Non Patent Literature 3: Tetrahedron Letters, 2012, 53, 3225-3229
Non Patent Literature 4: Bioorganic & Medicinal Chemistry Letters, 1992, 2, 579-582
Non Patent Literature 5: Journal of the American Chemical Society, 2014, 136, 12469-12478

### Summary of Invention

## Technical Problem

The methods described in Patent Literatures 1, 2 and Non Patent Literature 2, which uses an aziridine derived from serine, have problems in positional selectivity of reaction site.
The method described in Non Patent Literature 3, which performed in the presence of a base, is known to have an elimination of a hydroxyl group of the serine, and is limited to the production of highly reactive benzyl ethers.
The method described in Non Patent Literature 4, which is a method for production from trichloroacetimidate, can be used only for the production of O-substituted serine derivatives whose substituent on oxygen is an allyl group.
The method described in Non Patent Literature 5, which is a method by a coupling reaction of allyl ether, can be used only for the production of O-substituted serine derivatives whose substituent on oxygen is an allyl group.
In Patent Literature 3, a method for producing O-substituted serine derivatives via sulfamidate is developed for attaining high positional selectivity, chemical yield and optical purity. The method includes an oxidation step using an oxidizing agent and requires 4 or 5 steps starting from a commercially available serine derivative.
An object of the present invention is to provide a method for producing an O-substituted serine derivative, in which the O-substituted serine derivative can be obtained in a small number of steps.

## Solution to Problem

The present inventors have intensively studied the reductive hydrogenation of cyclic N,O-acetals. As the results, the present inventors have found a short-step method for producing an O-substituted serine derivative with excellent positional selectivity and chemical yield while maintaining the optical purity, and have completed the present invention.
That is, the present invention includes the following.

[1] A method for producing a compound represented by the following formula (1) or a salt thereof or a solvate thereof, the method comprising the step of:

(A) reacting a compound represented by the following formula (2) with a reducing agent to obtain a compound represented by the following formula (1):

[Formula 1]

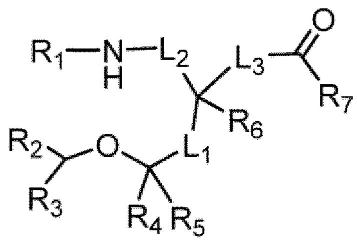

(1)

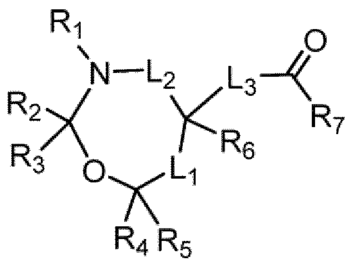

(2)

wherein

$R_1$ is an electron withdrawal group;
(i) $R_2$ and $R_3$ are each independently selected from the group consisting of hydrogen, $C_1$-$C_6$ alkyl optionally having a substituent, $C_3$-$C_6$ cycloalkyl optionally having a substituent, $C_2$-$C_6$ alkenyl optionally having a substituent, $C_2$-$C_6$ alkynyl optionally having a substituent, aralkyl optionally having a substituent, aryl optionally having a substituent, and heteroaryl optionally having a substituent, or (ii) $R_2$ and $R_3$ together with an intervening carbon atom form a 3- to 8-membered alicyclic ring or a 4- to 7-membered saturated heterocycle;
(i) $R_4$ and $R_5$ are each independently selected from the group consisting of hydrogen, $C_1$-$C_6$ alkyl optionally having a substituent, $C_3$-$C_6$ cycloalkyl optionally having a substituent, $C_2$-$C_6$ alkenyl optionally having a substituent, $C_2$-$C_6$ alkynyl optionally having a substituent, aralkyl optionally having a substituent, aryl optionally having a substituent, and heteroaryl optionally having a substituent, or (ii) $R_4$ and $R_5$ together with an intervening carbon atom form a 3- to 8-membered alicyclic ring or a 4- to 7-membered saturated heterocycle;
$R_6$ is hydrogen, $C_1$-$C_6$ alkyl optionally having a substituent, or aralkyl optionally having a substituent;

$R_7$ is $-OR_8$, $-NR_9R_{9'}$, an amino acid residue, or a peptide residue;
$R_8$, $R_9$ and $R_{9'}$ are each independently hydrogen, $C_1$-$C_6$ alkyl optionally having a substituent, or aralkyl optionally having a substituent, or $R_9$ and $R_{9'}$ together with an intervening nitrogen atom form a 4- to 7-membered saturated heterocycle;
$L_1$ is a single bond or $-CH_2-$;
$L_2$ is a single bond or $-CH_2-$;
$L_3$ is a single bond or $-CH_2-$;
provided that when $L_1$ is $-CH_2-$, $L_2$ is a single bond; when $L_2$ is $-CH_2-$, $L_1$ and $L_3$ each are a single bond; and when $L_3$ is $-CH_2-$, $L_2$ is single bond.

[1-1] A method for producing a compound represented by the following formula (1) or a salt thereof or a solvate thereof, the method comprising the step of:

(A) reacting a compound represented by the following formula (2) with a reducing agent to obtain a compound represented by the following formula (1):

[Formula 2]

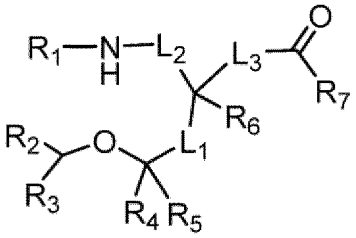

(1)

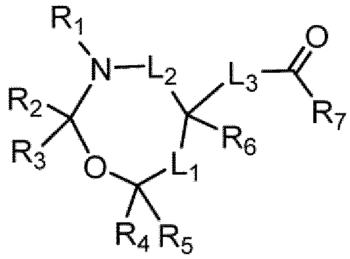

(2)

wherein

$R_1$ is an electron withdrawal group, an amino acid residue, or a peptide residue;
(i) $R_2$ and $R_3$ are each independently selected from the group consisting of hydrogen, $C_1$-$C_6$ alkyl optionally having a substituent, $C_3$-$C_6$ cycloalkyl optionally having a substituent, $C_2$-$C_6$ alkenyl optionally having a substituent, $C_2$-$C_6$ alkynyl optionally having a substituent, aralkyl optionally having a substituent, aryl optionally having a substituent, and heteroaryl optionally having a substituent, or (ii) $R_2$ and $R_3$ together with an intervening carbon atom form a 3- to 8-membered alicyclic ring or a 4- to 7-membered saturated heterocycle;
(i) $R_4$ and $R_5$ are each independently selected from the group consisting of hydrogen, $C_1$-$C_6$ alkyl optionally having a substituent, $C_3$-$C_6$ cycloalkyl optionally having a substituent, $C_2$-$C_6$ alkenyl optionally having a substituent, $C_2$-$C_6$ alkynyl optionally having a substituent, aralkyl optionally having a substituent, aryl optionally having a substituent, and heteroaryl optionally having a substituent, or (ii) $R_4$ and $R_5$ together with an intervening carbon atom form a 3- to 8-membered alicyclic ring or a 4- to 7-membered saturated heterocycle;
$R_6$ is hydrogen, $C_1$-$C_6$ alkyl optionally having a substituent, or aralkyl optionally having a substituent;
$R_7$ is $-OR_8$, $-NR_9R_{9'}$, an amino acid residue, or a peptide residue;
$R_8$, $R_9$ and $R_{9'}$ are each independently hydrogen, $C_1$-$C_6$ alkyl optionally having a substituent, or aralkyl optionally having a substituent, or $R_9$ and $R_{9'}$ together with an intervening nitrogen atom form a 4- to 7-membered saturated heterocycle;
$L_1$ is a single bond or $-CH_2-$;
$L_2$ is a single bond or $-CH_2-$;
$L_3$ is a single bond or $-CH_2-$;
provided that when $L_1$ is $-CH_2-$, $L_2$ is a single bond; when $L_2$ is $-CH_2-$, $L_1$ and $L_3$ each are a single bond; and when $L_3$ is $-CH_2-$, $L_2$ is single bond.

[2] The method according to [1], wherein the step (A) is performed in the presence of an acid.
[3] The method according to [2], wherein the acid is a metallic Lewis acid.
[4] The method according to [3], wherein the metallic Lewis acid is at least one selected from the group consisting of a

metal halide, a metal triflate, a metal alkoxide, and a metal halide alkoxide.

[5] The method according to [3] or [4], wherein metal in the metallic Lewis acid is at least one selected from the group consisting of titanium, tin, scandium, zirconium, zinc, and aluminum.

[6] The method according to [5], wherein metal in the metallic Lewis acid is at least one selected from the group consisting of titanium, tin, and scandium.

[7] The method according to [4], wherein the metallic Lewis acid is at least one selected from the group consisting of titanium tetrahalide, tin tetrahalide, scandium triflate, tetraalkoxytitanium, and alkoxytrichlorotitanium.

[8] The method according to [7], wherein the metallic Lewis acid is at least one selected from the group consisting of titanium tetrachloride, tin tetrachloride, scandium triflate, tetraisopropyl orthotitanate, and isopropoxytrichlorotitanium.

[9] The method according to [4], wherein the metallic Lewis acid is a combination of at least one selected from metal halides and at least one selected from metal alkoxides.

[10] The method according to [9], wherein the metallic Lewis acid is a combination of titanium tetrahalide and tetraalkoxytitanium.

[11] The method according to [10], wherein the metallic Lewis acid is a combination of titanium tetrachloride and tetraisopropyl orthotitanate.

[12] The method according to [11], wherein the metallic Lewis acid is a combination of titanium tetrachloride and tetraisopropyl orthotitanate at a molar ratio of 2 to 4 : 1.

[13] The method according to [11], wherein the metallic Lewis acid is a combination of titanium tetrachloride and tetraisopropyl orthotitanate at a molar ratio of 3 : 1.

[14] The method according to [4], wherein the metallic Lewis acid is at least one selected from the group consisting of titanium tetrahalide, tin tetrahalide, scandium triflate, and alkoxytrichlorotitanium.

[15] The method according to [14], wherein the metallic Lewis acid is one selected from the group consisting of titanium tetrachloride, tin tetrachloride, scandium triflate, and isopropoxytrichlorotitanium.

[16] The method according to [15], wherein the metallic Lewis acid is isopropoxytrichlorotitanium.

[17] The method according to any of [1] to [16], wherein the reducing agent used in the step (A) is a hydride-based reducing agent.

[18] The method according to [17], wherein the hydride-based reducing agent is at least one selected from the group consisting of a silane-based reducing agent and a borane-based reducing agent.

[19] The method according to [18], wherein the silane-based reducing agent is at least one selected from the group consisting of triethylsilane, triisopropylsilane, tristrimethylsilylsilane, phenylsilane, dimethylphenylsilane, tetraphenyldisilane, poly(methylhydrosiloxane), and 1,1,3,3-tetramethyldisiloxane.

[20] The method according to [19], wherein the silane-based reducing agent is at least one selected from the group consisting of triethylsilane, poly(methylhydrosiloxane), and 1,1,3,3-tetramethyldisiloxane.

[21] The method according to any of [2] to [20], wherein an amount of the acid used in the step (A) is 0.1 equivalents or more and 20 equivalents or less relative to the compound represented by formula (2), and an amount of the reducing agent used is 0.5 equivalents or more and 30 equivalents or less relative to the compound represented by formula (2).

[22] The method according to any of [2] to [20], wherein an amount of the acid used in the step (A) is 0.1 equivalents or more and 20 equivalents or less, 0.3 equivalents or more and 7 equivalents or less, or 0.5 equivalents or more and 5 equivalents or less, relative to the compound represented by formula (2).

[23] The method according to any of [2] to [20], wherein the amount of the acid used in the step (A) is 1 equivalent or more and 3 equivalents or less relative to the compound represented by formula (2).

[24] The method according to any of [1] to [20], wherein an amount of the reducing agent used in the step (A) is 0.5 equivalents or more and 30 equivalents or less, 1 equivalent or more and 20 equivalents or less, or 1.5 equivalents or more and 10 equivalents or less, relative to the compound represented by formula (2).

[25] The method according to any of [1] to [20], wherein the amount of the reducing agent used in the step (A) is 2 equivalents or more and 7 equivalents or less relative to the compound represented by formula (2).

[26] The method according to any of [1] to [25], wherein the step (A) is performed in the presence of a solvent, and the solvent is at least one selected from the group consisting of a halogenated solvent and a benzene-based solvent.

[27] The method according to [26], wherein the halogenated solvent is at least one selected from the group consisting of dichloromethane, 1,2-dichloroethane, and chloroform, and the benzene-based solvent is at least one selected from the group consisting of toluene, chlorobenzene, fluorobenzene, and benzotrifluoride.

[28] The method according to [26], wherein the solvent is at least one selected from the group consisting of dichloromethane, toluene, and chlorobenzene.

[29] The method according to any of [1] to [28], wherein the step (A) is performed further in the presence of a solubilizing agent.

[30] The method according to [29], wherein the solubilizing agent is at least one selected from the group consisting of a fluoroalcohol, an alcohol, a nitrile-type solvent, and an ester-type solvent.

[31] The method according to [30], wherein the fluoroalcohol is at least one selected from the group consisting of 2,2,2-trifluoroethanol and 1,1,1,3,3,3-hexafluoro-2-propanol, the alcohol is at least one selected from the group consisting of methanol, ethanol, and 2-propanol, the nitrile-type solvent is acetonitrile, and the ester-type solvent is at least one selected from the group consisting of ethyl acetate and dimethyl carbonate.
[32] The method according to [29], wherein the solubilizing agent is 2,2,2-trifluoroethanol or 1,1,1,3,3,3-hexafluoro-2-propanol.
[33] The method according to any of [1] to [32], wherein the step (A) is performed at a temperature of -50°C to 50°C for 5 minutes to 24 hours.
[34] The method according to any of [1] to [32], wherein the step (A) is performed at a temperature of -50°C to 50°C, -40°C to 40°C, or -30°C to 30°C.
[35] The method according to any of [1] to [32], wherein the step (A) is performed at a temperature of -20°C to 25°C.
[36] The method according to any of [1] to [32], wherein the step (A) is performed for 5 minutes to 24 hours, 10 minutes to 12 hours, or 20 minutes to 8 hours.
[37] The method according to any of [1] to [32], wherein the step (A) is performed for 30 minutes to 5 hours.
[38] The method according to any of [1] to [32], wherein the step (A) is performed at a temperature of -20°C to 25°C for 30 minutes to 5 hours.
[39] The method according to any of [1] to [38], comprising, after the step (A), a base treatment step of further adding a base to reduce a content of a compound (4) contained in a reaction mixture:

[Formula 3]

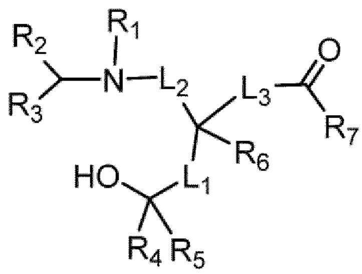

(4)

wherein $R_1$, $R_2$, $R_3$, $R_4$, $R_5$, $R_6$, $R_7$, $L_1$, $L_2$, and $L_3$ are as defined in [1].
[40] The method according to [39], wherein the base is at least one selected from the group consisting of sodium hydroxide, potassium hydroxide, lithium hydroxide, and tetraalkylammonium hydroxide.
[41] The method according to [40], wherein the base is sodium hydroxide or potassium hydroxide.
[42] The method according to any of [1] to [41], wherein the base treatment step is performed at a temperature of -5°C to 50°C, 0°C to 40°C, or 5°C to 30°C for 5 minutes to 24 hours, 10 minutes to 12 hours, or 20 minutes to 8 hours.
[43] The method according to any of [1] to [41], wherein the base treatment step is performed at a temperature of 10°C to 25°C.
[44] The method according to any of [1] to [41], wherein the base treatment step is performed for 30 minutes to 5 hours.
[45] The method according to any of [1] to [40], wherein the base treatment step is performed at a temperature of 10°C to 25°C for 30 minutes to 5 hours using sodium hydroxide or potassium hydroxide.
[46] The method according to any of [1] to [45], further comprising, prior to the step (A), the step of:
(B) reacting a compound represented by the following formula (3) with an aldehyde, a ketone, an acetal, or a vinyl ether to obtain a compound represented by the following formula (2):

[Formula 4]

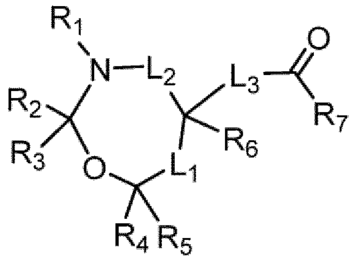

(2)

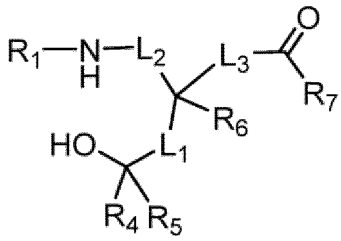

(3)

wherein $R_1$, $R_2$, $R_3$, $R_4$, $R_5$, $R_6$, $R_7$, $L_1$, $L_2$, and $L_3$ are as defined in [1].

[47] The method according to [46], wherein the step (B) is a step of reacting with an aldehyde or a ketone in the presence of an acid to obtain a compound represented by formula (2).

[48] The method according to [47], wherein the acid is a Lewis acid.

[49] The method according to [48], wherein the Lewis acid is at least one selected from the group consisting of a complex of boron trifluoride with an ether-based solvent, and a trialkylsilyl triflate.

[50] The method according to [49], wherein the Lewis acid is at least one selected from the group consisting of a boron trifluoride-tetrahydrofuran complex, a boron trifluoride-diethyl ether complex, and trimethylsilyl triflate.

[51] The method according to any of [46] to [50], wherein the step (B) is performed further in the presence of a silylating agent.

[52] The method according to [51], wherein the silylating agent is at least one selected from the group consisting of N,O-bis(trimethylsilyl)trifluoroacetamide, N,O-bis(trimethylsilyl)acetamide, and 1-trimethylsilylimidazole.

[53] The method according to [52], wherein the silylating agent is N,O-bis(trimethylsilyl)trifluoroacetamide.

[54] The method according to any of [46] to [53], wherein the step (B) is performed at a temperature of -10°C to 80°C, 0°C to 70°C, or 10°C to 60°C for 5 minutes to 24 hours, 10 minutes to 12 hours, or 20 minutes to 6 hours.

[55] The method according to any of [46] to [53], wherein the step (B) is performed at a temperature of 20°C to 50°C.

[56] The method according to any of [46] to [53], wherein the step (B) is performed for 30 minutes to 3 hours.

[57] The method according to any of [46] to [53], wherein the step (B) is performed at a temperature of 20°C to 50°C for 30 minutes to 3 hours.

[58] The method according to any of [1] to [57], wherein $R_7$ is $-OR_8$; and $R_8$ is hydrogen, $C_1$-$C_6$ alkyl optionally having a substituent, or aralkyl optionally having a substituent.

[59] The method according to [58], wherein $R_7$ is -OH.

[59-1] The method according to [58], wherein $R_7$ is $-OR_8$; and $R_8$ is $C_1$-$C_6$ alkyl.

[59-2] The method according to [58], wherein $R_7$ is $-OR_8$; and $R_8$ is methyl.

[60] The method according to any of [1] to [57], wherein $R_7$ is $-NR_9R_{9'}$; and $R_9$ and $R_{9'}$ are each independently hydrogen, $C_1$-$C_6$ alkyl optionally having a substituent, or aralkyl optionally having a substituent, or $R_9$ and $R_{9'}$ together with an intervening nitrogen atom form a 4- to 7-membered saturated heterocycle.

[61] The method according to any of [1] to [57], wherein $R_7$ is an amino acid residue or a peptide residue.

[61-1] The method according to any of [1] to [57], wherein $R_7$ is an amino acid residue whose C-terminus is protected with a $C_1$-$C_6$ alkyl ester.

[62] A method for producing a compound represented by the following formula (16) or a salt thereof or a solvate thereof, the method comprising the step of:

producing, by the method according to any of [1] to [61], the following compound (11):

[Formula 5]

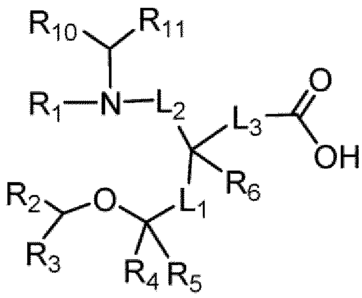

(16)

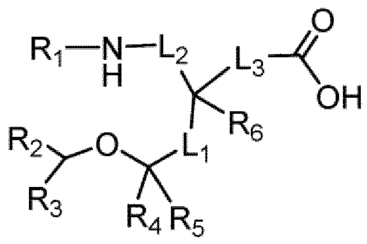

(11)

wherein $R_1$, $R_2$, $R_3$, $R_4$, $R_5$, $R_6$, $L_1$, $L_2$, and $L_3$ have the same meanings as those defined in [1], and (i) $R_{10}$ and $R_{11}$ are each independently selected from the group consisting of hydrogen, $C_1$-$C_6$ alkyl optionally having a substituent, $C_3$-$C_6$ cycloalkyl optionally having a substituent, $C_2$-$C_6$ alkenyl optionally having a substituent, $C_2$-$C_6$ alkynyl optionally having a substituent, aralkyl optionally having a substituent, aryl optionally having a substituent, and heteroaryl optionally having a substituent, or (ii) $R_{10}$ and $R_{11}$ together with an intervening carbon atom form a 3- to 8-membered alicyclic ring or a 4- to 7-membered saturated heterocycle.

[63] The method according to [62], further comprising, after the step of producing the following compound (11), the steps of:

(C) reacting the compound represented by the following formula (11) with an aldehyde, a ketone, an acetal, or a vinyl ether in the presence of a Lewis acid to obtain a compound represented by the following formula (15); and
(D) reacting the compound represented by the following formula (15) in the presence of a Lewis acid with a reducing agent to obtain a compound represented by the following formula (16):

[Formula 6]

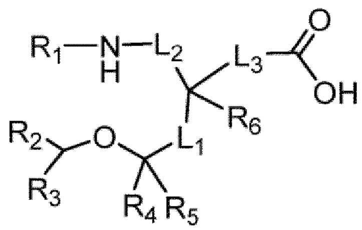

(11)

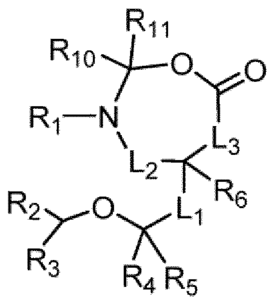

(15)

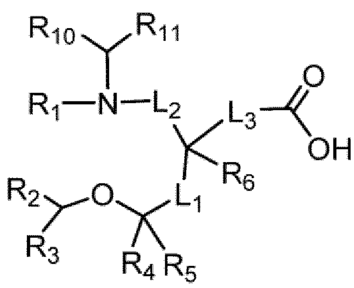

(16)

wherein $R_1$, $R_2$, $R_3$, $R_4$, $R_5$, $R_6$, $R_{10}$, $R_{11}$, $L_1$, $L_2$, and $L_3$ are as defined in [1] and [62].

[64] The method according to [63], wherein the Lewis acid in the step (C) is at least one selected from the group consisting of a complex of boron trifluoride with an ether-based solvent, a trialkylsilyl triflate, titanium tetrachloride, and a tetraalkoxytitanium.
[65] The method according to [64], wherein the Lewis acid in the step (C) is a boron trifluoride-diethyl ether complex or a boron trifluoride-tetrahydrofuran complex.
[66] The method according to any of [63] to [65], wherein the Lewis acid in the step (D) is at least one selected from the group consisting of a boron trifluoride-alkyl ether complex, a trialkylsilyl triflate, titanium tetrachloride, and a tetraalkoxytitanium.
[67] The method according to [66], wherein the Lewis acid in the step (D) is trimethylsilyl triflate.
[68] The method according to any of [63] to [67], wherein the reducing agent in the step (D) is a hydride-based reducing agent.
[69] The method according to [68], wherein the hydride-based reducing agent is at least one selected from the group

consisting of a silane-based reducing agent and a borane-based reducing agent.

[70] The method according to [69], wherein the silane-based reducing agent is at least one selected from the group consisting of triethylsilane, triisopropylsilane, tristrimethylsilylsilane, phenylsilane, dimethylphenylsilane, tetraphenyldisilane, poly(methylhydrosiloxane), and 1,1,3,3-tetramethyldisiloxane.

[71] The method according to [69], wherein the silane-based reducing agent is at least one selected from the group consisting of triethylsilane, poly(methylhydrosiloxane), and 1,1,3,3-tetramethyldisiloxane.

[72] The method according to any of [1] to [71], wherein

$R_1$ is -C(=O)-$R_{12}$, -C(=O)-$OR_{13}$, -S(=O)$_2$-$R_{14}$, -S(=O)$_2$-$OR_{15}$, -P(=O)-$R_{16}R_{17}$, or -P(=O)-$(OR_{18})_2$, wherein $R_{12}$, $R_{13}$, $R_{14}$, $R_{15}$, $R_{16}$, $R_{17}$, and $R_{18}$ each are $C_1$-$C_6$ alkyl optionally having a substituent, aralkyl optionally having a substituent, aryl optionally having a substituent, or heteroaryl optionally having a substituent;

$R_2$ and $R_3$ are each independently selected from the group consisting of hydrogen, methyl, ethyl, n-propyl, isopropyl, n-butyl, tert-butyl, isobutyl, isopentyl, benzyl, phenyl, and pyridyl, or $R_2$ and $R_3$ together with a carbon atom to which they are attached form a 3- to 8-membered alicyclic ring;

$R_4$ and $R_5$ are each independently selected from the group consisting of hydrogen, methyl, ethyl, n-propyl, n-butyl, tert-butyl, isobutyl, isopentyl, benzyl, phenyl, and pyridyl, or $R_4$ and $R_5$ together with a carbon atom to which they are attached form a 3- to 8-membered alicyclic ring; $R_6$ is hydrogen, methyl, ethyl, n-propyl, n-butyl, or benzyl; and

$L_1$, $L_2$, and $L_3$ each are a single bond.

[73] The method according to any of [1] to [72], wherein

$R_1$ is -C(=O)-$R_{12}$, -C(=O)-$OR_{13}$, -S(=O)$_2$-$R_{14}$, -S(=O)$_2$-$OR_{15}$, -P(=O)-$R_{16}R_{17}$, or -P(=O)-$(OR_{18})_2$, wherein $R_{12}$, $R_{13}$, $R_{14}$, $R_{15}$, $R_{16}$, $R_{17}$, and $R_{18}$ each are $C_1$-$C_6$ alkyl optionally having a substituent, aralkyl optionally having a substituent, aryl optionally having a substituent, or heteroaryl optionally having a substituent.

[74] The method according to [73], wherein

$R_1$ is -C(=O)-$R_{12}$, -C(=O)-$OR_{13}$, or -S(=O)$_2$-$R_{14}$, wherein $R_{12}$, $R_{13}$, and $R_{14}$ each are $C_1$-$C_6$ alkyl optionally having a substituent, aralkyl optionally having a substituent, aryl optionally having a substituent, or heteroaryl optionally having a substituent.

[75] The method according to [74], wherein $R_1$ is benzyloxycarbonyl (Cbz), 9-fluorenylmethyloxycarbonyl (Fmoc), acetyl, trifluoroacetyl, methanesulfonyl, paratoluene sulfonyl, (trifluoromethyl)sulfonyl, or 2-nitrobenzenesulfonyl (Nosyl).

[76] The method according to [75], wherein $R_1$ is benzyloxycarbonyl (Cbz) or 9-fluorenylmethyloxycarbonyl (Fmoc).

[76-1] The method according to any of [1] to [71], wherein $R_1$ is an amino acid residue, or a peptide residue;

$R_2$ and $R_3$ are each independently selected from the group consisting of hydrogen, methyl, ethyl, n-propyl, isopropyl, n-butyl, tert-butyl, isobutyl, isopentyl, benzyl, phenyl, and pyridyl, or $R_2$ and $R_3$ together with a carbon atom to which they are attached form a 3- to 8-membered alicyclic ring;

$R_4$ and $R_5$ are each independently selected from the group consisting of hydrogen, methyl, ethyl, n-propyl, n-butyl, tert-butyl, isobutyl, isopentyl, benzyl, phenyl, and pyridyl, or $R_4$ and $R_5$ together with a carbon atom to which they are attached form a 3- to 8-membered alicyclic ring; $R_6$ is hydrogen, methyl, ethyl, n-propyl, n-butyl, or benzyl; and

$L_1$, $L_2$, and $L_3$ each are a single bond.

[76-2] The method according to [76-1], wherein $R_1$ is an amino acid residue or a peptide residue whose N-terminus is protected with benzyloxycarbonyl (Cbz) or 9-fluorenylmethyloxycarbonyl (Fmoc).

[76-3] The method according to [76-1], wherein $R_1$ is an amino acid residue whose N-terminus is protected with benzyloxycarbonyl (Cbz) or 9-fluorenylmethyloxycarbonyl (Fmoc).

[77] The method according to any of [1] to [76], wherein $R_2$ and $R_3$ are each independently selected from the group consisting of hydrogen, methyl, ethyl, n-propyl, isopropyl, n-butyl, tert-butyl, isobutyl, isopentyl, benzyl, phenyl, and pyridyl, or $R_2$ and $R_3$ together with a carbon atom to which they are attached form a 3- to 8-membered alicyclic ring.

[78] The method according to [77], wherein $R_2$ is methyl, ethyl, n-propyl, n-butyl, or isobutyl and $R_3$ is hydrogen or methyl, or $R_2$ and $R_3$ together with a carbon atom to which they are attached form a 4- to 6-membered alicyclic ring.

[79] The method according to any of [1] to [78], wherein $R_4$ and $R_5$ are each independently selected from the group consisting of hydrogen, methyl, ethyl, n-propyl, n-butyl, tert-butyl, isobutyl, isopentyl, benzyl, phenyl, and pyridyl, or $R_4$ and $R_5$ together with a carbon atom to which they are attached form a 3- to 8-membered alicyclic ring.

[80] The method according to [79], wherein $R_4$ is hydrogen, methyl, ethyl, n-propyl, n-butyl, or isobutyl, and $R_5$ is hydrogen.

[81] The method according to [80], wherein $R_4$ is hydrogen or methyl, and $R_5$ is hydrogen.

[82] The method according to any of [1] to [81], wherein $R_6$ is hydrogen, methyl, ethyl, n-propyl, n-butyl, or benzyl.
[83] The method according to [82], wherein $R_6$ is hydrogen.
[84] The method according to any of [1] to [83], wherein $L_1$, $L_2$, and $L_3$ each are a single bond.
[85] A method for producing a peptide, comprising the method according to any of [1] to [84].
[86] A method for producing a peptide compound, the method comprising the steps of:

(i) producing, by the method according to any of [1] to [85], the compound (11) or (16) above; and

(ii) condensing a carboxy group of the compound (11) or (16) obtained in the step (i) above with an amino acid having an amino group or a peptide having an amino group in a solvent.

[87] A compound selected from the group consisting of:

2-(benzyloxycarbonylamino)-3-(cyclobutoxy)propanoic acid;
2-(benzyloxycarbonylamino)-3-isopentyloxy-propanoic acid;
2-(benzyloxycarbonylamino)-3-isopropoxy-butanoic acid;
2-[benzyloxycarbonyl(methyl)amino]-3-(cyclobutoxy)propanoic acid;
3-benzyloxycarbonyl-2-ethyl-oxazolidine-4-carboxylic acid;
3-benzyloxycarbonyl-2-isobutyl-oxazolidine-4-carboxylic acid;
benzyl 4-(cyclobutoxymethyl)-5-oxo-oxazolidine-3-carboxylate;
benzyl 4-{[1-methoxy-1-oxapropan-2-yl]carbamoyl}-2,2,5-trimethyloxazolidine-3-carboxylate; N-(benzyloxy)carbonyl-O-isopropyl-threonyl-alanine methyl ester; and
N-[(9H-fluoren-9-ylmethoxycarbonyl)-leucyl]-O-isopropyl-threonine
or a salt thereof or a solvate thereof.

[88] (2S)-2-(Benzyloxycarbonylamino)-3-(cyclobutoxy)propanoic acid or a salt thereof or a solvate thereof.
[89] (2S)-2-(Benzyloxycarbonylamino)-3-isopentyloxy-propanoic acid or a salt thereof or a solvate thereof.
[90] (2S,3R)-2-(Benzyloxycarbonylamino)-3-isopropoxy-butanoic acid or a salt thereof or a solvate thereof.
[91] (2S)-2-[Benzyloxycarbonyl(methyl)amino]-3-(cyclobutoxy)propanoic acid or a salt thereof or a solvate thereof.
[92] (4S)-3-Benzyloxycarbonyl-2-ethyl-oxazolidine-4-carboxylic acid or a salt thereof or a solvate thereof.
[93] (4S)-3-Benzyloxycarbonyl-2-isobutyl-oxazolidine-4-carboxylic acid or a salt thereof or a solvate thereof.
[94] Benzyl (4S)-4-(cyclobutoxymethyl)-5-oxo-oxazolidine-3-carboxylate or a salt thereof or a solvate thereof.
[95] Benzyl (4S,5R)-4-{[(S)-1-methoxy-1-oxapropan-2-yl]carbamoyl}-2,2,5-trimethyloxazolidine-3-carboxylate or a salt thereof or a solvate thereof.
[96] N-(Benzyloxy)carbonyl-O-isopropyl-L-threonyl-L-alanine methyl ester or a salt thereof or a solvate thereof.
[97] N-[(9H-Fluoren-9-ylmethoxycarbonyl)-L-leucyl]-O-isopropyl-L-threonine or a salt thereof or a solvate thereof.

In the above numberings, the number cited in the dependent item includes the branch number of the number, unless otherwise mentioned. For example, the [1] cited in the dependent item shows that not only [1] but also its branch number [1-1] is included. The same applies to other numberings.

**Advantageous Effects of Invention**

According to the present invention, a method for producing an O-substituted serine derivative, in which the O-substituted serine derivative can be obtained in a small number of steps, can be provided.

**Description of Embodiments**

(Abbreviations)

Examples and meanings of the abbreviations as used herein are listed below.

MTBE: tert-butyl methyl ether
HFIP: 1,1,1,3,3,3-hexafluoro-2-propanol
DCHA: dicyclohexylamine
MeTHF: 2-methyltetrahydrofuran
CPME: cyclopentyl methyl ether
PMHS: poly(methylhydrosiloxane)

TMDS: 1,1,3,3-tetramethyldisiloxane

(Terminology)

The term "room temperature" as used herein means a temperature of about 20°C to about 25°C.

The term "electron withdrawal group" as used herein is a substituent that is prone to attract an electron from the atomic side to which it is attached compared to a hydrogen atom. Examples of the electron withdrawal group include groups represented by $-C(=O)-R_{12}$, $-C(=O)-OR_{13}$, $-S(=O)_2-R_{14}$, $-S(=O)_2-OR_{15}$, $-P(=O)-R_{16}R_{17}$, or $-P(=O)-(OR_{18})_2$, in which $R_{12}$, $R_{13}$, $R_{14}$, $R_{15}$, $R_{16}$, $R_{17}$, and $R_{18}$ each are $C_1$-$C_6$ alkyl optionally having a substituent, aralkyl optionally having a substituent, aryl optionally having a substituent, or heteroaryl optionally having a substituent.

Examples the "halogen atom" as used herein include F, Cl, Br and I.

The term "alkyl" as used herein is a monovalent group derived from an aliphatic hydrocarbon by removing any one hydrogen atom, and has a subset of hydrocarbyl or hydrocarbon group structures that do not contain a hetero atom (which is an atom other than carbon and hydrogen atoms) or an unsaturated carbon-carbon bond, and contain hydrogen and carbon atoms in the backbone. The alkyl includes not only a linear form but also a branched form. Preferred examples of the alkyl include alkyl having 1 to 20 carbon atoms ($C_1$-$C_{20}$; hereinafter, "$C_p$-$C_q$" means that the number of carbon atoms is p to q), preferably $C_1$-$C_{10}$ alkyl, and more preferably $C_1$-$C_6$ alkyl. Specific examples of the alkyl include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

The term "alkenyl" as used herein is a monovalent group having at least one double bond (two adjacent $SP^2$ carbon atoms). Depending on the conformation of the double bond and a substituent (if present), the geometric morphology of the double bond can assume entgegen (E) or zusammen (Z) and cis or trans conformations. The alkenyl includes not only a linear form but a branched form. Preferred examples of the alkenyl include $C_2$-$C_{10}$ alkenyl, and more preferably $C_2$-$C_6$ alkenyl. Specific examples thereof include vinyl, allyl, 1-propenyl, 1-butenyl, 2-butenyl (including cis and trans), 3-butenyl, pentenyl, 3-methyl-2-butenyl, and hexenyl.

The term "alkynyl" as used herein is a monovalent group having at least one triple bond (two adjacent SP carbon atoms). The alkynyl includes not only a linear form but a branched form. Preferred examples of the alkynyl include $C_2$-$C_{10}$ alkynyl, and more preferably $C_2$-$C_6$ alkynyl. Specific examples thereof include ethynyl, 1-propynyl, propargyl, 3-butynyl, pentynyl, hexynyl, 3-phenyl-2-propynyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

The term "cycloalkyl" as used herein means a saturated or partially saturated cyclic monovalent aliphatic hydrocarbon group and includes a monocyclic ring, a bicyclo ring, and a spiro ring. Preferred examples of the cycloalkyl include $C_3$-$C_8$ cycloalkyl. Specific examples thereof include cyclopropyl, cyclobutyl, cyclopentyl, cyclohexyl, cycloheptyl, cyclooctyl, bicyclo[2.2.1]heptyl, and spiro[3.3]heptyl.

The term "aryl" as used herein means a monovalent aromatic hydrocarbon ring and an aromatic hydrocarbon ring group. Preferred examples of the aryl include $C_6$-$C_{10}$ aryl. Specific examples of the aryl include phenyl and naphthyl (for example, 1-naphthyl and 2-naphthyl).

The term "heteroaryl" as used herein means an aromatic cyclic monovalent group containing 1 to 5 heteroatoms in addition to a carbon atom, and an aromatic heterocyclic group. The ring may be a monocyclic ring or a condensed ring with another ring, and may be partially saturated. The number of atoms constituting the ring of heteroaryl is preferably 5 to 10 (5- to 10-membered heteroaryl), and more preferably 5 to 7 (5- to 7-membered heteroaryl). Specific examples of the heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzimidazolyl, benzotriazolyl, indolyl, isoindolyl, indazolyl, azaindolyl, quinolyl, isoquinolyl, cinnolinyl, quinazolinyl, quinoxalinyl, benzodioxolyl, indolizinyl, imidazopyridyl, pyrazolopyridyl, imidazopyridyl, triazolopyridyl, pyrrolopyrazinyl, and flopyridyl.

The term "aralkyl (arylalkyl)" as used herein means a group in which at least one hydrogen atom of the "alkyl" as defined above is replaced with the "aryl" as defined above. As the aralkyl, $C_7$-$C_{14}$ aralkyl is preferred, and $C_7$-$C_{10}$ aralkyl is more preferred. Specific examples of the aralkyl include benzyl, phenethyl, and 3-phenylpropyl.

The term "alicyclic ring" as used herein means a nonaromatic hydrocarbon ring. The alicyclic ring may have an unsaturated bond in the ring and may be a polycyclic ring having two or more rings. A carbon atom constituting the ring may form carbonyl through oxidation. The alicyclic ring is preferably a 3- to 8-membered alicyclic ring. Specific examples of the alicyclic ring include a cyclopropane ring, a cyclobutane ring, a cyclopentane ring, a cyclohexane ring, a cycloheptane ring, a cyclooctane ring, and a bicyclo[2.2.1]heptane ring.

The term "saturated heterocycle" as used herein means a nonaromatic heterocycle containing 1 to 5 heteroatoms in addition to a carbon atom, without containing a double bond and/or a triple bond in the ring. The saturated

heterocycle may be a monocyclic ring or may form a condensed ring with another ring, for example, an aromatic ring such as a benzene ring. Preferred examples of the saturated heterocycle include a 4- to 10-membered saturated heterocycle. Specific examples of the saturated heterocycle include an azetidine ring, an oxoazetidine ring, an oxetane ring, a tetrahydrofuran ring, a tetrahydropyran ring, a morpholine ring, a thiomorpholine ring, a pyrrolidine ring, a 2-oxopyrrolidine ring, a 4-oxopyrrolidine ring, a piperidine ring, a 4-oxopiperidine ring, a piperazine ring, a pyrazolidine ring, an imidazolidine ring, an oxazolidine ring, an isoxazolidine ring, a thiazolidine ring, an isothiazolidine ring, a thiadiazolidine ring, an oxazolidone ring, a dioxolane ring, a dioxane ring, a thietane ring, an octahydroindole ring, an indoline ring, an azepane ring, a dioxepane ring, and a 5,9-dioxaspiro[3.5]nonane ring.

The compound described herein can be a salt thereof or a solvate thereof. Examples of the salt of the compound include hydrochloride; hydrobromide; hydroiodide; phosphate; phosphonate; sulfate; sulfonate such as methanesulfonate and p-toluenesulfonate; carboxylate such as acetate, citrate, malate, tartrate, succinate, and salicylate; alkali metal salts such as sodium salt and potassium salt; alkaline earth metal salts such as magnesium salt and calcium salt; and ammonium salts such as ammonium salt, alkylammonium salt, dialkylammonium salt, trialkylammonium salt, and tetraalkylammonium salt. These salts are produced by, for example, bringing the compound into contact with an acid or a base. The "solvate" as used herein refers to a molecular population that is formed by a compound together with a solvent. Examples of the solvate include a hydrate, an alcohol solvate (such as ethanol solvate, methanol solvate, 1-propanol solvate, or 2-propanol solvate), and not only solvates formed with a single solvent such as dimethyl sulfoxide, but also solvates formed with a plurality of solvents per one molecule of the compound, or solvates formed with a plurality of types of solvents per one molecule of the compound. When the solvent is water, the solvate is called a hydrate. The solvate of the compound of the present invention is preferably a hydrate. Specific examples of such a hydrate include a mono- to deca-hydrate, preferably a mono- to penta-hydrate, further preferably a mono- to tri-hydrate.

The "amino acid" as used herein includes a natural amino acid and a non-natural amino acid (sometimes also referred to as an amino acid derivative). The "amino acid" as used herein may mean an amino acid residue. The term "natural amino acid" as used herein refers to Gly, Ala, Ser, Thr, Val, Leu, Ile, Phe, Tyr, Trp, His, Glu, Asp, Gln, Asn, Cys, Met, Lys, Arg, or Pro. The non-natural amino acids (amino acid derivatives) are not particularly limited, and examples thereof include a β-amino acid, a D-type amino acid, an N-substituted amino acid, an α,α-disubstituted amino acid, an amino acid having a side chain different from that of natural amino acids, and a hydroxycarboxylic acid. As the amino acids as used herein, amino acids having any conformation are acceptable. The selection of a side chain of the amino acid is not particularly restricted, and the side chain is freely selected from, in addition to a hydrogen atom, an alkyl group, an alkenyl group, an alkynyl group, an aryl group, a heteroaryl group, an aralkyl group, a heteroaralkyl group, a cycloalkyl group, and a spiro-bonded cycloalkyl group. Each of the side chains may have a substituent. The substituent is also not limited, and one or two or more substituents may be freely selected independently from any substituents including, for example, a halogen atom, an O atom, an S atom, an N atom, a B atom, a Si atom, or a P atom. That is, examples of the side chain include an alkyl group, an alkoxy group, an alkoxyalkyl group, an alkenyl group, an alkynyl group, an aryl group, a heteroaryl group, an aralkyl group, or a cycloalkyl group which may be substituted, or oxo, aminocarbonyl, and a halogen atom. In one non-limiting aspect, an amino acid herein may be a compound having a carboxyl group and an amino group within the same molecule, and specific examples thereof include 4-aminobutanoic acid, 5-aminopentanoic acid, 6-aminohexanoic acid, 4-piperidine carboxylic acid, and 4-aminobenzoic acid.

The "peptide" and "peptide compound" as used herein are not particularly limited as long as they are peptides formed from natural amino acids and/or non-natural amino acids linked via an amide bond or an ester bond. The number of residues contained in the peptide compound is preferably 5 to 30 residues, more preferably 7 to 15 residues, and further preferably 9 to 13 residues. The number of residues contained in the peptide is preferably 2 to 29 residues, more preferably 2 to 14 residues, and further preferably 2 to 12 residues. The peptide and the peptide compound may be a linear peptide or a cyclic peptide.

As used herein, the "amino acid residue" and "peptide residue" constituting a peptide compound is sometimes referred to simply as an "amino acid" and "peptide residue", respectively.

The term "optionally substituted" as used herein means that a group may be substituted with any of substituents. A substituent may be further added to each of the substituents. Such a substituent is not limited and can be one or two or more substituents each independently freely selected from any substituents including a halogen atom, an oxygen atom, a sulfur atom, a nitrogen atom, a boron atom, a silicon atom, or a phosphorus atom. Examples of the substituent include alkyl, alkoxy, fluoroalkyl, fluoroalkoxy, oxo, aminocarbonyl, alkylsulfonyl, alkylsulfonylamino, cycloalkyl, aryl, heteroaryl, heterocyclyl, arylalkyl, heteroarylalkyl, halogen, nitro, amino, monoalkylamino, dialkylamino, cyano, carboxyl, alkoxycarbonyl, and formyl.

Examples of the halogenated solvent as used herein include dichloromethane, chloroform, 1,2-dichloroethane, and carbon tetrachloride. Among these, dichloromethane, chloroform, and 1,2-dichloroethane are preferred.

Examples of the benzene-type solvent as used herein include benzene, toluene, xylene, fluorobenzene, chlorobenzene, 1,2-dichlorobenzene, bromobenzene, anisole, ethylbenzene, nitrobenzene, cumene, and benzotrifluoride. Among these, toluene, fluorobenzene, chlorobenzene, and benzotrifluoride are preferred.

Examples of the ether-type solvent as used herein include diethyl ether, tetrahydrofuran, 2-methyltetrahydrofuran, 1,4-dioxane, 1,3-dioxolane, 1,2-dimethoxyethane, diisopropyl ether, cyclopentyl methyl ether, t-butyl methyl ether, 4-methyltetrahydropyran, diglyme, triglyme, and tetraglyme.

Examples of the ester-type solvent as used herein include methyl acetate, ethyl acetate, butyl acetate, methyl propionate, propyl acetate, isopropyl acetate, isobutyl acetate, pentyl acetate, dimethyl carbonate, diethyl carbonate, and γ-valerolactone. Among these, ethyl acetate and dimethyl carbonate are preferred.

Examples of the nitrile-type solvent as used herein include acetonitrile and propionitrile. Among these, acetonitrile is preferred.

Examples of the fluoroalcohol as used herein include 2,2,2-trifluoroethanol and 1,1,1,3,3,3-hexafluoro-2-propanol.

Examples of the alcohol as used herein include methanol, ethanol, n-propanol, and 2-propanol. Among these, methanol, ethanol, and 2-propanol are preferred.

The term "one or more" as used herein means the number of 1 or 2 or larger. When the term "one or more" is used in the context associated with a substituent of a group, the term means a number from 1 to the maximum number of substituents acceptable by the group. Specific examples of the term "one or more" include 1, 2, 3, 4, 5, 6, 7, 8, 9, 10, and/or larger numbers.

The meaning of the term "and/or" as used herein includes any combination in which "and" and "or" are appropriately combined. Specifically, for example, the term "A, B, and/or C" includes the following seven variations: (i) A, (ii) B, (iii) C, (iv) A and B, (v) A and C, (vi) B and C, and (vii) A, B, and C.

(General Production Method)

General methods for producing the compounds of the present invention will be described.

In an aspect, the compounds represented by formula (1) can be produced, for example, by Production method 1 including step A as shown below.

Production method 1:

[Formula 7]

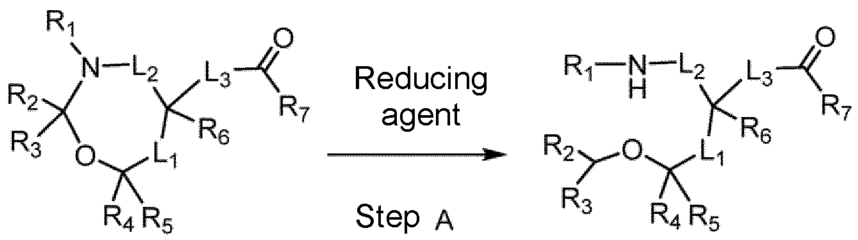


(2) (1)

In the formula, $R_1$ is an electron withdrawal group. $R_1$ is preferably -C(=O)-$R_{12}$, - C(=O)-O$R_{13}$, -S(=O)$_2$-$R_{14}$, -S(=O)$_2$-O$R_{15}$, -P(=O)-$R_{16}R_{17}$, or -P(=O)-(O$R_{18}$)$_2$, wherein $R_{12}$, $R_{13}$, $R_{14}$, $R_{15}$, $R_{16}$, $R_{17}$, and $R_{18}$ each are $C_1$-$C_6$ alkyl optionally having a substituent, aralkyl optionally having a substituent, aryl optionally having a substituent, or heteroaryl optionally having a substituent. Specific examples include methyl, ethyl, isopropyl, n-propyl, n-butyl, tert-butyl, isopentyl, trifluoromethyl, phenyl, nitrophenyl, pyridyl, benzyl, methoxybenzyl, fluorobenzyl, and 9-fluorenylmethyl. $R_1$ is more preferably -C(=O)-$R_{12}$, -C(=O)-O$R_{13}$, or -S(=O)$_2$-$R_{14}$, wherein $R_{12}$, $R_{13}$, and $R_{14}$ each are $C_1$-$C_6$ alkyl optionally having a substituent, aralkyl optionally having a substituent, aryl optionally having a substituent, or heteroaryl optionally having a substituent. Specific examples include methyl, ethyl, isopropyl, n-propyl, n-butyl, tert-butyl, isopentyl, trifluoromethyl, phenyl, nitrophenyl, pyridyl, benzyl, methoxybenzyl, fluorobenzyl, and 9-fluorenylmethyl. $R_1$ is further preferably benzyloxycarbonyl (Cbz), 9-fluorenylmethyloxycarbonyl (Fmoc), acetyl, trifluoroacetyl, methanesulfonyl, paratoluenesulfonyl, (trifluoromethyl)sulfonyl, and 2-nitrobenzenesulfonyl (Nosyl). $R_1$ is particularly preferably benzyloxycarbonyl (Cbz) and 9-fluorenylmethyloxycarbonyl (Fmoc). In an aspect, $R_1$ is an amino acid residue, or a peptide residue. In this case, $R_1$ is preferably an amino acid residue or a peptide residue whose N-terminus is protected with benzyloxycarbonyl (Cbz) or 9-fluorenylmethyloxycarbonyl (Fmoc), and more preferably an amino acid residue whose N-terminus is protected with benzyloxycarbonyl (Cbz) or 9-fluorenylmethyloxycarbonyl (Fmoc).

$R_2$ and $R_3$ in the formula are each independently hydrogen, $C_1$-$C_6$ alkyl optionally having a substituent, aralkyl optionally having a substituent, aryl optionally having a substituent, or heteroaryl optionally having a substituent, or $R_2$ and $R_3$ together with a carbon atom to which they are attached form a 3- to 8-membered alicyclic ring or a 4- to 7-membered saturated heterocycle. Preferably, $R_2$ and $R_3$ are each independently hydrogen, methyl, ethyl, n-propyl, n-butyl, tert-butyl, isobutyl, isopentyl, benzyl, phenyl, or pyridyl, or $R_2$ and $R_3$ together with a carbon atom to which they are attached form a 3- to 8-membered alicyclic ring. More preferably, when $R_2$ is methyl, ethyl, n-propyl, n-butyl, or isobutyl, $R_3$ is hydrogen or methyl, or $R_2$ and $R_3$ together with a carbon atom to which they are attached form a 4- to 6-membered alicyclic ring.

$R_4$ and $R_5$ in the formula are each independently hydrogen, $C_1$-$C_6$ alkyl optionally having a substituent, aryl optionally having a substituent, or heteroaryl optionally having a substituent, or $R_4$ and $R_5$ together with a carbon atom to which they are attached form a 3- to 8-membered alicyclic ring or a 4- to 7-membered saturated heterocycle. Preferably, $R_4$ and $R_5$ are each independently hydrogen, methyl, ethyl, n-propyl, n-butyl, tert-butyl, isobutyl, isopentyl, benzyl, phenyl, or pyridyl, or $R_4$ and $R_5$ together with a carbon atom to which they are attached form a 3-to 8-membered alicyclic ring. More preferably, $R_4$ is hydrogen, methyl, ethyl, n-propyl, n-butyl, or isobutyl, and $R_5$ is hydrogen. Further preferably, $R_4$ is hydrogen or methyl, and $R_5$ is hydrogen.

$R_6$ in the formula is hydrogen, $C_1$-$C_6$ alkyl optionally having a substituent, or aralkyl optionally having a substituent. Preferably, $R_6$ is hydrogen, methyl, ethyl, n-propyl, n-butyl, or benzyl, and more preferably, $R_6$ is hydrogen.

In the formula, $R_7$ is $-OR_8$, $-NR_9R_{9'}$, an amino acid residue, or a peptide residue; and $R_8$, $R_9$ and $R_{9'}$ are each independently hydrogen, $C_1$-$C_6$ alkyl optionally having a substituent, or aralkyl optionally having a substituent, or $R_9$ and $R_{9'}$ together with an intervening nitrogen atom form a 4- to 7-membered saturated heterocycle. Preferably, $R_7$ is $-OR_8$, and more preferably, $R_7$ is -OH. In an aspect, preferably $R_7$ is $-OR_8$, and $R_8$ is $C_1$-$C_6$ alkyl, more preferably $R_7$ is $- OR_8$, and $R_8$ is methyl. In an aspect, preferably $R_7$ is an amino acid residue whose C-terminus is protected with a $C_1$-$C_6$ alkyl ester.

In the formula, $L_1$, $L_2$, and $L_3$ each are a single bond or $-CH_2-$, provided that when $L_1$ is $- CH_2-$, $L_2$ is a single bond; when $L_2$ is $-CH_2-$, $L_1$ and $L_3$ each are a single bond; and when $L_3$ is $- CH_2-$, $L_2$ is single bond. Preferably, $L_1$, $L_2$, and $L_3$ each are a single bond.

Step A of the production method 1 is a step of producing an O-substituted serine derivative (1) by a reductive hydrogenation reaction with ring opening for a cyclic N,O-acetal derivative (2). This step can be performed in the presence or absence of a reducing agent, in the presence or absence of an acid, in the presence or absence of a solvent, and in the presence or absence of a solubilizing agent, preferably at a temperature of -50°C to 50°C, more preferably at a temperature of -40°C to 40°C, further preferably at a temperature of -30°C to 30°C, particularly preferably at a temperature of -20°C to 25°C, preferably for 5 minutes to 24 hours, more preferably for 10 minutes to 12 hours, further preferably for 20 minutes to 8 hours, particularly preferably for 30 minutes to 5 hours.

As the reducing agent, a hydride-based reducing agent can be used, and examples thereof include silane-based reducing agents such as triethylsilane, triisopropylsilane, tristrimethylsilylsilane, phenylsilane, dimethylphenylsilane, tetraphenyldisilane, poly(methylhydrosiloxane), and 1,1,3,3-tetramethyldisiloxane, and borane-based reducing agents. Among these, triethylsilane, poly(methylhydrosiloxane), and 1,1,3,3-tetramethyldisiloxane are preferably used, and triethylsilane is more preferably used.

The amount of the reducing agent used is not particularly limited, and examples thereof include 0.5 equivalents or more and 30 equivalents or less with respect to the compound represented by formula (2). The amount is preferably 1 equivalent or more and 20 equivalents or less, more preferably 1.5 equivalents or more and 10 equivalents or less, and further preferably 2 equivalents or more and 7 equivalents or less.

Examples of the acid that can be used include Brønsted acids such as trifluoromethanesulfonic acid (TfOH), methanesulfonic acid (MsOH), and trifluoroacetic acid (TFA); non-metallic Lewis acids such as a complex of boron trifluoride with an ether-based solvent and a trialkylsilyl triflate; and metallic Lewis acid as described below, and the metallic Lewis acids are preferred.

The metallic Lewis acid herein means a Lewis acid composed of a metal and an anionic group, and examples thereof include a metal halide, a metal triflate, a metal alkoxide, and a metal halide alkoxide. The metal in the metallic Lewis acid may be a known polyvalent metal, and examples thereof include titanium, tin, scandium, zirconium, zinc, aluminum, calcium, bismuth, antimony, cadmium, vanadium, molybdenum, tungsten, iron, copper, cobalt, lead, nickel, silver, and rare earth metals. Among these, titanium, tin, scandium, zirconium, zinc, and aluminum are preferred, titanium, tin, and scandium are more preferred, and titanium and tin are further preferred. The anionic group in the metallic Lewis acid may be a known anionic group, and examples thereof include anionic groups derived from trifluoromethanesulfonic acid (TfOH), methanesulfonic acid (MsOH), bis(trifluoromethanesulfonyl)imide ($Tf_2NH$), tris(trifluoromethanesulfonyl)methane ($HCTf_3$), pentafluorophenylbis(trifluryl)methane ($C_6H_5CHTf_2$), trifluoroacetic acid (TFA), 2,2,2-trifluoroethanol, 1,1,1,3,3,3-hexafluoro-2-propanol, methanol, ethanol, n-propanol, isopropanol, n-butanol, and phenol. The anionic group may be a combination of one or more independently selected from the group consisting of these anionic groups.

Specific examples of the metallic Lewis acid include metal halides such as titanium tetrahalide (e.g., titanium tetrachloride ($TiCl_4$)) and tin tetrahalide (e.g., tin tetrachloride ($SnCl_4$)), metal triflates such as scandium triflate ($Sc(OTf)_3$), metal alkoxides such as tetraalkoxytitanium (e.g., tetraisopropyl orthotitanate ($Ti(OiPr)_4$)) and alkoxytrichlorotitanium

(e.g., isopropoxytrichlorotitanium ($TiCl_3(OiPr)$)). Among these, isopropoxytrichlorotitanium is preferred. The metallic Lewis acid may also be a combination of plurality of Lewis acids, for example, a combination of titanium tetrahalide and tetraalkoxytitanium. Among these, a combination of titanium tetrachloride and tetraisopropyl orthotitanate is preferred. In this case, the molar ratio of titanium tetrahalide : tetraalkoxytitanium is not particularly limited, but is preferably 2 to 4 : 1, more preferably 3 : 1.

The amount of the acid used is not particularly limited, and examples thereof include 0.1 equivalents or more and 20 equivalents or less with respect to the compound represented by formula (2). The amount is preferably 0.5 equivalents or more and 5 equivalents or less, and more preferably 1 equivalent or more and 3 equivalents or less.

Examples of the solvent include halogenated solvents such as dichloromethane, 1,2-dichloroethane, and chloroform, and benzene-type solvents such as toluene, chlorobenzene, fluorobenzene, and benzotrifluoride. Among these, dichloromethane, toluene, and chlorobenzene are preferably used.

Step A of the production method 1 can be performed further in the presence of a solubilizing agent. Examples of the solubilizing agent include fluoroalcohols such as 2,2,2-trifluoroethanol and 1, 1, 1,3,3,3-hexafluoro-2-propanol, alcohols such as methanol, ethanol, and 2-propanol, nitrile-type solvents such as acetonitrile, and ester-type solvents such as ethyl acetate and dimethyl carbonate. Among these, 2,2,2-trifluoroethanol and 1,1,1,3,3,3-hexafluoro-2-propanol are preferably used.

In an aspect, the addition of a base in the post-treatment step of the step A of the production method 1 can reduce the content of a compound (4) contained as an impurity in the reaction mixture.

[Formula 8]

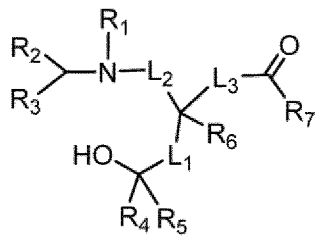

(4)

Examples of the base include sodium hydroxide, potassium hydroxide, lithium hydroxide, and tetraalkylammonium hydroxide such as tetramethylammonium hydroxide. Among these, sodium hydroxide and potassium hydroxide are preferably used.

This base treatment step can be performed preferably at a temperature of -5°C to 50°C, more preferably at a temperature of 0°C to 40°C, further preferably at a temperature of 5°C to 30°C, particularly preferably at a temperature of 10°C to 25°C, preferably for 5 minutes to 24 hours, more preferably for 10 minutes to 12 hours, further preferably for 20 minutes to 8 hours, particularly preferably for 30 minutes to 5 hours.

In an aspect, among the O-substituted serine derivatives (1) obtained by step A of the production method 1, for the O-substituted serine derivative (11) in which $R_7$ is -OH, the content of impurities contained in the reaction mixture can be reduced by forming a salt with a secondary amine, a tertiary amine, an alkali metal, or an alkaline earth metal and performing crystallization.

Examples of the salt include dicyclohexylamine, lithium hydroxide, sodium hydroxide, potassium hydroxide, and calcium hydroxide, and among these, dicyclohexylamine is preferably used.

In an aspect, the cyclic N,O-acetal derivative (2) used as the starting material in the step A of the production method 1 can be produced by a method including a step B shown below using a compound (3) as the starting material.

[Formula 9]

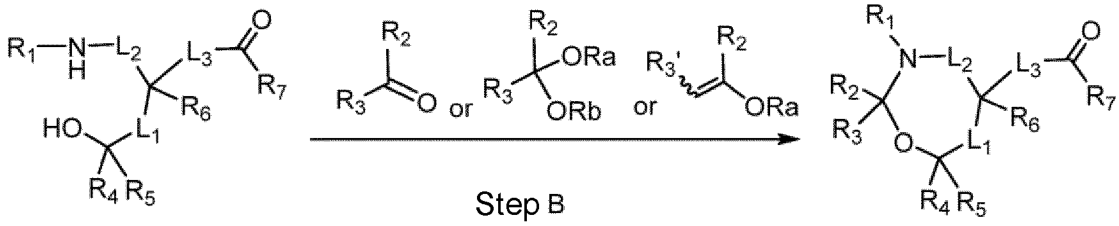


(3) (2)

In the formula, $R_1$, $R_2$, $R_3$, $R_4$, $R_5$, $R_6$, $R_7$, $L_1$, $L_2$, and $L_3$ have the same meanings as $R_1$, $R_2$, $R_3$, $R_4$, $R_5$, $R_6$, $R_7$, $L_1$, $L_2$, and $L_3$ defined in the step A.

In the formula, Ra and Rb are each independently a $C_1$-$C_6$ alkyl group, or Ra and Rb together with an intervening oxygen atom and carbon atom form a 5- to 7-membered saturated heterocycle.

In the formula, $R_{3'}$ is selected from the group consisting of hydrogen, $C_1$-$C_6$ alkyl optionally having a substituent, $C_3$-$C_6$ cycloalkyl optionally having a substituent, $C_2$-$C_6$ alkenyl optionally having a substituent, $C_2$-$C_6$ alkynyl optionally having a substituent, aralkyl optionally having a substituent, aryl optionally having a substituent, and heteroaryl optionally having a substituent, or $R_{3'}$ together with $R_2$ and an intervening carbon atom form a 4- to 8-membered alicyclic ring or a 4- to 7-membered saturated heterocycle.

The step B of the production method 1 is a step of reacting the compound (3) with an aldehyde, a ketone, an acetal, or a vinyl ether to produce the cyclic N,O-acetal derivative (2). This step can be performed in the presence or absence of an acid, in the presence or absence of a solvent, and in the presence or absence of a silylating agent, preferably at a temperature of -10°C to 80°C, more preferably at a temperature of 0°C to 70°C, further preferably at a temperature of 10°C to 60°C, particularly preferably at a temperature of 20°C to 50°C, preferably for 5 minutes to 24 hours, more preferably for 10 minutes to 12 hours, further preferably for 20 minutes to 6 hours, particularly preferably for 30 minutes to 3 hours.

Examples of the acid that can be used include Brønsted acids such as trifluoromethanesulfonic acid (TfOH), methanesulfonic acid (MsOH), and trifluoroacetic acid (TFA); non-metallic Lewis acids such as a complex of boron trifluoride with an ether-type solvent and a trialkylsilyl triflate; and metallic Lewis acid as described above, and the Lewis acids are preferred.

Specific examples of the Lewis acid include a boron trifluoride-tetrahydrofuran complex, a boron trifluoride-diethyl ether complex, and trimethylsilyl triflate.

The amount of the acid used is not particularly limited, and examples thereof include 0.05 equivalents or more and 1 equivalent or less with respect to the compound represented by formula (3). The amount is preferably 0.1 equivalents or more and 0.5 equivalents or less.

Examples of the solvent include halogenated solvents such as dichloromethane, 1,2-dichloroethane, and chloroform, benzene-type solvents such as toluene, chlorobenzene, fluorobenzene, and benzotrifluoride, and ether-type solvents such as tetrahydrofuran, 2-methyltetrahydrofuran, and cyclopentylmethyl ether. Among these, dichloromethane, toluene, and 2-methyltetrahydrofuran are preferably used.

Step B of the production method 1 can be performed further in the presence of a silylating agent. Examples of the silylating agent can include N,O-bis(trimethylsilyl)trifluoroacetamide, N,O-bis(trimethylsilyl)acetamide, and 1-trimethylsilylimidazole, and among these N,O-bis(trimethylsilyl)trifluoroacetamide is preferably used.

In an aspect, among the O-substituted serine derivatives (1) obtained by step A of the production method 1, the O-substituted serine derivative (11) in which $R_7$ is -OH can be used as a starting material to produce an O-substituted serine derivative (16) having a substituent ($-CHR_{10}R_{11}$) on a nitrogen atom by a method including step C and step D shown below.

[Formula 10]

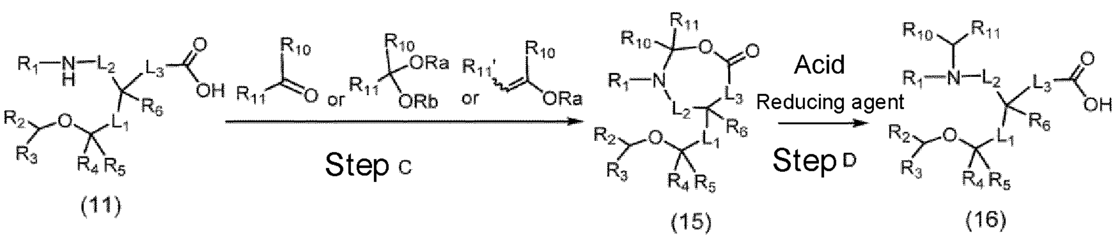


(11) (15) (16)

In the formula, $R_1$, $R_2$, $R_3$, $R_4$, $R_5$, $R_6$, $R_7$, $L_1$, $L_2$, and $L_3$ have the same meanings as $R_1$, $R_2$, $R_3$, $R_4$, $R_5$, $R_6$, $R_7$, $L_1$, $L_2$, and $L_3$ defined in the step A.

Ra and Rb in the formula have the same meaning as Ra and Rb in the step B, respectively.

In the formula, $R_{10}$ and $R_{11}$ are each independently selected from the group consisting of hydrogen, $C_1$-$C_6$ alkyl optionally having a substituent, $C_3$-$C_6$ cycloalkyl optionally having a substituent, $C_2$-$C_6$ alkenyl optionally having a substituent, $C_2$-$C_6$ alkynyl optionally having a substituent, aralkyl optionally having a substituent, aryl optionally having a substituent, and heteroaryl optionally having a substituent, or $R_{10}$ and $R_{11}$ together with an intervening carbon atom form a 3- to 8-membered alicyclic ring or a 4- to 7-membered saturated heterocycle.

In the formula, $R_{11'}$ is selected from the group consisting of hydrogen, $C_1$-$C_6$ alkyl optionally having a substituent, $C_3$-$C_6$ cycloalkyl optionally having a substituent, $C_2$-$C_6$ alkenyl optionally having a substituent, $C_2$-$C_6$ alkynyl optionally having a substituent, aralkyl optionally having a substituent, aryl optionally having a substituent, and heteroaryl optionally

having a substituent, or $R_{11}$, together with $R_{10}$ and an intervening carbon atom form a 4- to 8-membered alicyclic ring or a 4- to 7-membered saturated heterocycle.

The above step C is a step of reacting the O-substituted serine derivative (11) with an aldehyde, a ketone, an acetal, or a vinyl ether to obtain an oxazolidinone (15), according to, for example, the method of Freidinger et al. (J. Org. Chem., 1983, 48 (1), 77-81). This step can be performed in the presence or absence of a Lewis acid and in the presence or absence of a solvent.

Examples of the Lewis acid that can be used include non-metallic Lewis acids such as a complex of boron trifluoride with an ether-based solvent and a trialkylsilyl triflate; and metallic Lewis acid as described above.

Specific examples of the Lewis acid include a boron trifluoride-tetrahydrofuran complex, a boron trifluoride-diethyl ether complex, trimethylsilyl triflate, titanium tetrachloride, and tetraalkoxytitanium, and among these, a boron trifluoride-tetrahydrofuran complex and a boron trifluoride-diethyl ether complex are preferred.

The amount of the Lewis acid used is not particularly limited, and examples thereof include 0.2 equivalents or more and 5 equivalents or less with respect to the compound represented by formula (11). The amount is preferably 0.5 equivalents or more and 3 equivalents or less, and more preferably 1 equivalent or more and 2 equivalents or less.

Examples of the solvent include halogenated solvents such as dichloromethane, 1,2-dichloroethane, and chloroform, and benzene-type solvents such as toluene, chlorobenzene, fluorobenzene, and benzotrifluoride. Among these, dichloromethane, toluene, and chlorobenzene are preferably used.

The above step D is a step of performing a reductive ring-opening reaction on the oxazolidinone (15) to produce an O-substituted serine derivative (16) having a substituent ($-CHR_{10}R_{11}$) on a nitrogen atom. This step can be performed in the presence or absence of an acid, in the presence or absence of a reducing agent, and in the presence or absence of a solvent.

Examples of the acid that can be used include Brønsted acids such as trifluoromethanesulfonic acid (TfOH), methanesulfonic acid (MsOH), and trifluoroacetic acid (TFA); non-metallic Lewis acids such as a complex of boron trifluoride with an ether-based solvent and a trialkylsilyl triflate; and metallic Lewis acid as described above, and the Lewis acids are preferred.

Specific examples of the Lewis acid include a boron trifluoride-tetrahydrofuran complex, a boron trifluoride-diethyl ether complex, trimethylsilyl triflate, titanium tetrachloride, and tetraalkoxytitanium, and among these, trimethylsilyl triflate is preferred.

The amount of the Lewis acid used is not particularly limited, and examples thereof include 0.2 equivalents or more and 5 equivalents or less with respect to the compound represented by formula (15). The amount is preferably 0.5 equivalents or more and 3 equivalents or less, and more preferably 1 equivalent or more and 2 equivalents or less.

As the reducing agent, a hydride-based reducing agent can be used, and examples thereof include silane-based reducing agents such as triethylsilane, triisopropylsilane, tristrimethylsilylsilane, phenylsilane, dimethylphenylsilane, tetraphenyldisilane, poly(methylhydrosiloxane), and 1,1,3,3-tetramethyldisiloxane, and borane-based reducing agents. Among these, triethylsilane, poly(methylhydrosiloxane), and 1,1,3,3-tetramethyldisiloxane are preferably used, and triethylsilane is more preferably used.

The amount of the reducing agent used is not particularly limited, and examples thereof include 1 equivalent or more and 20 equivalents or less with respect to the compound represented by formula (15). The amount is preferably 1.5 equivalents or more and 10 equivalents or less, and more preferably 2 equivalents or more and 5 equivalents or less.

Examples of the solvent include halogenated solvents such as dichloromethane, 1,2-dichloroethane, and chloroform, and benzene-type solvents such as toluene, chlorobenzene, fluorobenzene, and benzotrifluoride. Among these, dichloromethane, toluene, and chlorobenzene are preferably used.

In an aspect, the O-substituted serine derivative represented by formula (11) or formula (16) obtained by the above production method can be used to produce a peptide compound, for example, by the production method described in WO2021132545 (synthesis method of peptide compounds). Specifically, by a similar manner to the production examples of Cbz-Ser(tBu)-MePhe-MeVal-Asp(tBu)-piperidine (4 mer), Cbz-MeIle-Ser(tBu)-MePhe-MeVal-Asp(tBu)-piperidine(5 mer), Cbz-MeGly-MeIle-Ser(tBu)-MePhe-MeVal-Asp(tBu)-piperidine(6 mer), Cbz-MeLeu-Val-MeGly-MeIle-Ser(tBu)-MePhe-MeVal-Asp(tBu)-piperidine(7 mer), Cbz-Val-MeGly-MeIle-Ser(tBu)-MePhe-MeVal-Asp(tBu)-piperidine(8 mer), Cbz-Leu-MeLeu-Val-MeGly-MeIle-Ser(tBu)-MePhe-MeVal-Asp(tBu)-piperidine(9 mer), Cbz-MePhe-Leu-MeLeu-Val-MeGly-MeIle-Ser(tBu)-MePhe-MeVal-Asp(tBu)-piperidine(10 mer), or Cbz-MeAla-MePhe-Leu-MeLeu-Val-MeGly-MeIle-Ser(tBu)-MePhe-MeVal-Asp(tBu)-piperidine (11mer) described in Example 14 of WO2021132545, a peptide compound can be produced by using the O-substituted serine derivative represented by formula (11) or formula (16) instead of Cbz-Ser(tBu)-OH. In this case, $R_1$ in formula (11) or formula (16) is a protecting group of an amino group, preferably Cbz. The amino acid sequences described above are just one example, and the kind of amino acids is not limited.

In an aspect, the O-substituted serine derivative represented by formula (11) or formula (16) can be used to produce a peptide compound or a cyclic peptide compound, for example, by the production method described in WO2013100132 (cyclization method of peptide compounds). Specifically, a peptide compound or a cyclic peptide

compound can be produced in accordance with the methods for producing a peptide compound or a cyclic peptide compound in Examples 18 to 20 of WO2013100132 by using the O-substituted serine derivative represented by Formula (11) or Formula (16) instead of Fmoc-Ser(tBu)-OH. In this case, $R_1$ in formula (11) or formula (16) is a protecting group of an amino group, preferably Fmoc. The amino acid sequences described above are just one example, and the kind of amino acids is not limited.

In an aspect, the present invention is a compound or a salt thereof or a solvate thereof or a salt thereof or a solvate thereof selected from the group consisting of:
a compound selected from the group consisting of:

2-(benzyloxycarbonylamino)-3-(cyclobutoxy)propanoic acid;
2-(benzyloxycarbonylamino)-3-isopentyloxy-propanoic acid;
2-(benzyloxycarbonylamino)-3-isopropoxy-butanoic acid;
2-[benzyloxycarbonyl(methyl)amino]-3-(cyclobutoxy)propanoic acid;
3-benzyloxycarbonyl-2-ethyl-oxazolidine-4-carboxylic acid;
3-benzyloxycarbonyl-2-isobutyl-oxazolidine-4-carboxylic acid;
benzyl 4-(cyclobutoxymethyl)-5-oxo-oxazolidine-3-carboxylate;
benzyl 4-{[1-methoxy-1-oxapropan-2-yl]carbamoyl}-2,2,5-trimethyloxazolidine-3-carboxylate; N-(benzyloxy)carbonyl-O-isopropyl-threonyl-alanine methyl ester; and
N-[(9H-fluoren-9-ylmethoxycarbonyl)-leucyl]-O-isopropyl-threonine.

These compounds or their salts or their solvates can be produced by the production methods described herein.

In an aspect, the present invention is the following compound or a salt thereof or a solvate thereof or a salt thereof or a solvate thereof:

(2S)-2-(benzyloxycarbonylamino)-3-(cyclobutoxy)propanoic acid or a salt thereof or a solvate thereof or a salt thereof or a solvate thereof;
(2S)-2-(benzyloxycarbonylamino)-3-isopentyloxy-propanoic acid or a salt thereof or a solvate thereof or a salt thereof or a solvate thereof;
(2S,3R)-2-(benzyloxycarbonylamino)-3-isopropoxy-butanoic acid or a salt thereof or a solvate thereof;
(2S)-2-[benzyloxycarbonyl(methyl)amino]-3-(cyclobutoxy)propanoic acid or a salt thereof or a solvate thereof;
(4S)-3-benzyloxycarbonyl-2-ethyl-oxazolidine-4-carboxylic acid or a salt thereof or a solvate thereof;
(4S)-3-benzyloxycarbonyl-2-isobutyl-oxazolidine-4-carboxylic acid or a salt thereof or a solvate thereof;
benzyl (4S)-4-(cyclobutoxymethyl)-5-oxo-oxazolidine-3-carboxylate or a salt thereof or a solvate thereof;
benzyl (4S,5R)-4-{[(S)-1-methoxy-1-oxapropan-2-yl]carbamoyl}-2,2,5-trimethyloxazolidine-3-carboxylate or a salt thereof or a solvate thereof;
N-(benzyloxy)carbonyl-O-isopropyl-L-threonyl-L-alanine methyl ester or a salt thereof or a solvate thereof; and
N-[(9H-fluoren-9-ylmethoxycarbonyl)-L-leucyl]-O-isopropyl-L-threonine or a salt thereof or a solvate thereof.

These compounds or their salts or their solvates can be produced by the production methods described herein.

It should be noted that all of the prior art cited herein are incorporated herein by reference.

**Examples**

Hereinafter, the present disclosure will be described in more detail based on Examples, but the present disclosure is not limited to the following Examples.

In the following Examples, high performance liquid chromatography (HPLC) analysis was performed using any of the analytical conditions described below. Detection of each compound was performed using a photodiode array detector or mass spectrometer, although other techniques such as evaporative light scattering detection may also be used.

<u>HPLC analysis condition method 1</u>

Apparatus: Waters ACQUITY UPLC H-Class
Column: Ascentis Express 90A C18 (Sigma-Aldrich Co. LLC), 2.1 mm ID × 50 mm, 2.7 μm
Mobile phase: 0.05% TFA/water (A), 0.05% TFA/MeCN (B)
Elution method: B): 5% (0 min) → 100% (5 min) → 5% (5.1 min) → 5% (7 min)
Flow rate: 0.5 mL/min

Column temperature: 35°C
Detection wavelength: 210 nm (PDA)

HPLC analysis condition method 2

Apparatus: Waters ACQUITY UPLC H-Class
Column: CHIRALPAK IC-3 (Daicel Corporation) 4.6 mm ID × 150 mm, 3 μm
Mobile phase: 0.05% TFA/water (A), 0.05% TFA/MeCN (B)
Elution method: B): 5% (0 min) → 60% (20 min) → 5% (20.1 min) → 5% (25 min)
Flow rate: 1.0 mL/min
Column temperature: 30°C
Detection wavelength: 210 nm (PDA)

HPLC analysis condition method 3

Apparatus: Waters ACQUITY UPLC H-Class + ACQUITY QDA
Column: Ascentis Express 90A C18 (Sigma-Aldrich Co. LLC), 2.1 mm ID × 50 mm, 2.7 μm
Mobile phase: 0.05% TFA/water (A), 0.05% TFA/MeCN (B)
Elution method: B): 5% (0 min) → 100% (6 min) → 5% (6.1 min) → 5% (8 min)
Flow rate: 0.5 mL/min
Column temperature: 35°C
Detection wavelength: 210 nm (PDA)

HPLC analysis condition method 4

Apparatus: Waters ACQUITY UPLC H-Class
Column: Ascentis Express C18 (Sigma-Aldrich Co. LLC), 4.6 mm ID × 10 cm, 2.7 μm
Mobile phase: 0.05% TFA/water (A), 0.05% TFA/MeCN (B)
Elution method: B): 20% (0 min) → 100% (9 min) → 20% (9.1 min) → 20% (13 min)
Flow rate: 0.7 mL/min
Column temperature: 35°C
Detection wavelength: 210 nm (PDA)

HPLC analysis condition method 5

Apparatus: Waters ACQUITY UPLC H-Class + ACQUITY QDA
Column: CAPCELL CORE ADME manufactured (OSAKA SODA CO., LTD.), 2.1 mm ID × 50 mm, 2.7 μm
Mobile phase: 0.05% TFA/water (A), 0.05% TFA/MeCN (B)
Elution method: B): 5% (0 min) → 100% (5 min) → 5% (5.1 min) → 5% (7 min)
Flow rate: 0.5 mL/min
Column temperature: 35°C
Detection wavelength: 210 nm (PDA)

HPLC analysis condition method 6

Apparatus: Waters ACQUITY UPLC H-Class
Column: CHIRALPAK IC-3 (Daicel Corporation) 4.6 mm ID × 150 mm, 3 μm
Mobile phase: 0.05% TFA/water (A), 0.05% TFA/MeCN (B)

Elution method: B) 35% (0 min) → 90% (20 min) → 100% (20.1 min) → 100% (22 min) → 35% (22.1 min)→ 35% (27 min)
Flow rate: 1.0 mL/min
Column temperature: 30°C
Detection wavelength: 210 nm (PDA)

$^1$H-NMR spectra were measured using a nuclear magnetic resonance device ECX500II (manufactured by JEOL Ltd.) and referenced to the deuterium lock signal from the sample solvent. As the sample solvent, commercially available deuterated solvents were used, depending on the purpose of measurement. The chemical shift of tetramethylsilane used as an internal standard was set to 0 ppm, and the chemical shift of the signal of the compound to be analyzed was expressed in ppm. Abbreviations of a signal were expressed as follows: s = singlet, brs = broad singlet, d = doublet, t = triplet, q = quartet, dd = double doublet, and m = multiplet. The splitting width of a signal was expressed as a J value (Hz). The integral value of signals was calculated on the basis of a ratio of the signal area intensity of each signal.

The measuring method by qNMR was performed by dissolving a residue containing the target compound and an internal standard in DMSO-$d_6$ and subjecting to the following analysis conditions. The yield was calculated by the following expression using the value of the Content of the target compound in the residue calculated by qNMR, and the value of the Purity of the target compound in the residue calculated by HPLC.

[Expression 1]

$$\text{Yield (\%)} = \frac{\text{Weight of residue (mg)} \times \text{Content (\%)} \times \text{Purity (\%)}}{\text{Theoretical yield (mg)}} \times 100$$

Measuring apparatus: JNM-ECZ500R
Internal standard: 3,5-bis(trifluoromethyl)benzoic acid
Measurement conditions ($^1$H-NMR): DMSO-$d_6$, pulse angle 90°C, digital resolution 0.25 Hz, relaxation time 60 seconds, no spin, cumulative number 8 times

Example 1: Condition screening for synthesis of (2S)-2-(benzyloxycarbonylamino)-3-propoxy-propanoic acid (compound 3A) and (2S)-2-(benzyloxycarbonylamino)-3-isopropoxy-propanoic acid (compound 3B)

(1-1): Synthesis of (4S)-3-benzyloxycarbonyl-2-ethyl-oxazolidine-4-carboxylic acid (compound 2A)

[Formula 11]

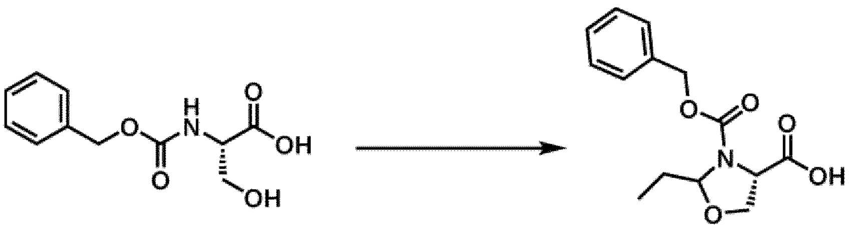

1 2A

After the atmosphere in a reaction vessel was replaced with nitrogen, N-benzyloxycarbonyl-L-serine (compound 1) (2.01 g, 8.40 mmol), magnesium sulfate (3.01 g, 25 mmol), and toluene (10 mL) were added to the reaction vessel at room temperature. Then, propionaldehyde (0.90 mL, 12.6 mmol) and boron trifluoride diethyl ether complex (0.21 mL, 1.7 mmol) were added, and the mixture was stirred at 40°C for 2.5 hours. The reaction mixture was filtered, then water (10 mL) was added to the filtrate and the resulting mixture was stirred. After the aqueous layer was discarded, the resulting organic layer was washed twice with 5% aqueous sodium carbonate solution (10 mL). The resulting aqueous layers were mixed, a 5% aqueous sodium bisulfate monohydrate solution (55 mL) was added, and then the resulting mixture was washed three times with toluene (10 mL). The resulting organic layer was washed with 5% aqueous NaCl solution (10 mL). The resulting organic layer was concentrated, then azeotropic dehydration with the addition of toluene (6 mL) was repeated three times

to afford 2.26 g of residue containing compound 2A (86.5% content) (83.2% yield from HPLC and qNMR analyses).

UV intensity ratio (as diastereomer mixture): 98.0%
(Detection wavelength 210 nm, retention time 2.735 and 2.776 minutes, HPLC analysis condition method 1)

(1-2): Screening of acids for synthesis of (2S)-2-(benzyloxycarbonylamino)-3-propoxy-propanoic acid (compound 3A)

[Formula 12]

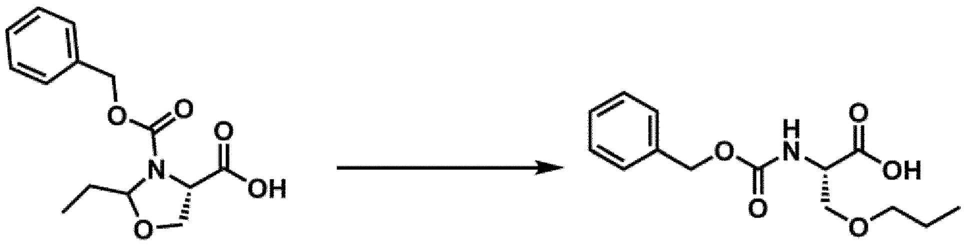

2A 3A

The reaction products obtained under the conditions using the various acids described in Table 1 below were examined as follows.

To a reaction vessel, (4S)-3-benzyloxycarbonyl-2-ethyl-oxazolidine-4-carboxylic acid (compound 2A) obtained in (1-1) above (63.3 mg, 0.20 mmol), dichloromethane (0.28 mL) and triethylsilane (0.16 mL, 0.98 mmol) were added, and the acid (0.20 mmol) described in Table 1 was added at the external temperature of 0°C. The mixture was stirred at the external temperature of 0°C for 1 hour. The resulting reaction mixture, was analyzed using HPLC analysis condition method 1.

The results are shown in Table 1. Table 1 shows the HPLC area ratios of the peak of compound 3A, compound 4A, compound 2A or compound 1 with respect to the peaks of all.

Compound 3A: retention time 2.836 min, LCMS m/z 282 $[M+H]^+$
Compound 4A: retention time 2.418 min, LCMS m/z 282 $[M+H]^+$

(Structure of compound 4A)

[Formula 13]

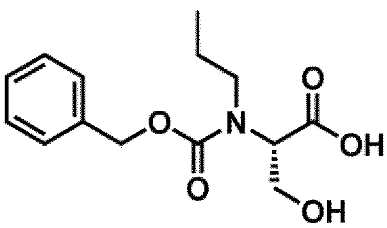

4A

Compound 2A (diastereomer mixture): total of retention times 2.744 and 2.783 minutes, LCMS m/z 280 $[M+H]^+$
Compound 1: retention time 1.829 min, LCMS m/z 240 $[M+H]^+$

[Table 1]

| run | Acid | Compound 3A (Target) | Compound 4A (Impurity) | Compound 2A (Starting material) | Compound 1 (Impurity) |
|---|---|---|---|---|---|
| 1 | $BF_3 \cdot OEt_2$ | 11 | 18 | 52 | 6 |
| 2 | $TiCl_4$ | 55 | 10 | 27 | 8 |
| 3 | $Sc(OTf)_3$ | 68 | 3 | 1 | 24 |
| 4 | $SnCl_4$ | 74 | 5 | < 1 | 19 |
| 5 | $ZrCl_4$ | 41 | 1 | 48 | 7 |

From the above results, target compound 3A formed when any acid was used. However, when $BF_3 \cdot OEt_2$ was used, the production of impurity 4A was larger than that of compound 3A (run 1). In contrast, when a metallic Lewis acid such as $TiCl_4$, $Sc(OTf)_3$, $SnCl_4$, or $ZrCl_4$ was used, the production of impurity 4A was reduced, and it was found that compound 3A was selectively obtained (runs 2 to 5).

(1-3): Screening of reducing agent, solvent, and solubilizing agent for synthesis of (2S)-2-(benzyloxycarbonylamino)-3-propoxy-propanoic acid (compound 3A)

The effects of the acids, reducing agents, solvents and solubilizing agents described in Table 2 below were examined as follows.

To a reaction vessel, (4S)-3-benzyloxycarbonyl-2-ethyl-oxazolidine-4-carboxylic acid (compound 2A) obtained in (1-1) above (68.4 mg, 0.21 mmol), a solvent (0.30 mL), a solubilizing agent (0.85 mmol), and a reducing agent (1.06 mmol) were added, and the acid (0.21 mmol) described in Table 2 was added at the external temperature of 0°C. The mixture was stirred at the external temperature of 0°C for 1 hour. For the resulting reaction mixture, HPLC analysis was performed using HPLC analysis condition method 1.

The results are shown in Table 2. Table 2 shows the HPLC area ratios of the peak of compound 3A, compound 4A, compound 2A or compound 1 with respect to the peaks of all.

Compound 3A: retention time 2.836 min, LCMS m/z 282 $[M+H]^+$
Compound 4A: retention time 2.418 min, LCMS m/z 282 $[M+H]^+$

(Structure of compound 4A)

[Formula 14]

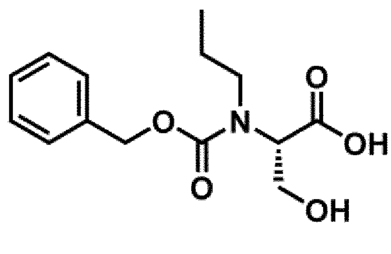

**4A**

Compound 2A (diastereomer mixture): total of retention times 2.744 and 2.783 minutes, LCMS m/z 280 $[M+H]^+$
Compound 1: retention time 1.829 min, LCMS m/z 240 $[M+H]^+$

[Table 2]

| run | Acid | Reducing agent | Solvent | Solubilizing agent | Compound 3A (Target) | Compound 4A (Impurity) | Compound 2A (Starting material) | Compound 1 (Impurity) |
|---|---|---|---|---|---|---|---|---|
| 1 | $SnCl_4$ | $Et_3SiH$ | DCM | - | 74 | 5 | < 1 | 19 |
| 2 | $SnCl_4$ | $Et_3SiH$ | toluene | HFIP | 61 | 3 | <1 | 28 |
| 3 | $SnCl_4$ | $Et_3SiH$ | toluene | MeCN | 10 | 5 | 46 | 16 |
| 4 | $TiCl_4$ | $Et_3SiH$ | DCM | - | 55 | 10 | 27 | 8 |
| 5* | $TiCl_4$ | $Et_3SiH$ | toluene * | - | 28 | 7 | 51 | 9 |
| 6* | TfOH | $Et_3SiH$ | Toluene* | | 26 | 24 | 2 | 32 |
| 7 | $TiCl_4$ | PMHS | DCM | - | 39 | 6 | 39 | 13 |
| 8 | $TiCl_4$ | TMDS | DCM | - | 13 | 3 | 59 | 25 |
| *) The solvent amount was 4 v/w in runs 5 and 6. | | | | | | | | |

From the above results, it was confirmed that compound 3A of interest can also be obtained by the present reaction even when using a toluene solvent (run 5). Instead of metallic Lewis acids, as a result of examining the conditions using a Brønsted acid such as trifluoromethanesulfonic acid (TfOH), compound 3A was obtained, but approximately the same amount of impurity 4A was generated (run 6). It should be noted that the reaction in toluene solvent sometimes generates insoluble materials resulting in formation of heterogeneous mixture, while it was confirmed that the reaction progresses even with the addition of a solubilizing agent such as HFIP or MeCN (runs 2 and 3). In addition, it was confirmed that target compound 3A was selectively obtained relative to impurity 4A even when a hydride reducing agent other than triethylsilane, such as PMHS or TMDS, was used (runs 7 and 8).

(1-4): Synthesis of (4S)-3-benzyloxycarbonyl-2,2-dimethyl-oxazolidine-4-carboxylic acid (compound 2B)

[Formula 15]

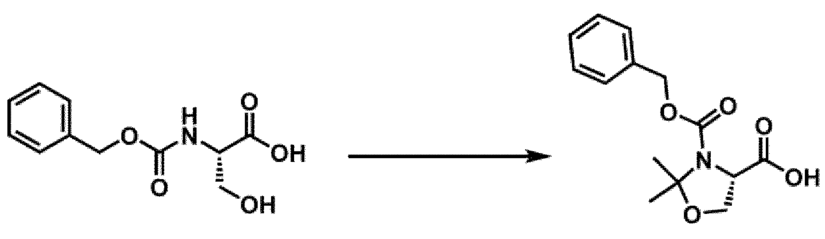

1

2B

N-Benzyloxycarbonyl-L-serine (compound 1) (3.49 g, 14.6 mmol), acetone (17.4 mL), and 2,2-dimethoxypropane (15.7 mL, 128 mmol) were added to a reaction vessel. Boron trifluoride diethyl ether complex (0.19 mL, 1.5 mmol) was added at room temperature, and then the mixture was stirred at the external temperature of 40°C for 45 minutes. Subsequently, triethylamine (0.20 mL, 1.5 mmol) was added at room temperature and the mixture was stirred for 5 minutes. The resulting residue was concentrated, and toluene (18 mL) and water (21 mL) were added, and the mixture was stirred. After the aqueous layer was discarded, the resulting organic layer was washed with 5% aqueous NaCl solution (10.5 mL). Then azeotropic dehydration with the addition of toluene (10.5 mL) was repeated three times to afford 4.43 g of residue containing compound 2B (12.7 mmol) (87.1% yield from HPLC and qNMR analyses). UV intensity ratio: 96.7%

(Detection wavelength 210 nm, retention time 2.673 minutes, HPLC analysis condition method 1)

(1-5): Screening of solvent and solubilizing agent for synthesis of (2S)-2-(benzyloxycarbonylamino)-3-isopropoxy-propanoic acid (compound 3B)

[Formula 16]

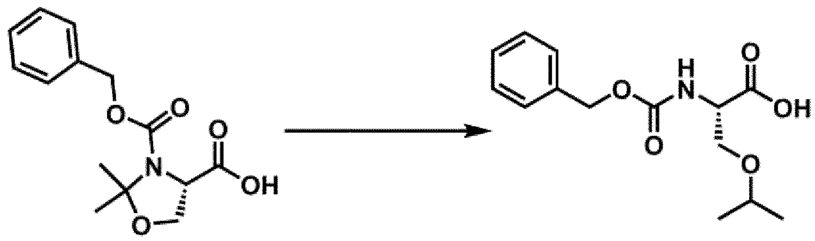

2B 3B

The effects of the solvents and solubilizing agents described in Table 3 below were examined as follows.

To a reaction vessel, (4S)-3-benzyloxycarbonyl-2,2-dimethyl-oxazolidine-4-carboxylic acid (compound 2B) obtained in (1-4) above (69.0 mg, 0.20 mmol), a solvent (0.28 mL), a solubilizing agent (0.79 mmol), and a reducing agent (0.99 mmol) were added, and the acid (0.20 mmol) described in Table 3 was added at the external temperature of 0°C. The mixture was stirred at the external temperature of 0°C for 1 hour. The resulting reaction mixture was analyzed using HPLC analysis condition method 1.

The results are shown in Table 3. Table 3 shows the HPLC area ratios of the peak of compound 3B, compound 4B, compound 2B or compound 1 with respect to the peaks of all.

Compound 3B: retention time 2.776 min, LCMS m/z m/z 282 $[M+H]^+$
Compound 4B: retention time 2.346 min, LCMS m/z m/z 282 $[M+H]^+$

(Structure of compound 4B)

[Formula 17]

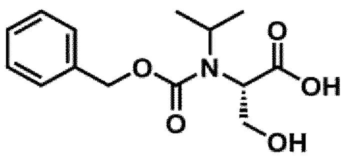

4B

Compound 2B: retention time 2.675 min, LCMS m/z m/z 280 $[M+H]^+$
Compound 1: retention time 1.833 min, LCMS m/z 240 $[M+H]^+$

[Table 3]

| run | Acid | Reducing agent | Solvent | Solubilizing agent | Compound 3B (Target) | Compound B (Impurity) | Compound 2B (Starting material) | Compound 1 (Impurity) |
|---|---|---|---|---|---|---|---|---|
| 1 | $TiCl_4$ | $Et_3SiH$ | DCM | - | 68 | < 1 | 22 | 9 |
| 2 | $TiCl_4$ | $Et_3SiH$ | toluene | - | 42 | < 1 | 35 | 14 |
| 3 | $TiCl_4$ | $Et_3SiH$ | toluene | MeCN | 53 | < 1 | 29 | 16 |
| 4 | $TiCl_4$ | $Et_3SiH$ | toluene | AcOEt | 23 | < 1 | 45 | 29 |

(continued)

| run | Acid | Reducing agent | Solvent | Solubilizing agent | Compound 3B (Target) | Compound B (Impurity) | Compound 2B (Starting material) | Compound 1 (Impurity) |
|---|---|---|---|---|---|---|---|---|
| 5 | $TiCl_4$ | $Et_3SiH$ | toluene | HFIP | 80 | < 1 | < 1 | 18 |

From the above results, it was confirmed that compound 3B of interest can also be obtained even when the reaction was conducted with toluene solvent (run 2). It should be noted that the reaction in toluene solvent sometimes generates insoluble materials resulting in formation of heterogeneous mixture, while it was confirmed that the reaction progresses even with the addition of a solubilizing agent such as MeCN, ethyl acetate, and HFIP (runs 3 to 5). Especially, compound 3B was obtained in high yield under conditions in which HFIP was added as a solubilizing agent.

(1-6): Screening of acids and solvents for synthesis of (2S)-2-(benzyloxycarbonylamino)-3-isopropoxy-propanoic acid (compound 3B)

The effects of the acids and solvents described in Table 4 below were examined as follows.

The (4S)-3-benzyloxycarbonyl-2,2-dimethyl-oxazolidine-4-carboxylic acid (compound 2B) obtained in (1-4) above (0.11 g, 0.34 mmol), a solvent (0.47 mL), and triethylsilane (1.68 mmol) were mixed to prepare a substrate solution.

To another reaction vessel, 1 M solution of $TiCl_4$ in toluene (0.25 mL, 0.25 mL), $Ti(OiPr)_4$ (0.084 mmol), and HFIP (0.21 mL, 2.0 mmol) were added to prepare a solution containing $TiCl_3(OiPr)$ (1 equivalent). The solution containing $TiCl_3(OiPr)$ was cooled at the external temperature of 0°C, and then the substrate solution prepared above was added thereto. After stirring at the external temperature of 0°C for 0.5 hours, the resulting reaction mixture was analyzed by using HPLC analysis condition method 1 (For Table 4, run 3, a solution containing $TiCl_3(OiPr)$ was prepared using $TiCl_4$, $Ti(OiPr)_4$, and chlorobenzene).

The results are shown in Table 4. Table 4 shows the area ratios of the peak of compound 3B, compound 4B, compound 2B or compound 1 with respect to the peaks of all.

[Table 4]

| run | Acid (equivalent) | Solvent | Total solvent amount (v/w) | Compound 3B (Target) | Compound 4B (Impurity) | Compound 2B (Starting material) | Compound 1 (Impurity) |
|---|---|---|---|---|---|---|---|
| 1 | $TiCl_3(OiPr)$ 1 Equivalent | toluene | 7.9 | 50 | < 1 | 12 | 37 |
| 2 | $TiCl_3(OiPr)$ 2 Equivalents | toluene | 10.6 | 91 | < 1 | < 1 | 7 |
| 3 | $TiCl_3(OiPr)$ 2 Equivalents | PhCl | 10 | 95 | < 1 | < 1 | 3 |

From the above results, it was found that the present reaction proceeds with higher selectivity when $TiCl_3(OiPr)$, which has a lower Lewis acidity than $TiCl_4$, was used (runs 1 and 2). It was also confirmed that compound 3B can be obtained with high selectivity using not only aromatic hydrocarbon solvents such as toluene, but also halogen-based aromatic solvents such as chlorobenzene (run 3).

<u>Example 2: Synthesis of (2S)-2-(benzyloxycarbonylamino)-3-propoxy-propanoic acid·dicyclohexylamine salt (compound 3A·DCHA salt)</u>

(2-1): Synthesis of (4S)-3-benzyloxycarbonyl-2-ethyl-oxazolidine-4-carboxylic acid (compound 2A)

[Formula 18]

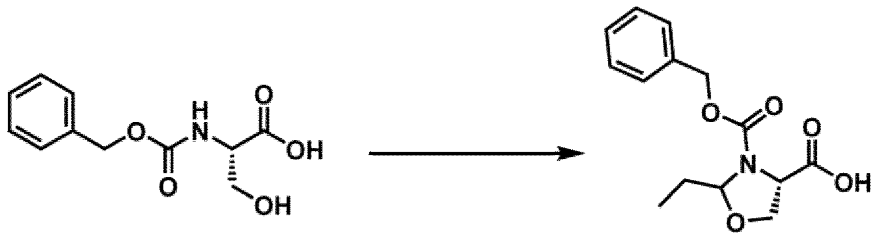

1 2A

After the atmosphere in a reaction vessel was replaced with nitrogen gas, N-benzyloxycarbonyl-L-serine (compound 1) (10.0 g, 41.8 mmol), magnesium sulfate (15.1 g, 125 mmol), and toluene (50 mL) were added to the reaction vessel at room temperature. Then, propionaldehyde (4.50 mL, 62.7 mmol) and boron trifluoride diethyl ether complex (1.06mL, 8.36 mmol) were added, and the resulting mixture was stirred at 40°C for 3 hours. Subsequently, triethylamine (1.17 mL, 8.36 mmol) was added and the mixture was stirred. Water (50 mL) was then added at the external temperature of 25°C and the mixture was stirred for 1 hour. The aqueous layer was discarded, and the resulting organic layer was washed with water (50 mL). The resulting organic layer, a 10% aqueous sodium carbonate solution (50 mL) was added and the mixture was stirred, and then the organic layer was discarded. To the resulting aqueous layer, a 10% aqueous sodium bisulfate monohydrate solution (140 mL) and toluene (100 mL) were added. The resulting mixture was stirred. The aqueous layer was discarded, and then the resulting organic layer was washed with water (30 mL). The resulting organic layer was concentrated. Azeotropic dehydration with the addition of toluene (20 mL) was repeated three times to afford 10.8 g of residue containing compound 2A.

UV intensity ratio (as diastereomer mixture): 97.7%
(Detection wavelength 210 nm, retention time 2.744 and 2.783 minutes, HPLC analysis condition method 1)

(2-2): Synthesis of (2S)-2-(benzyloxycarbonylamino)-3-propoxy-propanoic acid (compound 3A)

[Formula 19]

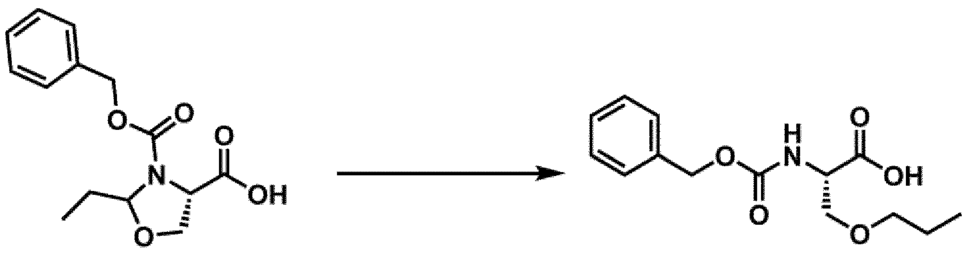

2A 3A

To the residue (10.8 g) containing compound 2A obtained in (2-1) above, chlorobenzene (25 mL) and triethylsilane (14.3 mL, 89.3 mmol) were added to prepare a substrate solution. After the atmosphere in a reaction vessel was replaced with nitrogen, chlorobenzene (25 mL) and titanium tetrachloride (6.69 mL, 60.7 mmol) were added. Then 2,2,2-trifluoroethanol (7.65 mL, 107 mmol) and tetraisopropyl orthotitanate (5.75 mL, 19.6 mmol) were added to the reaction vessel at the external temperature of -10°C. The substrate solution was added to the reaction vessel at the external temperature of -10°C. and then the mixture was stirred at the external temperature of -10°C for 4 hours, and then at the external temperature of 0°C for 1.5 hours. A 10% aqueous ammonium chloride solution (50 mL) was added while stirring, and then the resulting mixture was stirred at the external temperature of 25°C for 10 minutes. After discarding the aqueous layer, toluene (50 mL) and citric acid (50 mL) were added to the resulting organic layer, and the resulting mixture was stirred. The aqueous layer was discarded, and then the obtained organic layer was washed with water (50 mL). (HPLC analysis using HPLC analysis condition method 1 using a portion of the organic layer obtained here resulted in an peak area ratio of compound 3A (retention time 2.818 minutes) : compound 4A (retention time 2.399 minutes) of 90.6 : 9.4.)

To the resulting organic layer, a 2 M aqueous sodium hydroxide solution (50 mL) was added, and the mixture was stirred for 1 hour.
(HPLC analysis using HPLC analysis condition method 1 using a portion of the aqueous layer obtained here resulted in an area ratio of compound 3A (retention time 2.852 minutes) : compound 4A (retention time 2.399 minutes) of 99.9 or more: 0.1 or less.)
After discarding the organic layer, the resulting aqueous layer was washed with CPME (50 mL). To the resulting aqueous layer, 2 M hydrochloric acid (50 mL) and toluene (50 mL) were added and the mixture was stirred. After discarding the aqueous layer, the obtained organic layer was washed twice with water (50 mL). The resulting organic layer was concentrated, and azeotropic dehydration with the addition of toluene (25 mL) was repeated twice to afford 7.99 g of residue containing compound 3A.

UV intensity ratio: 94.6%
(Detection wavelength 210 nm, retention time 2.912 minutes, HPLC analysis condition method 1)

(2-3): Synthesis of (2S)-2-(benzyloxycarbonylamino)-3-propoxy-propanoic acid·dicyclohexylamine salt (compound 3A·DCHA salt)

[Formula 20]

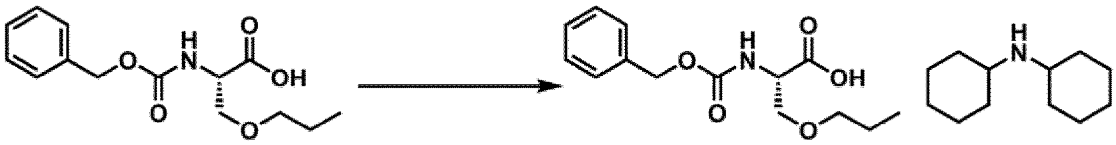

3A

3A·DCHA salt

To the residue (7.99 g) containing compound 3A obtained in (2-2) above, toluene (68 mL) and DCHA (5.64 mL, 28.0 mmol) were added while stirring at the external temperature of 25°C. Then heptane (38 mL) and the seed crystals (39.1 mg) obtained in (2-4) described below were added and the mixture was stirred for 30 minutes. Subsequently, heptane (150 mL) was added over 1 hour, and then the mixture was stirred for 2 hours. The resulting solid was filtered and washed under reduced pressure (washing solvent: 24 mL of toluene + 41 mL of heptane) and dried under reduced pressure to afford 9.89 g of compound 3A·DCHA salt (3-step yield 51.1%) as a white solid.

Optical purity: 99.7 %ee
(Detection wavelength 210 nm, retention time 12.896 minutes, HPLC analysis condition method 2)
UV intensity ratio: 99.9%
(Detection wavelength 210 nm, retention time 2.880 minutes, HPLC analysis condition method 1)
$^1$H-NMR(DMSO-$d_6$,500 MHz) δ: 8.78 (brs, 1H), 7.33-7.26 (m, 5H), 6.55 (s,1H), 4.97 (s, 2H), 3.78-3.75 (s, 1H), 3.59-3.51 (m, 2H), 3.29-3.20 (m, 3H),2.86 (brs, 2H), 1.89-1.87 (m, 4H), 1.67-1.63 (m, 4 H), 1.56-1.53 (m, 2H),1.43-1.36 (m, 2H), 1.23-1.13 (m, 8H), 1.07-1.02 (m, 2H), 0.77 (t, J = 7.4 Hz,3H)

(2-4):Preparation of seed crystals of (2S)-2-(benzyloxycarbonylamino)-3-propoxy-propanoic acid·dicyclohexylamine salt (compound 3A·DCHA salt)

To the residue (0.50 g) containing compound 3A obtained by the same method as above (2-2), MTBE (4.0 mL) and DCHA (0.32 mL) were added, and the mixture was stirred at room temperature for 10 minutes to obtain compound 3A·DCHA salt (0.60 g) as a powder crystal.
Example 2 above showed that compound 3A·DCHA salt was obtained in yield of 51.1% from the starting material compound 1 in 3 steps by the present method. In addition, in the above step (2-2), the reaction mixture obtained by treating with acid contained 9% or more of compound 4A as an impurity, but the content of compound 4A could be reduced to less than 0.1% by treating with a base (aqueous sodium hydroxide solution). The present method does not require purification by column chromatography, and thus can be applicable to large scale synthesis.

Example 3: Synthesis of (2S)-2-(benzyloxycarbonylamino)-3-propoxy-propanoic acid·dicyclohexylamine salt (compound 3B·DCHA salt)

(3-1): Synthesis of (4S)-3-benzyloxycarbonyl-2,2-dimethyl-oxazolidine-4-carboxylic acid (compound 2B)

[Formula 21]

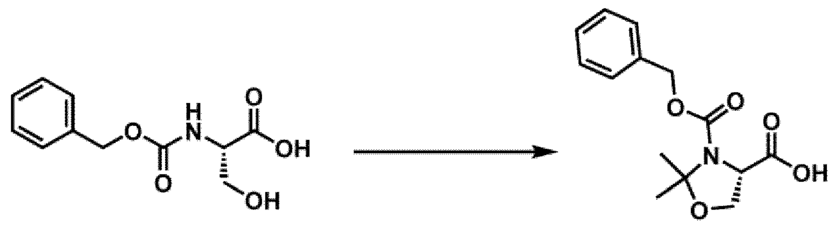

1 2B

N-Benzyloxycarbonyl-L-serine (compound 1) (3.49 g, 14.6 mmol), acetone (17.4 mL), and 2,2-dimethoxypropane (15.7 mL, 128 mmol) were added to a reaction vessel. Boron trifluoride diethyl ether complex (0.19 mL, 1.5 mmol) was added to the reaction vessel at room temperature, and then the mixture was stirred at the external temperature of 40°C for 45 minutes. Subsequently, triethylamine (0.20 mL, 1.5 mmol) was added to the mixture at room temperature and the resulting mixture was stirred for 5 minutes. The resulting residue was concentrated, and toluene (18 mL) and water (21 mL) were added and the mixture was stirred. The resulting aqueous layer was discarded, and then the resulting organic layer was washed with 5% aqueous NaCl solution (10.5 mL). Then azeotropic dehydration with the addition of toluene (10.5 mL) was repeated three times to afford 4.43 g of residue containing compound 2B (12.7mmol) (87.1% yield from HPLC and qNMR analyses).

UV intensity ratio: 96.7%
(Detection wavelength 210 nm, retention time 2.673 minutes, HPLC analysis condition method 1)

(3-2): Synthesis of (2S)-2-(benzyloxycarbonylamino)-3-isopropoxy-propanoic acid (compound 3B)

[Formula 22]

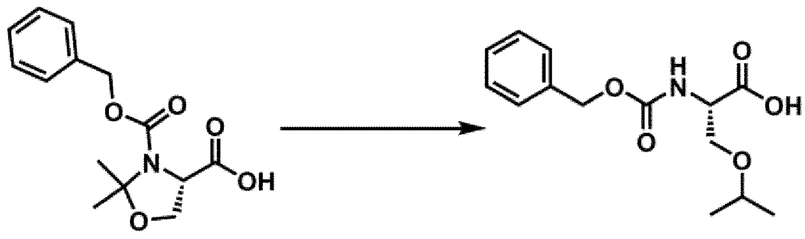

2B 3B

To the residue containing compound 2B obtained in (3-1) above (1.62 g, containing 4.64 mmol of compound 2B), triethylsilane (3.70 mL, 23.2 mmol) and toluene (6.5 mL) were added to prepare a substrate solution. A reaction vessel was replaced with nitrogen, a solution of titanium tetrachloride in toluene (1.0 M, 5.6 mL) was added. HFIP (2.89 mL, 27.8 mmol) was then added to the reaction vessel at the external temperature of -5°C. Subsequently, the substrate solution was added to the mixture in the reaction vessel at the external temperature of - 6°C. The mixture was stirred at -5°C for 45 minutes, then a 10% aqueous ammonium chloride solution (3.9 mL) was added. Subsequently, the mixture was stirred at room temperature and the aqueous layer was discarded. The organic layer obtained was washed with a 5% aqueous sodium carbonate solution twice (12 mL followed by 5 mL). The resulting aqueous layers were mixed, then a 5% aqueous sodium bisulfate monohydrate solution (42 mL) was added. The resulting aqueous solution was washed three times with toluene, and then three times with CPME. The resulting organic layer was washed with 5% aqueous NaCl solution (6 mL). The resulting organic layer was dried over sodium sulfate, filtered, and concentrated under reduced pressure. The obtained residue was purified by silica gel column chromatography (eluent: dichloromethane/methanol) to afford white solid A of compound 3B (0.496 g, 38.0% yield) and white solid B of compound 3B (0.481 g, 36.9% yield).

Optical purity (analysis of white solid B): 99.8%ee
(Detection wavelength 210 nm, retention time 13.166 minutes, HPLC analysis condition method 2)
UV intensity ratio (white solid A): 99.2%
(Detection wavelength 210 nm, retention time 2.770 minutes, HPLC analysis condition method 1)
UV intensity ratio (white solid B): 99.8%
(Detection wavelength 210 nm, retention time 2.764 minutes, HPLC analysis condition method 1)

(3-3): Synthesis of (2S)-2-(benzyloxycarbonylamino)-3-propoxy-propanoic acid·dicyclohexylamine salt (compound 3B·DCHA salt)

[Formula 23]

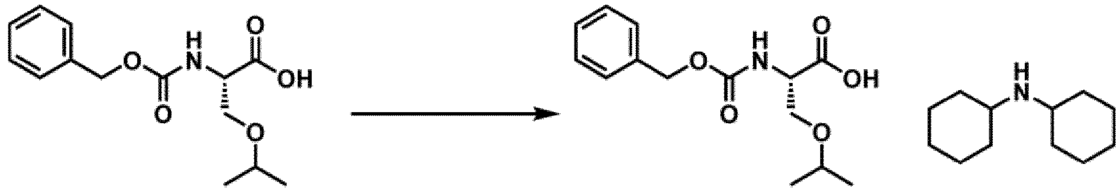

3B

3B·DCHA salt

The compound 3B (0.407 g, 1.45 mmol) obtained in (3-2) above was dissolved in MTBE (1.6 mL). Subsequently, DCHA (0.289 mL, 1.45 mmol) and heptane (3.3 mL) were added at room temperature, and the resulting precipitate was filtered under reduced pressure. The resulting solid was dried under reduced pressure to afford 0.608 g of compound 3B·DCHA salt (yield 90.8%) as a white solid.

UV intensity ratio: 99.2%
(Detection wavelength 210 nm, retention time 2.787 minutes, HPLC analysis condition method 3)
$^1$H-NMR(DMSO-$d_6$,500 MHz) δ: 8.85 (brs, 1H), 7.35-7.28 (m, 5H), 6.49 (d, J = 6.5 Hz, 1H), 5.01 (s, 2H), 3.82-3.75 (m, 1H), 3.64-3.55 (m, 2H), 3.52-3.45(m, 1H), 3.43 (brs, 1H), 2.91 (brs, 2H), 1.92 (s, 4H), 1.73-1.67 (m, 4H), 1.58(d, J = 12.5 Hz, 2H), 1.28-1.18 (m, 8H), 1.10-1.04 (m, 2H), 1.01 (d, J = 6.1 Hz,6H)

Example 3 above showed that compound 3B·DCHA salt was obtained in yield of 59% or more from the starting material compound 1 in 3 steps by the present method.

Example 4: Synthesis of (2S)-2-(benzyloxycarbonylamino)-3-propoxy-propanoic acid (compound 3C)

(4-1): Synthesis of (7S)-8-benzyloxycarbonyl-5-oxa-8-azaspiro[3.4]octane-7-carboxylic acid (compound 2C)

[Formula 24]

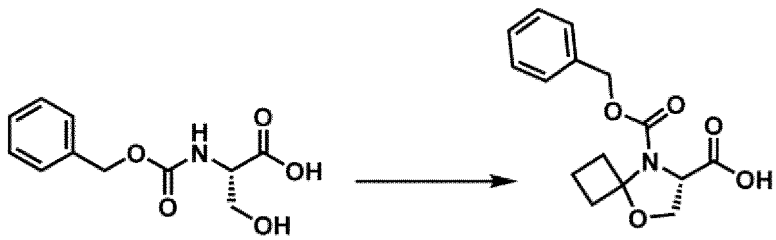

1

2C

N-Benzyloxycarbonyl-L-serine (compound 1) (2.70 g, 11.3 mmol) and MeTHF (10.8 mL) were placed in a reaction vessel at room temperature. After the a reaction vessel was replaced with nitrogen, N,O-bis(trimethylsilyl) trifluoroacetamide (3.00 mL, 11.3 mmol) was added at the external temperature of 0°C, and the mixture was stirred for 10

minutes. Subsequently, cyclobutanone (0.94 mL, 12.4 mmol) and trimethylsilyl trifluoromethanesulfonate (0.41 mL, 2.3 mmol) were added, and the resulting mixture was stirred at the external temperature of 0°C for 2 hours. A 5% aqueous dipotassium hydrogen phosphate solution (8.1 mL) was added, and then water (8.1 mL) was added at room temperature and the mixture was stirred. The aqueous layer was discarded, and then a 10% aqueous potassium carbonate solution (10.8 mL) was added. After the organic layer was discharged, 2 M hydrochloric acid (9.5 mL) and toluene (10.8 mL) were added to the resulting aqueous layer, and the resulting mixture was stirred. After discarding the aqueous layer, the resulting organic layer was washed twice with water (10.8 mL). The resulting organic layer was concentrated, then azeotropic dehydration with the addition of toluene (8.1 mL) was performed three times to afford 4.66 g of residue containing compound 2C (9.35 mmol) (82.8% yield from HPLC and qNMR analyses). UV intensity ratio: 98.9%

(Detection wavelength 210 nm, retention time 5.347 minutes, HPLC analysis condition method 4)

(4-2): Synthesis of (2S)-2-(benzyloxycarbonylamino)-3- (cyclobutoxy)propanoic acid (compound 3C)

[Formula 25]

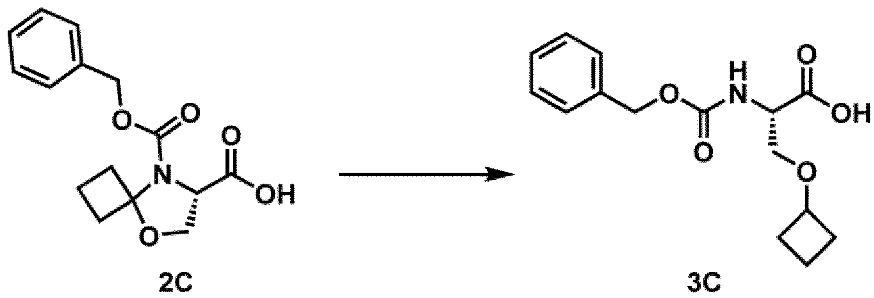

2C 3C

To the residue (4.66 g) containing compound 2C (9.35 mmol) obtained in (4-1) above, triethylsilane (4.53 mL, 28.4 mmol) and chlorobenzene (5 mL) were added to prepare a substrate solution. After the atmosphere in a reaction vessel was replaced with nitrogen, chlorobenzene (6.9 mL) was added. Subsequently, titanium tetrachloride (1.77 mL, 16.1 mmol), 2,2,2-trifluoroethanol (2.03 mL, 28.4 mmol), and tetraisopropyl orthotitanate (1.53 mL, 5.20 mmol) were added at the external temperature of -16°C, and the mixture was stirred. Then, the substrate solution prepared above was added at the external temperature of -16°C, and the mixture was stirred for 1 hour and 15 minutes while setting the external temperature to -6°C.

(HPLC analysis using HPLC analysis condition method 1 using a portion of the reaction solution obtained here resulted in a peak area ratio of compound 3C (retention time 3.031 minutes) : compound 4C (retention time 2.572 minutes) of 87.7 : 12.3.)

(Structure of compound 4C)

[Formula 26]

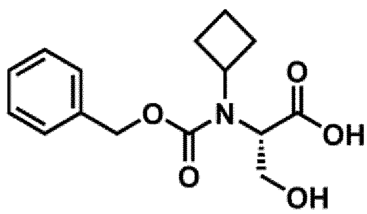

4C

A 10% aqueous ammonium chloride solution (13.8 mL) was then added, and the resulting mixture was stirred at room temperature. After discarding the aqueous layer, a 5% aqueous citric acid solution (13.8 mL) and MTBE (8.3 mL)

were added to the resulting organic layer. After stirring the mixture, the aqueous layer was discarded, and then the resulting organic layer was washed with water (13.8 mL). A 2 M aqueous sodium hydroxide solution (13.8 mL) was added to the resulting organic layer, and the resulting mixture was stirred for 1.5 hours.

(HPLC analysis using HPLC analysis condition method 1 using a portion of the aqueous layer obtained here resulted in a peak area ratio of compound 3C (retention time 3.004 minutes) : compound 4C (retention time 2.548 minutes) of 99.4 : 0.6.)

After discarding the organic layer, the resulting aqueous layer was washed with MTBE (13.8 mL). To the resulting aqueous layer, 2 M hydrochloric acid (13.5 mL) and toluene (27.6 mL) were added and the mixture was stirred. The aqueous layer was discarded, and then the resulting organic layer was washed with water (13.8 mL). Then, azeotropic dehydration with the addition of toluene (8.3 mL) was performed three times to afford a residue (6.18 g) containing compound 3C. To the obtained residue, toluene (15.2 mL) was added, and the seed crystals (9.7 mg) of compound 3C obtained in (4-3) below were added at room temperature and the mixture was stirred for 20 minutes. Heptane (19.6 mL) was then added dropwise over 1 hour while stirring and then the mixture was stirred for 1.5 hours. The resulting precipitate was filtered under reduced pressure and the obtained solid was dried under reduced pressure to afford 1.78 g of compound 3C (yield 64.9%) as a white solid.

Optical purity: 99.9%ee
(Detection wavelength 210 nm, retention time 13.647 minutes, HPLC analysis condition method 2)
UV intensity ratio: 99.9%
(Detection wavelength 210 nm, retention time 2.986 minutes, HPLC analysis condition method 1)
$^1$H-NMR(DMSO-$d_6$,500 MHz) $\delta$: 12.77 (s, 1H), 7.51 (d, J = 8.0 Hz, 1H),7.37-7.30 (m, 5H), 5.04 (s, 2H), 4.18-4.14 (m, 1H), 3.92-3.87 (m, 1H),3.54-3.51 (m, 2H), 2.12-2.07 (m, 2H), 1.84-1.76 (m, 2H), 1.63-1.57 (m, 1H),1.47-1.40 (m, 1H)

(4-3): Preparation of seed crystals of (2S)-2-(benzyloxycarbonylamino)-3-(cyclobutoxy)propanoic acid (compound 3C)

To a residue (0.87 g) containing compound 3C obtained by the same method as above (4-2), toluene (1.0 mL) and heptane (1.0 mL) were added, and the mixture was stirred at room temperature until precipitate formed. Heptane was further added and the mixture was stirred additionally, and then the resulting precipitate was filtered under reduced pressure to afford compound 3C (0.46 g) as a powder crystal.

Example 4 above showed that compound 3C was obtained in yield of 53% or more from the starting material compound 1 in 2 steps by the present method. In addition, in the above step (4-2), the reaction mixture contained 12% or more of compound 4A as an impurity, but the content of compound 4A could be reduced to less than 1% by treating with a base (aqueous sodium hydroxide solution). The present method does not require purification by column chromatography, and thus can be applicable to large scale synthesis.

Example 5: Synthesis of (2S)-2-(benzyloxycarbonylamino)-3-isopentyloxy-propanoic acid·dicyclohexylamine salt (compound 3D·DCHA salt)

(5-1): Synthesis of (4S)-3-benzyloxycarbonyl-2-isobutyl-oxazolidine-4-carboxylic acid (compound 2D)

[Formula 27]

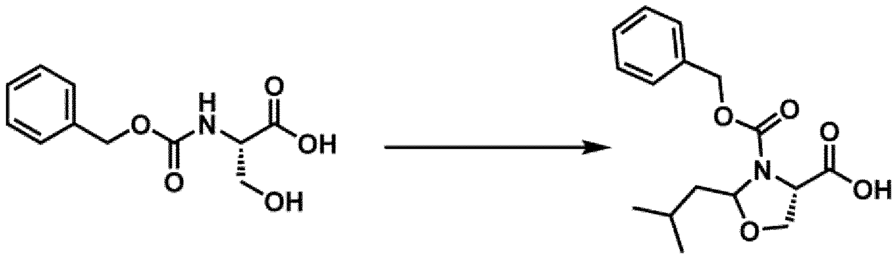

1 2D

N-Benzyloxycarbonyl-L-serine (compound 1) (3.04 g, 12.7 mmol) and magnesium sulfate (1.51 g, 12.5 mmol) were added to a reaction vessel at the external temperature of 25°C. After the atmosphere in a reaction vessel was

replaced with nitrogen, toluene (15 mL), isovaleraldehyde (2.05 mL, 19.1 mmol), and boron trifluoride tetrahydrofuran complex (0.28 mL, 2.5 mmol) were added, and the mixture was stirred at 25°C for 2 hours. Triethylamine (0.35 mL, 2.5 mmol) was then added, and the resulting mixture was stirred for 5 minutes. The mixture was washed with water (15 mL x 2) and then with 5% aqueous NaCl solution (15 mL), then azeotropic dehydration of the resulting organic layer with the addition of toluene (15 mL) was performed twice to afford 7.47 g of a residue containing compound 2D.

UV intensity ratio (as diastereomer mixture): 95.8%

(Detection wavelength 210 nm, retention time 3.224 and 3.250 minutes, HPLC analysis condition method 5)

(5-2): Synthesis of (2S)-2-(benzyloxycarbonylamino)-3-isopentyloxy-propanoic acid (compound 3D)

[Formula 28]

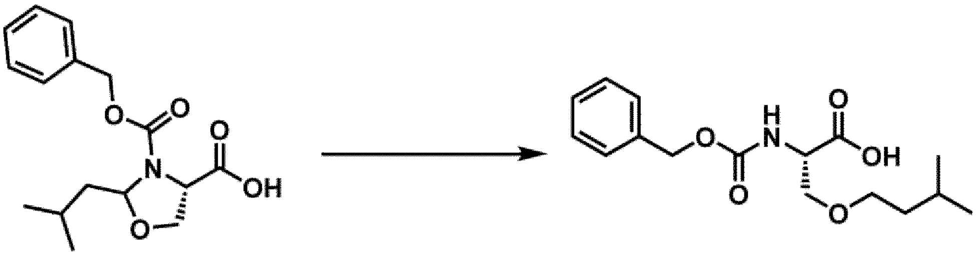

2D

3D

To the residue (7.47 g) containing compound 2D obtained in (5-1) above, triethylsilane (5.07 mL, 31.8 mmol) was added to prepare a substrate solution. After the atmosphere in a reaction vessel was replaced with nitrogen, chlorobenzene (7.5 mL) and titanium tetrachloride (2.38 mL, 21.6 mmol) were added. Then, 2,2,2-trifluoroethanol (2.72 mL, 38.1 mmol) and tetraisopropyl orthotitanate (2.05 mL, 7.0 mmol) were added to the reaction vessel at the external temperature of -15°C, and the mixture was stirred. The substrate solution prepared above was added to the mixture in the reaction vessel at the external temperature of -15°C, and the resulting mixture was stirred for 4 hours at the external temperature of 0°C. The resulting mixture was stirred for 1 hour additionally at the external temperature of 10°C and then a 5% aqueous ammonium chloride solution (15 mL) was added at the external temperature of -10°C. The resulting mixture was stirred for 5 minutes at the external temperature of 25°C. After discarding the aqueous layer, the resulting organic layer was washed with a 5% aqueous citric acid solution (15 mL) and then with water (15 mL). The aqueous layer was discharged, and then a 2 M aqueous sodium hydroxide solution (9 mL) and MTBE (9 mL) were added to the resulting organic layer and the mixture was stirred at the external temperature of 25°C for 3.5 hours. A 2 M aqueous sodium hydroxide solution (6 mL) was added to the mixture, and then the resulting mixture was stirred for 0.5 hours additionally. After discarding the organic layer, the resulting aqueous layer was washed with MTBE (15 mL). To the resulting aqueous layer, 2 M hydrochloric acid (15 mL) and MeTHF (30 mL) were added, and the mixture was stirred for 10 minutes. The aqueous layer was discarded, and then the resulting organic layer was washed with a 5% aqueous NaCl solution (15 mL). Then, azeotropic dehydration of the resulting organic layer with the addition of MeTHF (15 mL) was performed three times to afford a MeTHF solution (9 mL) containing compound 3D.

UV intensity ratio: 88.6%

(Detection wavelength 210 nm, retention time 3.290 minutes, HPLC analysis condition method 5)

(5-3): Synthesis of (2S)-2-(benzyloxycarbonylamino)-3-isopentyloxy-propanoic acid·dicyclohexylamine salt (compound 3D·DCHA salt)

[Formula 29]

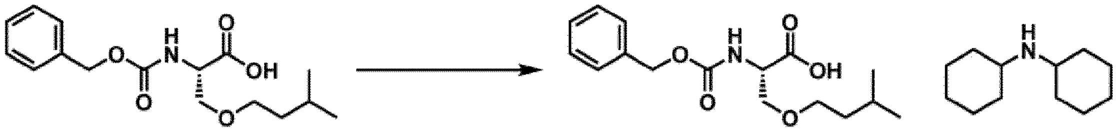

3D

3D·DCHA salt

To the MeTHF solution (9 mL) containing compound 3D obtained in (5-2) above, DCHA (2.53 mL, 12.7 mmol) and heptane (9 mL) were added while stirring at the external temperature of 25°C. The resulting solid was filtered and washed under reduced pressure (washing solution: 10 mL of MeTHF + 40 mL of heptane), and dried under reduced pressure to afford 3.94 g of compound 3D·DCHA salt (3-step yield 63.2%) as a white solid.

Optical purity: 99.9%ee
(Detection wavelength 210 nm, retention time 15.933 minutes, HPLC analysis condition method 2)
UV intensity ratio: 99.0%
(Detection wavelength 210 nm, retention time 3.232 minutes, HPLC analysis condition method 5)
$^1$H-NMR(DMSO-$d_6$,500 MHz) $\delta$: 8.83 (brs, 1H), 7.37-7.29 (m, 5H), 6.58 (s,1H), 5.00 (s, 2H), 3.81-3.79 (m, 1H), 3.63-3.55 (m, 2H), 3.37-3.32 (m, 3H),2.91 (brs, 2H), 1.91 (brs, 4H), 1.70 (brs, 4H), 1.63-1.57 (m, 2H), 1.35-1.30(m, 2H), 1.27-1.21 (m, 8H), 1.13-1.04 (m, 2H), 0.86 (t, J = 6.7 Hz, 1H), 0.83(dd, J = 6.7, 1.7 Hz, 6H)

Example 5 above showed that compound 3D·DCHA salt was obtained in yield of 63.2% from the starting material compound 1 in 3 steps by the present method. The present method does not require purification by column chromatography, and thus can be applicable to large scale synthesis.

Example 6: Synthesis of (2S,3R)-2-(benzyloxycarbonylamino)-3-isopropoxy-butanoic acid·dicyclohexylamine salt (compound 7B·DCHA salt)

(6-1): Synthesis of (4S,5R)-3-benzyloxycarbonyl-2,2,5-trimethyl-oxazolidine-4-carboxylic acid (compound 6B)

[Formula 30]

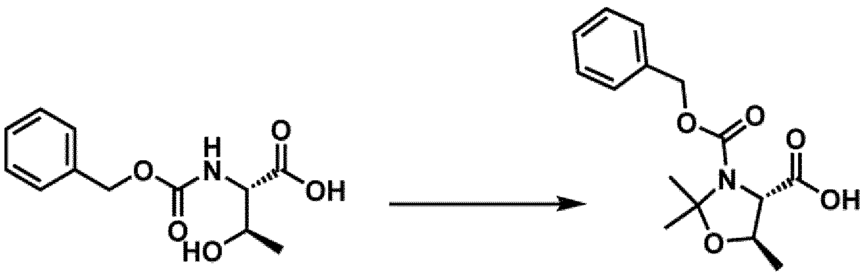

5

6B

N-benzyloxycarbonyl-L-threonine (compound 5) (1.00 g, 3.96 mmol), acetone (5 mL), and 2,2-dimethoxypropane (1.94 mL, 15.8 mmol) were added to a reaction vessel at the external temperature of 25°C, and the atmosphere in a reaction vessel was replaced with nitrogen. Boron trifluoride tetrahydrofuran complex (0.087 mL, 0.79 mmol) was added, and the mixture was stirred at 40°C for 0.5 hours. Triethylamine (0.11 mL, 0.79 mmol) was then added at the external temperature of 25°C, and the mixture was stirred for 5 minutes. The resulting residue was concentrating the mixture, toluene (5 mL) was added to the resulting residue and the residue was further concentrated. To the resulting residue, toluene (3 mL) and water (5 mL) were added, and the mixture was stirred at 25°C for 10 minutes. After discarding the aqueous layer, the resulting organic layer was washed with a 5% aqueous NaCl solution (5 mL). Azeotropic dehydration of the organic layer with the addition of toluene (5 mL) was performed three times to obtain 3.39 g of a residue containing compound 6B.

UV intensity ratio (as diastereomer mixture): 98.3%

(Detection wavelength 210 nm, retention time 2.783 minutes, HPLC analysis condition method 3)

(6-2): Synthesis of (2S,3R)-2-(benzyloxycarbonylamino)-3-isopropoxy-butanoic acid (compound 7B)

[Formula 31]

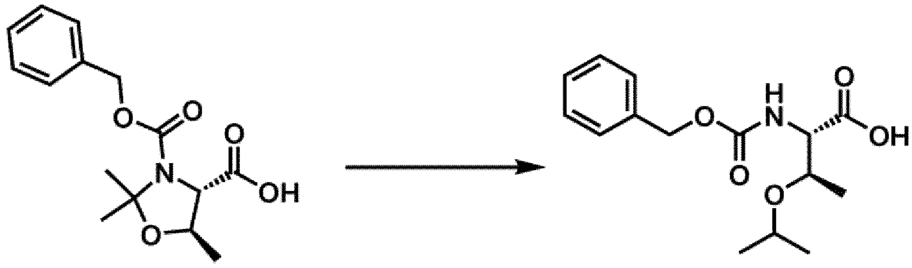

6B 7B

To the residue (3.39 g) containing compound 6B obtained in (6-1) above, triethylsilane (1.58 mL, 9.89 mmol) was added to prepare a substrate solution. After the atmosphere in a reaction vessel was replaced with nitrogen, chlorobenzene (2.5 mL) and titanium tetrachloride (0.74 mL, 6.7 mmol) were added. Then 2,2,2-trifluoroethanol (0.85 mL, 11.9 mmol) and tetraisopropyl orthotitanate (0.64 mL, 2.2 mmol) were added to the reaction vessel at the external temperature of -15°C, and the mixture was stirred. The substrate solution prepared above was then added to the mixture in the reaction vessel at the external temperature of -15°C, and the resulting mixture was stirred for 2 hours at the external temperature of 0°C. (Compound 8B was not detected by HPLC analysis using HPLC analysis condition method 3 using a portion of the reaction solution obtained here.)

(Structure of compound 8B)

[Formula 32]

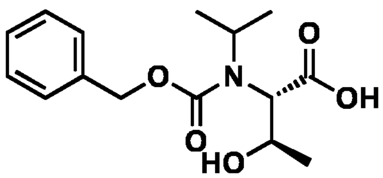

8B

A 5% aqueous ammonium chloride solution (5 mL) was added at the external temperature of -10°C, and then the external temperature was set to 25°C. Subsequently, MTBE (3 mL) was added and the mixture was stirred for 10 minutes. After discarding the aqueous layer, the resulting organic layer was washed with a 5% aqueous citric acid solution (5 mL) and then with water (5 mL). The aqueous layer was discarded, and then a 1 M aqueous sodium hydroxide solution (5 mL) was added to the resulting organic layer, and the mixture was stirred at the external temperature of 25°C for 2 hours. After discarding the organic layer, the resulting aqueous layer was washed with MTBE (5 mL). To the resulting aqueous layer, 2 M hydrochloric acid (2.5 mL) and MTBE (10 mL) were added, and the mixture was stirred for 10 minutes. After discarding the aqueous layer, the resulting organic layer was washed with a 5% aqueous NaCl solution (5 mL). Azeotropic dehydration of the resulting organic layer with the addition of MTBE (10 mL) was performed twice to afford a MTBE solution (4 mL) containing compound 7B.

UV intensity ratio: 97.7%

(Detection wavelength 210 nm, retention time 2.842 minutes, HPLC analysis condition method 3)

(6-3): Synthesis of (2S,3R)-2-(benzyloxycarbonylamino)-3-isopropoxy-butanoic acid·dicyclohexylamine salt (compound 7B·DCHA salt)

[Formula 33]

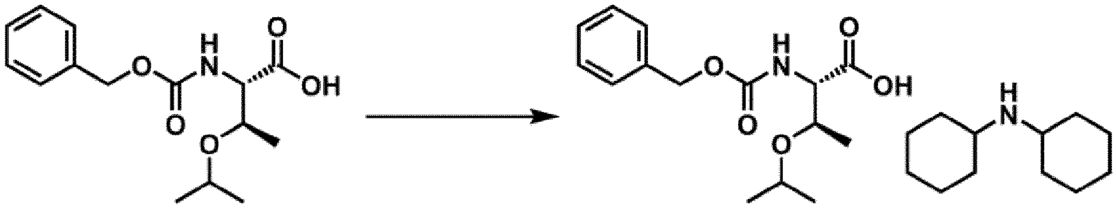

7B

7B · DCHA salt

To the MTBE solution (4 mL) containing compound 7B obtained in (6-2) above, DCHA (0.788 mL, 3.96 mmol) and heptane (4 mL) were added at room temperature while stirring. The resulting solid was filtered and washed under reduced pressure (washing solution: 2.5 mL of MTBE + 7.5 mL of heptane), and dried under reduced pressure to afford 1.29 g of compound 7B·DCHA salt (3-step yield 68.2%) as a white solid.

Diastereomeric excess (7B and diastereomer 7B'): 99.7%de
(Detection wavelength 210 nm, retention time 13.367 minutes, HPLC analysis condition method 2)

[Formula 34]

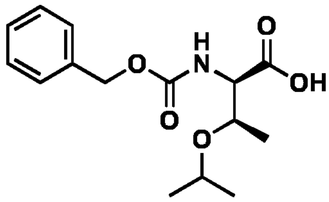

7B'

UV intensity ratio: 99.1%
(Detection wavelength 210 nm, retention time 2.851 minutes, HPLC analysis condition method 3)
$^1$H-NMR(DMSO-$d_6$,500 MHz) δ: 7.39-7.29 (m, 5H), 6.07 (d, J = 7.2Hz, 1H), 5.02 (s, 2H), 3.97-3.92 (m, 1H), 3.66-3.59 (m, 2H), 3.37 (brs, 2H),2.87 (brs, 2H), 1.92-1.90 (m, 4H), 1.77-1.66 (m, 4H), 1.59-1.57 (m,2H),1.27-1.15 (m, 8H), 1.11-1.05 (m, 2H), 1.02 (d, J = 6.5 Hz, 3H), 1.00 (d, J =6.1 Hz, 3H), 0.97-0.93 (d, J = 6.1 Hz, 3H)

Example 6 above showed that compound 7B·DCHA salt was obtained in yield of 68.2% from the starting material compound 5 in 3 steps by the present method. The present method does not require purification by column chromatography, and thus can be applicable to large scale synthesis.

Example 7: Synthesis of (2S)-2-(9H-fluoren-9-ylmethoxycarbonylamino)-3-isopropoxy-propanoic acid (compound 11B)

(7-1): Synthesis of (4S)-3-(9H-fluoren-9-ylmethoxycarbonyl)-2,2-dimethyl-oxazolidine-4-carboxylic acid (compound 10B)

[Formula 35]

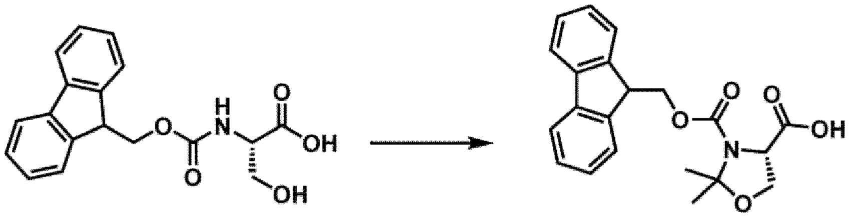

9 10B

N-[(9H-fluoren-9-ylmethoxy)carbonyl]-L-serine (compound 9) (1.02 g, 3.10 mmol), acetone (5.1 mL), and 2,2-dimethoxypropane (1.52 mL, 12.4 mmol) were added to the reaction vessel at the external temperature of 25°C, and the atmosphere in a reaction vessel was replaced with nitrogen. Boron trifluoride tetrahydrofuran complex (0.068 mL, 0.62 mmol) was added, and the mixture was stirred at 40°C for 1 hour. Subsequently, triethylamine (0.087 mL, 0.62 mmol) was added at the external temperature of 25°C, and the mixture was stirred for 5 minutes. After concentration of the residue, dichloromethane (5 mL) and water (5 mL) were added to the resulting concentrate, and the resulting mixture was stirred at room temperature for 10 minutes. The aqueous layer was discarded, the resulting organic layer was dried over adding magnesium sulfate. Filtration followed by concentration under reduced pressure afforded a residue containing compound 10B, then dichloromethane was added to the residue to obtain a dichloromethane solution (3 mL) containing compound 10B.

UV intensity ratio (as diastereomer mixture): 84.9%

(Detection wavelength 210 nm, retention time 3.283 minutes, HPLC analysis condition method 3)

(7-2): Synthesis of (2S)-2-(9H-fluoren-9-ylmethoxycarbonylamino)-3-isopropoxy-propanoic acid (compound 11B)

[Formula 36]

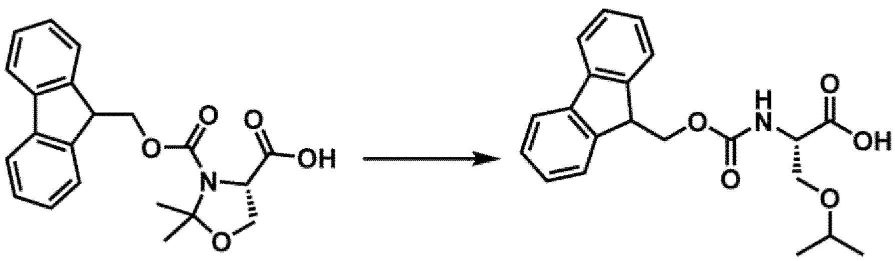

10B 11B

To the dichloromethane solution (3 mL) containing compound 10B obtained in (7-1) above, triethylsilane (1.24 mL, 7.76 mmol) was added to prepare a substrate solution. After the atmosphere in a reaction vessel was replaced with nitrogen, dichloromethane (2.5 mL) and titanium tetrachloride (0.41 mL, 3.7 mmol) were added. The substrate solution prepared above was added to the reaction vessel at the external temperature of -15°C, and the resulting mixture was stirred for 2 hours at the external temperature of 0°C. (HPLC analysis using HPLC analysis condition method 3 using a portion of the reaction solution obtained here resulted in an area ratio of compound 11B (retention time 3.424 minutes) : compound 12B (retention time 3.022 minutes) of 98.2 : 1.8.)

[Formula 37]

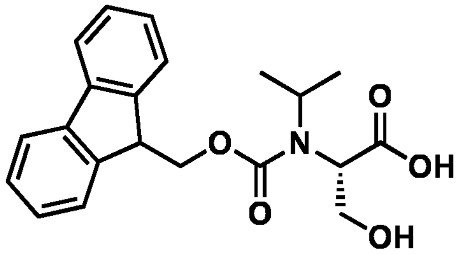

12B

A 5% aqueous ammonium chloride solution (5 mL) was added at the external temperature of -10°C, then the external temperature of 25°C. MTBE (5 mL) was added, and the mixture was stirred for 5 minutes additionally. After discarding the aqueous layer, the resulting organic layer was washed with a 5% aqueous citric acid solution (5 mL) and then with 5% aqueous NaCl solution (5 mL). After discarding the aqueous layer, azeotropic dehydration of the organic layer was performed by the addition of MTBE. The obtained residue was purified by silica gel column chromatography (eluent: dichloromethane/methanol) to afford 0.783 g of compound 11B (2-step yield 68.3%) as a white solid.

Optical purity: 99.8 %ee
(Detection wavelength 210 nm, retention time 17.398 minutes, HPLC analysis condition method 2)
UV intensity ratio: 91.8%
(Detection wavelength 210 nm, retention time 3.408 minutes, HPLC analysis condition method 3)
$^1$H-NMR(DMSO-$d_6$,500 MHz) δ: 12.74 (brs, 1H), 7.90 (d, J = 7.6 Hz, 2H),7.75 (d, J = 7.6 Hz, 2H), 7.56 (d, J = 8.4 Hz, 1H), 7.44-7.41 (m, 2H),7.34-7.31 (m, 2H), 4.31-4.27 (m, 2H), 4.25-4.21 (m, 1H), 4.17-4.14 (m, 1H),3.64-3.53 (m, 3H), 1.07 (t, J = 5.0 Hz, 6H)

Example 7 above showed that compound 11B was obtained in yield of 68.3% from the starting material compound 9 in 2 steps by the present method.

Example 8: Synthesis of (2S)-2-[benzyloxycarbonyl(methyl)amino]-3-(cyclobutoxy)propanoic acid·dicyclohexylamine salt (compound 14C·DCHA salt)

(8-1): Synthesis of (7S)-8-benzyloxycarbonyl-5-oxa-8-azaspiro[3.4]octane-7-carboxylic acid (compound 2C)

[Formula 38]

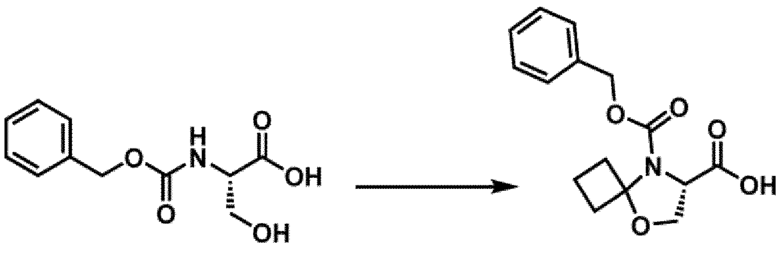

1 2C

N-Benzyloxycarbonyl-L-serine (compound 1) (5.13 g, 21.4 mmol), toluene (10.3 mL) and acetonitrile (10.3 mL) were added to the reaction vessel at room temperature. After the atmosphere in a reaction vessel was replaced with nitrogen, N,O-bis(trimethylsilyl)acetamide (4.8 mL, 19.6 mmol) was added at room temperature, and the mixture was stirred for 15 minutes. Subsequently, cyclobutanone (1.78 mL, 23.6 mmol) and trimethylsilyl trifluoromethanesulfonate (3.49 mL, 19.3 mmol) were added, and the mixture was stirred at room temperature for 2.5 hours. A 5% aqueous dipotassium hydrogen phosphate solution (15.5 mL) was added and the mixture was stirred. After stirring with the addition of CPME (10.3 mL), the aqueous layer was discarded. The resulting organic layer was treated with a 10% aqueous potassium carbonate solution (26 mL). The resulting aqueous layer was collected, and 2 M hydrochloric acid (21 mL) and toluene (52 mL) were added, then the mixture was stirred. After discarding the aqueous layer, the resulting organic layer

was washed with water (15.5 mL). The resulting organic layer was concentrated, and then azeotropic dehydration with the addition of toluene (15.5 mL) was performed to afford 7.09 g of residue containing compound 2C.

UV intensity ratio: 95.8%

(Detection wavelength 210 nm, retention time 5.381 minutes, HPLC analysis condition method 4)

(8-2): Synthesis of (2S)-2-(benzyloxycarbonylamino)-3-(cyclobutoxy)propanoic acid (compound 3C)

[Formula 39]

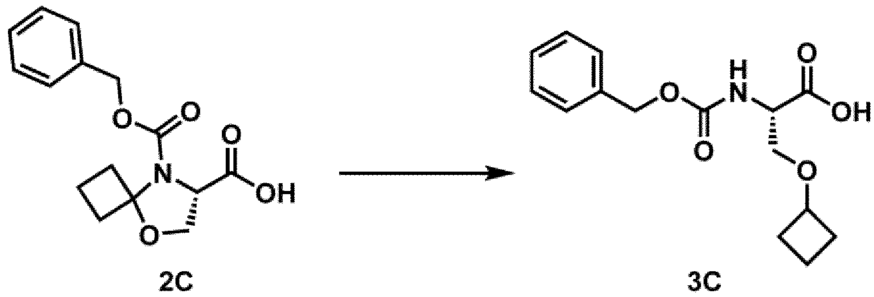

2C 3C

3.47 g of 7.09 g of the residue containing compound 2C obtained in (8-1) above was taken out, then triethylsilane (3.57 mL) and chlorobenzene (3.9 mL) were added thereto to prepare a substrate solution. After the atmosphere in a reaction vessel was replaced with nitrogen, chlorobenzene (5.4 mL), titanium tetrachloride (1.36 mL), tetraisopropyl orthotitanate (1.20 mL), and 2,2,2-trifluoroethanol (2.13 mL) were added, and the mixture was stirred. Then, the substrate solution prepared above was added to the reaction vessel at the external temperature of -6°C, and the mixture was stirred for 1.5 hours. A 10% aqueous ammonium chloride solution (11 mL) was then added, and the mixture was stirred at room temperature. After discarding the aqueous layer, a 5% aqueous citric acid solution (11 mL) and MTBE (11 mL) were added to the resulting organic layer and the mixture was stirred. After the mixture was stirred, the aqueous layer was discarded, and the resulting organic layer was washed with water (11 mL). The aqueous layer was discarded, and then a 2 M aqueous sodium hydroxide solution (11 mL) was added to the organic layer, and the resulting mixture was stirred for 2 hours. After discarding the organic layer, the resulting aqueous layer was washed with MTBE (11 mL). To the resulting aqueous layer, 2 M hydrochloric acid (10 mL) and toluene (17.5 mL) were added and the mixture was stirred. After discarding the aqueous layer, the resulting organic layer was washed with water (11 mL). Then, azeotropic dehydration of the organic layer with the addition of toluene (7 mL) was performed to obtain a residue (3.59 g) containing compound 3C. To the obtained residue, toluene (10.8 mL) was added, and the seed crystals (6.4 mg) of compound 3C obtained in (4-3) above were added at room temperature and the mixture was stirred for 20 minutes. Heptane (13.3 mL) was then added while stirring and then the mixture was stirred for 1 hour. The resulting precipitate was filtered under reduced pressure and the obtained solid was dried under reduced pressure to afford compound 3C (1.19 g) as a white solid.

UV intensity ratio: 99.9%

(Detection wavelength 210 nm, retention time 3.039 minutes, HPLC analysis condition method 1)

(8-3): Synthesis of (2S)-2-(benzyloxycarbonylamino)-3-(cyclobutoxy)propanoic acid (compound 3C)

[Formula 40]

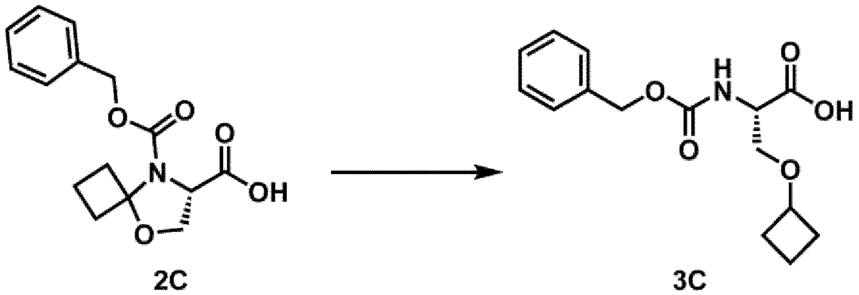

2C 3C

3.31 g of 7.09 g of the residue containing compound 2C obtained in (8-1) above was taken out, then triethylsilane (3.41 mL) and chlorobenzene (3.7 mL) were added thereto to prepare a substrate solution. After the atmosphere in a reaction vessel was replaced with nitrogen, chlorobenzene (5.2 mL) and titanium tetrachloride (1.33 mL) were added. Then 2,2,2-trifluoroethanol (1.53 mL) and tetraisopropyl orthotitanate (1.15 mL) were added to the reaction vessel at the external temperature of -16°C, and the mixture was stirred. Then, the substrate solution prepared above was added to the mixture in the reaction vessel at the external temperature of -16°C. The resulting mixture was stirred at the external temperature of -6°C for 3 hours, then a 10% aqueous ammonium chloride solution (10.4 mL) was added, and the mixture was stirred at room temperature. The aqueous layer was discarded, and then a 5% aqueous citric acid solution (10.4 mL) and toluene (10.4 mL) were added to the resulting organic layer and the resulting mixture was stirred. After discarding the aqueous layer, the resulting organic layer was washed with water (10.4 mL). The aqueous layer was discharged, and then a 2 M aqueous sodium hydroxide solution (10.4 mL) was added to the resulting organic layer, and the mixture was stirred for 1.5 hours. After discarding the organic layer, the resulting aqueous layer was washed with MTBE (10.4 mL). To the resulting aqueous layer, 2 M hydrochloric acid (9.5 mL) and toluene (21 mL) were added and the mixture was stirred. The aqueous layer was discarded, and then the resulting organic layer was washed with water (10.4 mL). Then, azeotropic dehydration of the organic layer with the addition of toluene (6 mL) was performed to obtain a residue (4.72 g) containing compound 3C. To the obtained residue, toluene (10.1 mL) was added, and the seed crystal (6.8 mg) of compound 3C obtained in (4-3) above was added at room temperature and the mixture was stirred for 30 minutes. Heptane (13.7 mL) was then added while stirring and then the mixture was stirred for 6 hours. The resulting precipitate was filtered under reduced pressure and the obtained solid was dried under reduced pressure to afford compound 3C (1.20 g) as a white solid.

UV intensity ratio: 99.9%

(Detection wavelength 210 nm, retention time 3.042 minutes, HPLC analysis condition method 1)

(8-4): Synthesis of benzyl (4S)-4-(cyclobutoxymethyl)-5-oxo-oxazolidine-3-carboxylic acid (compound 13C)

[Formula 41]

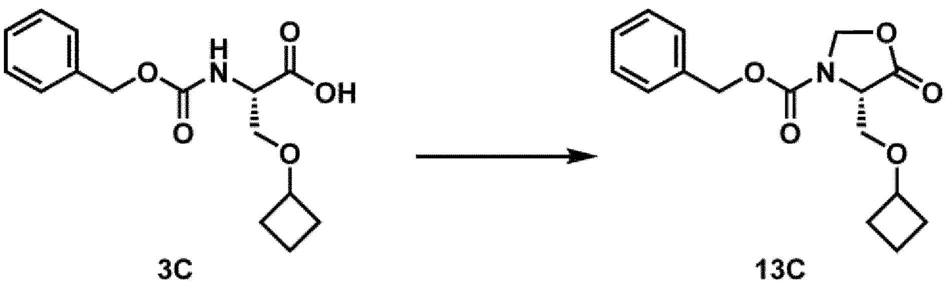

3C 13C

To the reaction vessel, paraformaldehyde (1.26 g, 90% content, 37.7 mmol), compound 3C (1.85 g, 6.30 mmol) obtained in (8-2) and (8-3) above, magnesium sulfate (0.76 g, 6.3 mmol), and toluene (9.2 mL) were added. After the atmosphere in a reaction vessel was replaced with nitrogen, a boron trifluoride diethyl ether complex (0.80 mL, 6.3 mmol) was added to the mixture at room temperature, and then the mixture was stirred for 2 hours. The resulting mixture was filtered and the filtrate was washed twice with a 10% aqueous potassium carbonate solution (9.2 mL) and once with water (9.2 mL). The resulting organic layer was filtered and the resulting filtrate was washed with water (9.2 mL). The resulting organic layer was concentrated, and subsequent azeotropic dehydration with the addition of toluene (5.5 mL) afforded

3.75 g of residue containing compound 13C.

UV intensity ratio: 98.5%

(Detection wavelength 210 nm, retention time 3.620 minutes, HPLC analysis condition method 1)

(8-5): Synthesis of (2S)-2-[benzyloxycarbonyl(methyl)amino]-3-(cyclobutoxy)propanoic acid (compound 14C)

[Formula 42]

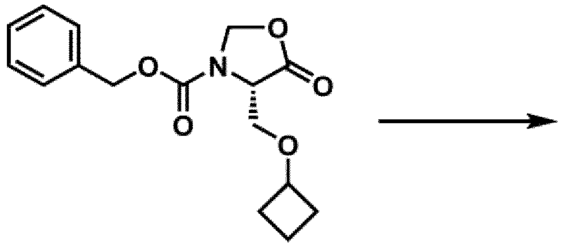

13C 14C

2.92 g of 3.75 g of the residue containing compound 13C obtained in (8-4) above was taken out and placed in a reaction vessel, then triethylsilane (1.69 mL, 10.6 mmol), and chlorobenzene (3.3 mL) were added. After the atmosphere in a reaction vessel was replaced with nitrogen, trimethylsilyl trifluoromethanesulfonate (0.64 mL, 3.5 mmol) were added to the mixture at the external temperature of -3°C, and the resulting mixture was stirred at room temperature for 2 hours. A 5% aqueous dipotassium hydrogen phosphate solution (3 mL) was added at the external temperature of -3°C, and the mixture was stirred at room temperature. After discarding the aqueous layer, a 10% aqueous potassium carbonate solution (5.4 mL) was added to the resulting organic layer and the mixture was stirred. After the organic layer was discharged, 2 M hydrochloric acid (4.3 mL) and toluene (8.6 mL) were added to the resulting aqueous layer, and the mixture was stirred. After discarding the aqueous layer, the resulting organic layer was washed with water (5.4 mL). Azeotropic dehydration of the organic layer with the addition of toluene (3.2 mL) was performed to obtain a residue (1.09 g) containing compound 14C.

UV intensity ratio: 99.6%

(Detection wavelength 210 nm, retention time 3.117 minutes, HPLC analysis condition method 1)

(8-6): Synthesis of (2S)-2-[benzyloxycarbonyl(methyl)amino]-3-(cyclobutoxy)propanoic acid·dicyclohexylamine salt (compound 14C·DCHA salt)

[Formula 43]

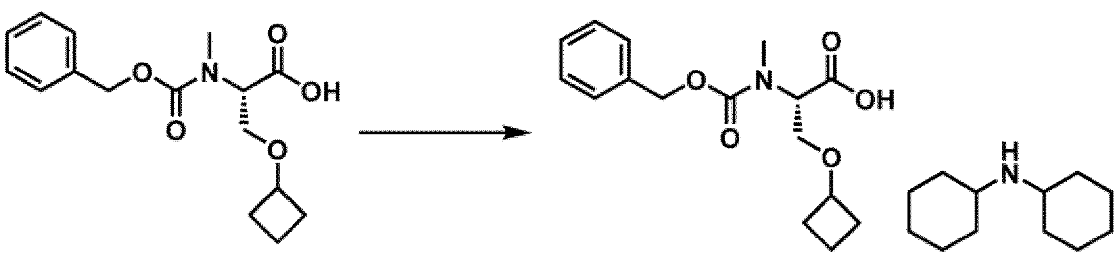

14C 14C·DCHA salt

To the residue (1.09 g) containing compound 14C obtained in (8-5) above, MTBE (3.8 mL) was added. DCHA

(0.63 mL) was then added at room temperature while stirring, and then MTBE (3.8 mL) was added additionally. The resulting solid formed was filtered, washed under reduced pressure (washing solution: 5 mL of MTBE) and dried under reduced pressure to afford compound 14C·DCHA salt (1.46 g) as a white solid.

UV intensity ratio: 99.8%
(Detection wavelength 210 nm, retention time 3.110 minutes, HPLC analysis condition method 1)
$^1$H-NMR(DMSO-$d_6$,500 MHz) δ: 8.70 (brs, 1H), 7.38-7.28 (m, 5H),5.09-4.98 (m, 2H), 4.51-4.43 (m, 1H), 3.92-3.84 (m, 1H), 3.67 (dd, J = 10.8,4.1 Hz, 1H), 3.52-3.44 (m, 2H), 2.91 (brs, 2H), 2.78 (d, J = 20.9 Hz, 3H),2.11-2.05 (m, 2H), 1.92 (brs, 4H), 1.81-1.69 (m, 6H), 1.61-1.57 (m, 3H),1.47-1.36 (m, 1H), 1.31-1.18 (m, 8H), 1.10-1.05 (m, 2H)

Example 8 above showed that compound 14C·DCHA salt was obtained from the starting material compound 1 in 5 steps by the present method. The present method does not require purification by column chromatography, and thus can be applicable to large scale synthesis. It was also found that the method of the present invention is applicable to the synthesis of O-substituted serine derivatives having N-substituents.

Example 9: Synthesis of (2S)-2-(benzyloxycarbonylamino)-3-isobutyloxypropanoic acid (compound 3E)

(9-1): Synthesis of (4S)-3-benzyloxycarbonyl-2-isopropyloxazolidine-4-carboxylic acid (compound 2E)

[Formula 44]

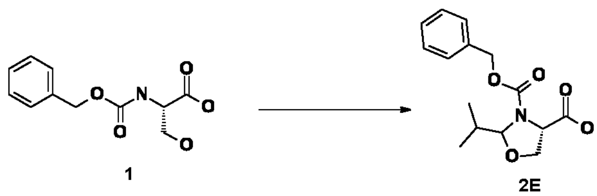

1 2E

After the atmosphere in a reaction vessel was replaced with nitrogen gas, N-benzyloxycarbonyl-L-serine (compound 1) (4.02 g, 16.8 mmol), magnesium sulfate (2.02 g, 16.8 mmol), and toluene (20 mL) were added to the reaction vessel at room temperature. Then isobutylaldehyde (2.30 mL, 25.2 mmol) and boron trifluoride tetrahydrofuran complex (0.37 mL, 3.4 mmol) were added to the reaction vessel, and the mixture was stirred at 25°C for 2 hours. Subsequently, triethylamine (0.47 mL, 3.4 mmol) was added and the mixture was stirred for 5 minutes. Water (20 mL) was added to the reaction mixture, and the mixture was stirred. After discarding the aqueous layer, the resulting organic layer was washed with water (20 mL) and then with 5% aqueous NaCl solution (15 mL). The resulting organic layer was concentrated, and azeotropic dehydration with the addition of toluene (20 mL), filtration and concentration was performed to afford a residue (8.83 g) containing compound 2E (15.2 mmol) (90.7% yield from HPLC and qNMR analyses).

UV intensity ratio (as diastereomer mixture): 97.1%

(Detection wavelength 210 nm, retention time 2.993 and 3.048 minutes, HPLC analysis condition method 3)

(9-2): Synthesis of (2S)-2-(benzyloxycarbonylamino)-3-isobutyloxypropanoic acid (compound 3E)

[Formula 45]

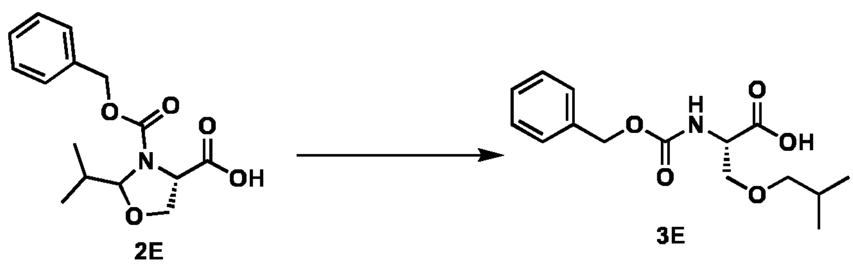

2E 3E

To the residue containing compound 2E obtained in (9-1) above (7.85 g, containing 13.5 mmol of compound 2E), triethylsilane (5.4 mL, 34 mmol) and chlorobenzene (6 mL) were added to prepare a substrate solution.

After the atmosphere in a reaction vessel was replaced with nitrogen, chlorobenzene (10 mL) and titanium tetrachloride (2.76 mL, 25.1 mmol) were added. Then 2,2,2-trifluoroethanol (2.90 mL, 40.6 mmol) and tetrabutyl orthotitanate (2.53 mL, 7.45 mmol) were added to the reaction vessel at the external temperature of -15°C. Subsequently, the substrate solution was added to the mixture in the reaction vessel at the external temperature of -15°C, and then the resulting mixture was stirred at the external temperature of 0°C for 3 hours.

(HPLC analysis using HPLC analysis condition method 1 using a portion of the reaction solution obtained here resulted in an peak area ratio of compound 3E (retention time 3.230 minutes) : compound 4E (retention time 2.755 minutes) of 93.2 : 6.8.)

[Formula 46]

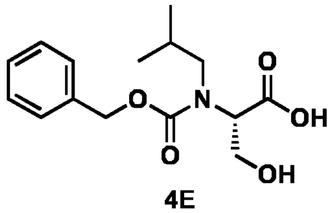

4E

Then a 5% aqueous ammonium chloride solution (20 mL) was added at the external temperature of -10°C. MTBE (12 mL) was added at room temperature and the mixture was stirred. The aqueous layer was discarded, and the resulting organic layer was washed with a 5% aqueous citric acid solution (20 mL) and then with water (20 mL). After the addition of a 1 M aqueous NaOH solution (20 mL) to the organic layer, the mixture was stirred for 3.5 hours. Subsequently, 1 M aqueous NaOH solution (4 mL) was added, and the mixture was stirred for 1.5 hours, and then 1 M aqueous NaOH solution (20 mL) was added, and the resulting mixture was further stirred for 1.5 hours. (HPLC analysis using HPLC analysis condition method 1 using a portion of the aqueous layer obtained here resulted in an area ratio of compound 3E (retention time 3.229 minutes) : compound 4E (retention time 2.751 minutes) of 99.4 : 0.6.)

After discarding the organic layer, the resulting aqueous layer was washed with MTBE (24 mL). To the resulting aqueous layer, 2 M hydrochloric acid (16 mL) and toluene (40 mL) were added and the mixture was stirred, and then the aqueous layer was discharged. The resulting organic layer was washed with 5% aqueous NaCl solution (24 mL), and concentrated under reduced pressure. The resulting residue was filtered and concentrated under reduced pressure to afford a residue (4.44 g) containing compound 3E. To the obtained residue, toluene (9.7 mL) and heptane (19.5 mL) were added, and the seed crystal suspension (about 0.3 mL) of compound 3E obtained in (9-3) below was added at the external temperature of 25°C, and the mixture was stirred for 20 minutes. Heptane (19.5 mL) was then added at the external temperature of 10°C over 1 hour while stirring and then the resulting mixture was stirred for 3.5 hours. The resulting precipitate was filtered under reduced pressure and the obtained solid was dried under reduced pressure to afford 2.76 g of compound 3E (yield 68.1%) as a white solid. Optical purity: 99.6 %ee

(Detection wavelength 210 nm, retention time 14.302 minutes, HPLC analysis condition method 2)
UV intensity ratio: 98.9%
(Detection wavelength 210 nm, retention time 3.209 minutes, HPLC analysis condition method 1)
$^1$H-NMR(DMSO-$d_6$, 500 MHz) δ: 12.77 (brs, 1H), 7.47 (d, J = 8.2 Hz, 1H), 7.38-7.31 (m, 5H), 5.08-5.01 (m, 2H), 4.23-4.19 (m, 1H), 3.61 (d, J = 5.4 Hz, 2H), 3.19-3.11 (m, 2H), 1.91-1.71 (m, 1H), 0.84 (d, J = 6.7 Hz, 6H)

(9-3): Preparation of seed crystal suspension of compound 3E

To a residue (0.1 g) containing compound 3E obtained by the same method as above (9-2), toluene (0.1 mL) and heptane (0.2 mL) were added, and the mixture was stored at an external temperature of -20°C to afford a seed crystal suspension of compound 3E.

Example 9 above showed that compound 3E was obtained in yield of 61% or more from the starting material compound 1 in 2 steps by the present method.

<u>Example 10: Synthesis of methyl (2S)-2-(benzyloxycarbonylamino)-3-isopropoxypropanoate (compound 17B)</u>

(10-1): Synthesis of methyl (4S)-3-benzyloxycarbonyl-2,2-dimethyl-oxazolidine-4-carboxylate (compound 16B)

[Formula 47]

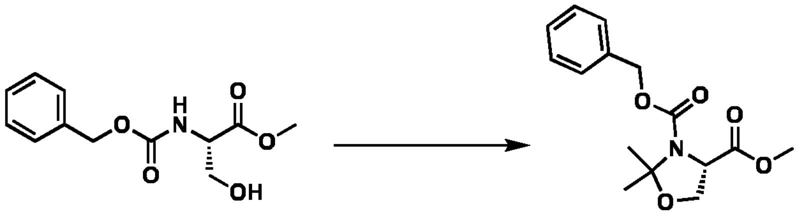

15 16B

N-Benzyloxycarbonyl-L-serine methyl ester (compound 15) (1.22 g, 4.82 mmol), acetone (6 mL), and 2,2-dimethoxypropane (1.12 mL, 9.63 mmol) were added to the reaction vessel. A boron trifluoride tetrahydrofuran complex (0.11 mL, 0.96 mmol) was added to the reaction vessel at room temperature, and the resulting mixture was stirred at room temperature for 1 hour. 2,2-Dimethoxypropane (0.59 mL, 4.8 mmol) was added, and the mixture was further stirred for 1 hour. Subsequently, triethylamine (0.13 mL, 0.96 mmol) was added at room temperature and the mixture was stirred for 5 minutes. The resulting residue was concentrated, and toluene (6 mL) and water (6 mL) were added and the mixture was stirred. The aqueous layer was discharged, and then the resulting organic layer was washed with 5% aqueous NaCl solution (6 mL). Then azeotropic dehydration of the organic layer with the addition of toluene (6 mL) was repeated twice to obtain 1.64 g of residue containing compound 16B (4.15 mmol) (86.1% yield from HPLC and qNMR analyses).

UV intensity ratio: 90.47%

(Detection wavelength 210 nm, retention time 3.234 minutes, HPLC analysis condition method 3)

(10-2): Synthesis of methyl (2S)-2-(benzyloxycarbonylamino)-3-isopropoxypropanoate (compound 17B)

[Formula 48]

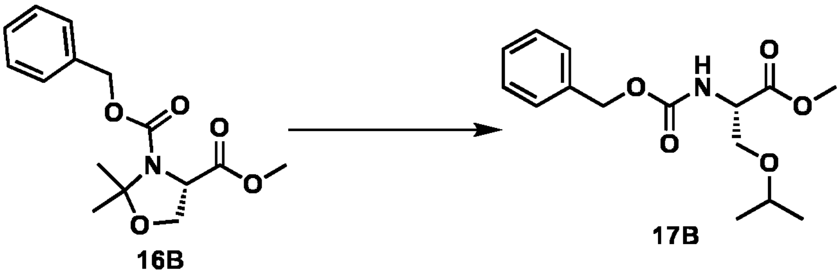

16B 17B

To the residue containing compound 16B obtained in (10-1) above (0.28 g, containing 0.70 mmol of compound 16B), triethylsilane (0.56 mL, 3.5 mmol), acetic acid (0.08 mL, 1.4 mmol), and dichloromethane (1.0 mL) were added to prepare a substrate solution.

After the atmosphere in a reaction vessel was replaced with nitrogen, dichloromethane (1.0 mL), titanium tetrachloride (0.29 mL, 2.6 mmol), and tetraisopropyl orthotitanate (0.26 mL, 0.86 mmol) were added at the external temperature of 0°C. Then, the substrate solution prepared above was added to the mixture in the reaction vessel at the external temperature of 0°C, and the resulting mixture was stirred at room temperature for 90 minutes. (HPLC analysis using HPLC analysis condition method 3 using a portion of the reaction solution obtained here resulted in an peak area ratio of compound 17B (retention time 3.293 minutes) : compound 18B (retention time 2.761 minutes) of 98.8 : 1.2.)

[Formula 49]

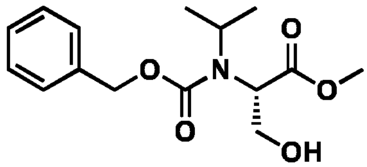

18B

Then a 5% aqueous ammonium chloride solution (1.0 mL) was added at the external temperature of 0°C. The resulting mixture was stirred at room temperature. After discarding the aqueous layer, the resulting organic layer was washed with a 5% aqueous citric acid solution (1.0 mL), with water (1.0 mL), and then with a 5% aqueous dipotassium hydrogen phosphate solution (1.0 mL). The resulting organic layer was dried over magnesium sulfate, filtered, and concentrated under reduced pressure. The residue obtained was purified by reverse-phase column chromatography (eluent: water/acetonitrile) to afford compound 17B (0.151 g, yield 72.9%) as a white solid.

Optical purity: 99.5 %ee
(Detection wavelength 210 nm, retention time 17.720 minutes, HPLC analysis condition method 2)
UV intensity ratio: 99.2%
(Detection wavelength 210 nm, retention time 3.289 minutes, HPLC analysis condition method 3)
$^1$H-NMR(DMSO-$d_6$, 500 MHz) δ: 7.65 (d, J = 7.9 Hz, 1H), 7.39-7.23 (m, 5H), 5.04 (s, 2H), 4.27-4.23 (m, 1H), 3.64-3.50 (m, 6H), 1.06-1.04 (m, 6H)

Example 10 above showed that compound 17B was obtained in yield of 62% or more from the starting material compound 15 in 2 steps by the present method.

<u>Example 11: Synthesis of N-(benzyloxy)carbonyl-O-isopropyl-L-threonyl-L-alanine methyl ester (compound 20B)</u>

(11-1): Synthesis of (4S,5R)-3-benzyloxycarbonyl-2,2,5-trimethyl-oxazolidine-4-carboxylic acid (compound 6B)

[Formula 50]

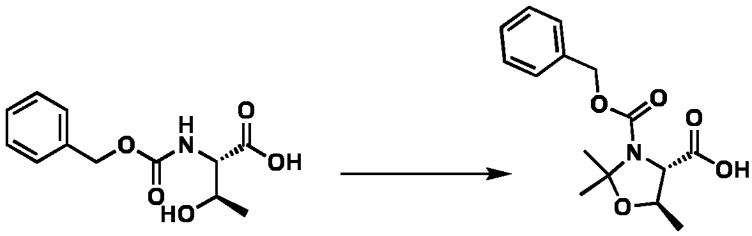

5 6B

N-Benzyloxycarbonyl-L-threonine (compound 5) (4.15 g, 16.4 mmol), acetone (20.8 mL), and 2,2-dimethoxypropane (8.03 mL, 65.5 mmol) were added to the reaction vessel. Boron trifluoride tetrahydrofuran complex (0.36 mL, 3.3 mmol) was added at room temperature, and then the resulting mixture was stirred at room temperature for 1 hour. Subsequently, triethylamine (0.46 mL, 3.3 mmol) was added at room temperature and the mixture was stirred for 5 minutes. The resulting residue was concentrated, and MTBE (20 mL) and water (20 mL) were added and the mixture was stirred. After discarding the aqueous layer, azeotropic dehydration (three times) by the addition of MTBE (20 mL), filtration, and concentration of the organic layer afforded 6.96 g of residue containing compound 6B (96.0% yield from HPLC and qNMR analyses).

UV intensity ratio: 98.82%

(Detection wavelength 210 nm, retention time 2.856 minutes, HPLC analysis condition method 3)

(11-2): Synthesis of Benzyl (4S,5R)-4-{[(S)-1-methoxy-1-oxapropan-2-yl]carbamoyl}-2,2,5-trimethyloxazolidine-3-carboxylate (compound 19B)

[Formula 51]

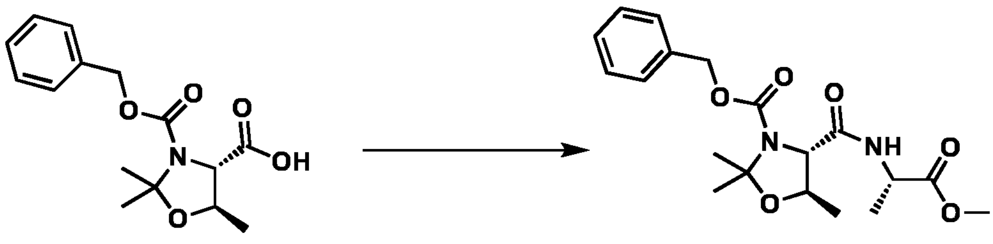

6B 19B

To the residue containing compound 6B obtained in (11-1) above (2.97 g, containing 6.73 mmol of compound 6B), L-alanine methyl ester hydrochloride (0.99 g, 7.0 mmol), MeTHF (8 mL), acetonitrile (4 mL), and N,N-diisopropylethylamine (4.7 mL, 27 mmol) were added. Subsequently, 1-[bis(dimethylamino)methylene]-1H-1,2,3-triazolo[4,5-b]pyridinium 3-oxide hexafluorophosphate (HATU, 3.1 g, 8.1 mmol) was added, and the resulting mixture was stirred at room temperature for 90 minutes. Then, MeTHF (8 mL), a 5% aqueous potassium carbonate solution (4 mL), and 1-methylimidazole (0.54 mL, 6.7 mmol) were added, and the resulting mixture was stirred for 10 minutes. After stirring with the addition of 10% ammonia water (6 mL), the aqueous layer was discarded. The resulting organic layer was sequentially washed with 10% ammonia water (10 mL), with 5% sulfuric acid (10 mL, then 16 mL), and then with 5% aqueous potassium carbonate solution (10 mL). Azeotropic dehydration by the addition of MeTHF (10 mL) was performed twice to afford 3.26 g of residue containing compound 19B (5.87 mmol) (87.2% yield from HPLC and qNMR analyses).

UV intensity ratio: 96.4%

(Detection wavelength 210 nm, retention time 2.899 minutes, HPLC analysis condition method 3)

(11-3): Synthesis of N-(benzyloxy)carbonyl-O-isopropyl-L-threonyl-L-alanine methyl ester (compound 20B)

[Formula 52]

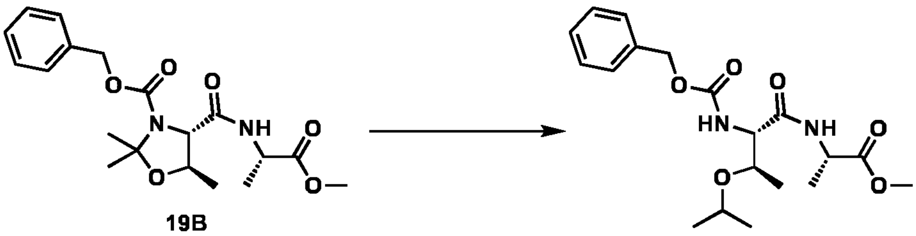

19B 20B

To the residue containing compound 19B obtained in (11-2) above (0.633 g, containing 1.14 mmol of compound 19B), triethylsilane (0.91 mL, 5.7 mmol), acetic acid (0.13 mL, 2.3 mmol), and dichloromethane (2.2 mL) were added to prepare a substrate solution.

After the atmosphere in a reaction vessel was replaced with nitrogen, dichloromethane (2.2 mL), titanium tetrachloride (0.38 mL, 3.4 mmol), and tetraisopropyl orthotitanate (0.33 mL, 1.1 mmol) were added at the external temperature of 0°C. Then, the substrate solution prepared above was added to the reaction vessel at the external temperature of 0°C, and the resulting mixture was stirred at room temperature for 90 minutes. (Compound 21B was not detected by HPLC analysis using HPLC analysis condition method 3 using a portion of the reaction solution obtained here.)

[Formula 53]

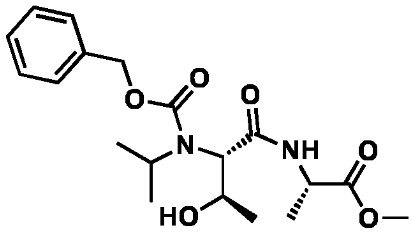

21B

Then a 5% aqueous ammonium chloride solution (2.2 mL) was added at the external temperature of 0°C. The mixture was stirred at room temperature. After discarding the aqueous layer, the resulting organic layer was washed with a 5% aqueous citric acid solution (2.2 mL×2), with water (2.2 mL), and with a 5% aqueous sodium dihydrogen phosphate solution (2.2 mL), successively. The resulting organic layer was dried over magnesium sulfate, filtered, and concentrated under reduced pressure. The obtained residue was purified by reverse-phase column chromatography (eluent: water/-acetonitrile) to afford compound 20B (0.239 g, yield 55.0%) as a white solid.

UV intensity ratio: 98.7%
(Detection wavelength 210 nm, retention time 3.245 minutes, HPLC analysis condition method 3)
$^1$H-NMR(DMSO-$d_6$, 500 MHz) δ: 8.30 (d, J = 6.7 Hz, 1H), 7.37-7.30 (m, 5H), 6.94 (d, J = 9.4 Hz, 1H), 5.08-5.02 (m, 2H), 4.32-4.27 (m, 1H), 4.06-4.01 (m, 1H), 3.75-3.70 (m, 1H), 3.65-3.59 (m, 4H), 1.29-1.21 (m, 3H), 1.07 (d, J = 6.1 Hz, 3H), 1.03 (d, J = 5.9 Hz, 3H), 0.99 (d, J = 6.1 Hz, 3H)

Example 11 above showed that compound 20B was obtained in yield of 46% or more from the starting material compound 5 in 3 steps by the present method.

Example 12: Synthesis of N-[(9H-fluoren-9-ylmethoxycarbonyl)-L-leucyl]-O-isopropyl-L-threonine (compound 23B)

(12-1): Synthesis of N-[(9H-fluoren-9-ylmethoxycarbonyl)-L-leucyl]-O-isopropyl-L-threonine (compound 23B)

[Formula 54]

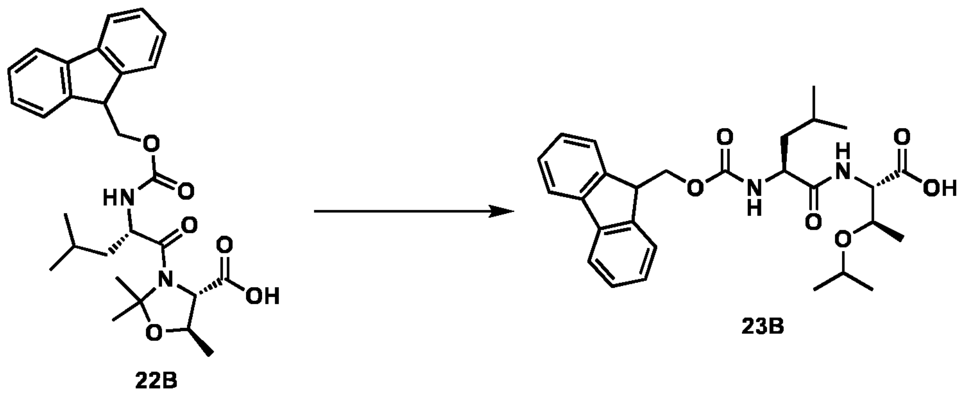

22B

23B

Compound 22B (manufactured by Merck KGaA, catalog number: 8.52184.0001, 197 mg, 0.398 mmol) was dissolved in dichloromethane (0.98 mL) and triethylsilane (0.32 mL, 2.0 mmol) was added to prepare a substrate solution.

After the atmosphere in a reaction vessel was replaced with nitrogen, dichloromethane (0.98 mL), titanium tetrachloride (0.13 mL, 1.2 mmol), and tetrabutyl orthotitanate (0.12 mL, 0.36 mmol) were added at the external temperature of 0°C. Then, the substrate solution prepared above was added to the reaction vessel at the external temperature of 0°C, and the resulting mixture was stirred at room temperature for 60 minutes. (HPLC analysis using HPLC analysis condition method 1 using a portion of the reaction solution obtained here resulted in a UV intensity ratio of target

23B and starting material 22B of 26.1 : 73.9. In addition, as a result of HPLC analysis using HPLC analysis condition method 3, compound 24B was not detected).

[Formula 55]

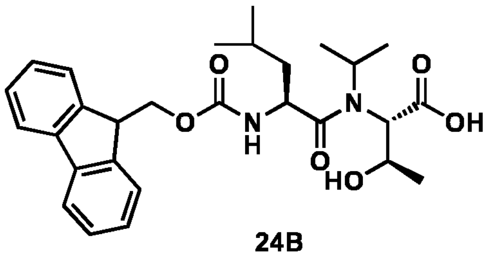

24B

Then a 5% aqueous ammonium chloride solution (1 mL) was added. The mixture was stirred at room temperature. After discarding the aqueous layer, the resulting organic layer was washed with a 5% aqueous ammonium chloride solution (1 mL) and then with water (1 mL). The resulting organic layer was concentrated under reduced pressure, and azeotropic dehydration by the addition of MTBE (5 mL) was repeated four times. The obtained residue was purified by reverse-phase column chromatography (eluent: water/acetonitrile) to afford 29.0 mg of compound 23B (15% yield) and 14.2 mg of target 23B containing starting material 22B (UV intensity ratio of target 23B and starting material 22B in HPLC analysis using HPLC analysis condition method 1 was 93.7: 6.3) as white solids, respectively.

Diastereomeric excess (23B and diastereomer 23B'): 99.9 %de
(Detection wavelength 210 nm, retention time 8.790 minutes, HPLC analysis condition method 6)

[Formula 56]

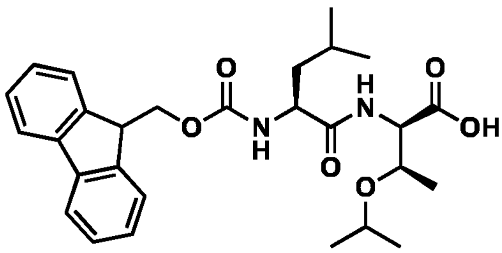

23B'

UV intensity ratio: 99.5%
(Detection wavelength 210 nm, retention time 3.839 minutes, HPLC analysis condition method 1)
$^1$H-NMR (DMSO-$d_6$, 500 MHz) δ: 12.58 (s, 1H), 7.89 (d, J = 7.5 Hz, 2H), 7.71 (t, J = 8.5 Hz, 2H), 7.65 (d, J = 9.2 Hz, 1H), 7.58 (d, J = 9.2 Hz, 1H), 7.41 (dd, J = 7.5, 7.5 Hz, 2H), 7.34-7.30 (m, 2H), 4.31-4.14 (m, 5H), 4.02-3.97 (m, 1H), 3.61-3.56 (m, 1H), 1.64-1.60 (m, 1H), 1.53-1.43 (m, 2H), 1.02 (t, J = 6.5 Hz, 6H), 0.99 (d, J = 6.0 Hz, 3H), 0.89 (d, J = 6.5 Hz, 3H), 0.85 (d, J = 6.5 Hz, 3H)

**Industrial Applicability**

The present invention provides a method for producing an O-substituted serine derivative, in which the O-substituted serine derivative can be obtained in a small number of steps. The production method of the present invention can be used to provide non-natural amino acids useful for the exploration of peptide pharmaceuticals and/or the drug substance supply of pharmaceuticals with high positional selectivity, chemical yield, and optical purity.

**Claims**

1. A method for producing a compound represented by the following formula (1) or a salt thereof or a solvate thereof, the method comprising the step of:

(A) reacting a compound represented by the following formula (2) with a reducing agent to obtain a compound represented by the following formula (1):

[Formula 1]

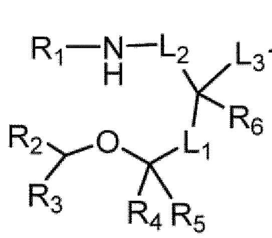

(1)

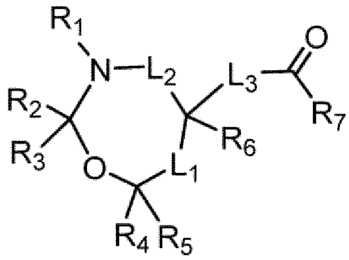

(2)

wherein

$R_1$ is an electron withdrawal group, an amino acid residue, or a peptide residue;
(i) $R_2$ and $R_3$ are each independently selected from the group consisting of hydrogen, $C_1$-$C_6$ alkyl optionally having a substituent, $C_3$-$C_6$ cycloalkyl optionally having a substituent, $C_2$-$C_6$ alkenyl optionally having a substituent, $C_2$-$C_6$ alkynyl optionally having a substituent, aralkyl optionally having a substituent, aryl optionally having a substituent, and heteroaryl optionally having a substituent, or (ii) $R_2$ and $R_3$ together with an intervening carbon atom form a 3- to 8-membered alicyclic ring or a 4- to 7-membered saturated heterocycle;
(i) $R_4$ and $R_5$ are each independently selected from the group consisting of hydrogen, $C_1$-$C_6$ alkyl optionally having a substituent, $C_3$-$C_6$ cycloalkyl optionally having a substituent, $C_2$-$C_6$ alkenyl optionally having a substituent, $C_2$-$C_6$ alkynyl optionally having a substituent, aralkyl optionally having a substituent, aryl optionally having a substituent, and heteroaryl optionally having a substituent, or (ii) $R_4$ and $R_5$ together with an intervening carbon atom form a 3- to 8-membered alicyclic ring or a 4- to 7-membered saturated heterocycle;
$R_6$ is hydrogen, $C_1$-$C_6$ alkyl optionally having a substituent, or aralkyl optionally having a substituent;
$R_7$ is $-OR_8$, $-NR_9R_{9'}$, an amino acid residue, or a peptide residue;
$R_8$, $R_9$ and $R_{9'}$ are each independently hydrogen, $C_1$-$C_6$ alkyl optionally having a substituent, or aralkyl optionally having a substituent, or $R_9$ and $R_{9'}$ together with an intervening nitrogen atom form a 4- to 7-membered saturated heterocycle;
$L_1$ is a single bond or $-CH_2-$;
$L_2$ is a single bond or $-CH_2-$;
$L_3$ is a single bond or $-CH_2-$;
provided that when $L_1$ is $-CH_2-$, $L_2$ is a single bond; when $L_2$ is $-CH_2-$, $L_1$ and $L_3$ each are a single bond; and when $L_3$ is $-CH_2-$, $L_2$ is single bond.

**2.** The method according to claim 1, wherein the step (A) is performed in the presence of an acid.

**3.** The method according to claim 2, wherein the acid is a metallic Lewis acid.

**4.** The method according to claim 3, wherein the metallic Lewis acid is at least one selected from the group consisting of titanium tetrachloride, tin tetrachloride, scandium triflate, tetraisopropyl orthotitanate, and isopropoxytrichlorotitanium.

**5.** The method according to any one of claims 1 to 4, wherein the reducing agent used in the step (A) is a hydride-based reducing agent.

**6.** The method according to any one of claims 2 to 5, wherein an amount of the acid used in the step (A) is 0.1 equivalents or more and 20 equivalents or less relative to the compound represented by formula (2), and an amount of the reducing agent used is 0.5 equivalents or more and 30 equivalents or less relative to the compound represented by formula (2).

**7.** The method according to any one of claims 1 to 6, wherein the step (A) is performed in the presence of a solvent, and

the solvent is at least one selected from the group consisting of a halogenated solvent and a benzene-based solvent.

**8.** The method according to any one of claims 1 to 7, wherein the step (A) is performed further in the presence of a solubilizing agent.

**9.** The method according to any one of claims 1 to 8, wherein the step (A) is performed at a temperature of -50°C to 50°C for 5 minutes to 24 hours.

**10.** The method according to any one of claims 1 to 9, comprising, after the step (A), a base treatment step of further adding a base to reduce a content of a compound (4) contained in a reaction mixture:

[Formula 2]

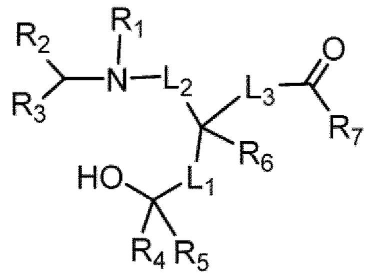

(4)

wherein $R_1$, $R_2$, $R_3$, $R_4$, $R_5$, $R_6$, $R_7$, $L_1$, $L_2$, and $L_3$ are as defined in claim 1.

**11.** The method according to any one of claims 1 to 10, further comprising, prior to the step (A), the step of:
(B) reacting a compound represented by the following formula (3) with an aldehyde, a ketone, an acetal, or a vinyl ether to obtain a compound represented by the following formula (2):

[Formula 3]

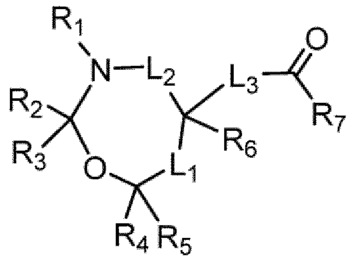

(2)

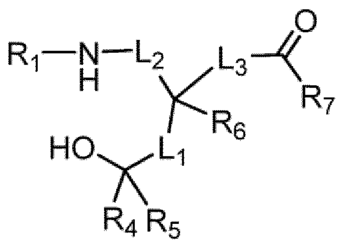

(3)

wherein $R_1$, $R_2$, $R_3$, $R_4$, $R_5$, $R_6$, $R_7$, $L_1$, $L_2$, and $L_3$ are as defined in claim 1.

**12.** A method for producing a compound represented by the following formula (16) or a salt thereof or a solvate thereof, the method comprising the step of:

producing, by the method according to any one of claims 1 to 11, the following compound (11):

[Formula 4]

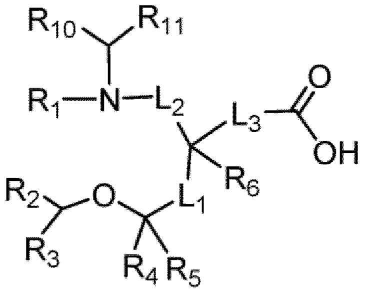

(16)

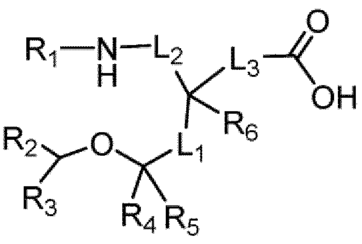

(11)

wherein $R_1$, $R_2$, $R_3$, $R_4$, $R_5$, $R_6$, $L_1$, $L_2$, and $L_3$ have the same meanings as those defined in claim 1; and (i) $R_{10}$ and $R_{11}$ are each independently selected from the group consisting of hydrogen, $C_1$-$C_6$ alkyl optionally having a substituent, $C_3$-$C_6$ cycloalkyl optionally having a substituent, $C_2$-$C_6$ alkenyl optionally having a substituent, $C_2$-$C_6$ alkynyl optionally having a substituent, aralkyl optionally having a substituent, aryl optionally having a substituent, and heteroaryl optionally having a substituent, or (ii) $R_{10}$ and $R_{11}$ together with an intervening carbon atom form a 3- to 8-membered alicyclic ring or a 4- to 7-membered saturated heterocycle.

**13.** The method according to claim 12, further comprising, after the step of producing the following compound (11), the steps of:

(C) reacting the compound represented by the following formula (11) with an aldehyde, a ketone, an acetal, or a vinyl ether in the presence of a Lewis acid to obtain a compound represented by the following formula (15); and
(D) reacting the compound represented by the following formula (15) in the presence of a Lewis acid and a reducing agent to obtain a compound represented by the following formula (16),

[Formula 5]

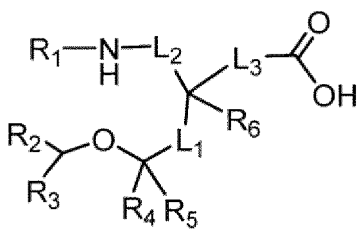

(11)

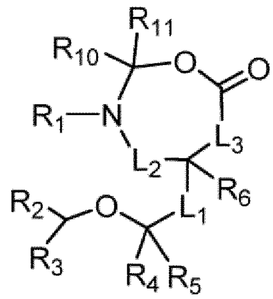

(15)

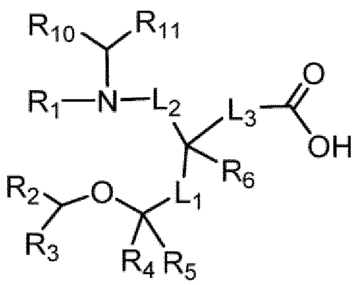

(16)

wherein $R_1$, $R_2$, $R_3$, $R_4$, $R_5$, $R_6$, $R_{10}$, $R_{11}$, $L_1$, $L_2$, and $L_3$ are as defined in claims 1 and 12.

**14.** The method according to any one of claims 1 to 13, wherein
$R_1$ is -C(=O)-$R_{12}$, -C(=O)-O$R_{13}$, -S(=O)$_2$-$R_{14}$, -S(=O)$_2$-O$R_{15}$, -P(=O)-$R_{16}R_{17}$, or -P(=O)-(O$R_{18}$)$_2$, wherein $R_{12}$, $R_{13}$, $R_{14}$, $R_{15}$, $R_{16}$, $R_{17}$, and $R_{18}$ each are $C_1$-$C_6$ alkyl optionally having a substituent, aralkyl optionally having a substituent, aryl optionally having a substituent, or heteroaryl optionally having a substituent;

$R_2$ and $R_3$ are each independently selected from the group consisting of hydrogen, methyl, ethyl, n-propyl, isopropyl, n-butyl, tert-butyl, isobutyl, isopentyl, benzyl, phenyl, and pyridyl, or $R_2$ and $R_3$ together with a carbon atom to which they are attached form a 3- to 8-membered alicyclic ring;
$R_4$ and $R_5$ are each independently selected from the group consisting of hydrogen, methyl, ethyl, n-propyl, n-butyl, tert-butyl, isobutyl, isopentyl, benzyl, phenyl, and pyridyl, or $R_4$ and $R_5$ together with a carbon atom to which they are attached form a 3- to 8-membered alicyclic ring;
$R_6$ is hydrogen, methyl, ethyl, n-propyl, n-butyl, or benzyl; and
$L_1$, $L_2$, and $L_3$ each are a single bond.

**15.** A method for producing a peptide compound, the method comprising the steps of:

(i) producing, by the method according to any one of claims 1 to 14, the compound (11) or (16) above; and
(ii) condensing a carboxy group of the compound (11) or (16) obtained in the step (i) above with an amino acid having an amino group or a peptide having an amino group in a solvent.

**16.** A compound selected from the group consisting of:

2-(benzyloxycarbonylamino)-3-(cyclobutoxy)propanoic acid;
2-(benzyloxycarbonylamino)-3-isopentyloxy-propanoic acid;
2-(benzyloxycarbonylamino)-3-isopropoxy-butanoic acid;
2-[benzyloxycarbonyl(methyl)amino]-3-(cyclobutoxy)propanoic acid;
3-benzyloxycarbonyl-2-ethyl-oxazolidine-4-carboxylic acid;
3-benzyloxycarbonyl-2-isobutyl-oxazolidine-4-carboxylic acid;
benzyl 4-(cyclobutoxymethyl)-5-oxo-oxazolidine-3-carboxylate;
benzyl 4-{[1-methoxy-1-oxapropan-2-yl]carbamoyl}-2,2,5-trimethyloxazolidine-3-carboxylate;
N-(benzyloxy)carbonyl-O-isopropyl-threonyl-alanine methyl ester; and
N-[(9H-fluoren-9-ylmethoxycarbonyl)-leucyl]-O-isopropyl-threonine;
or a salt thereof or a solvate thereof.

## INTERNATIONAL SEARCH REPORT

International application No. **PCT/JP2023/027836**

**A. CLASSIFICATION OF SUBJECT MATTER**

***C07C 269/06***(2006.01)i; ***C07C 271/22***(2006.01)i; ***C07D 263/04***(2006.01)i; ***C07D 263/06***(2006.01)i; ***C07D 263/16***(2006.01)i
FI: C07C269/06; C07C271/22 CSP; C07D263/06; C07D263/04; C07D263/16

According to International Patent Classification (IPC) or to both national classification and IPC

**B. FIELDS SEARCHED**

Minimum documentation searched (classification system followed by classification symbols)

C07C269/06; C07C271/22; C07D263/04; C07D263/06; C07D263/16

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2021/090855 A1 (CHUGAI SEIYAKU KABUSHIKI KAISHA) 14 May 2021 (2021-05-14)<br>claims (in particular, claim 16), paragraphs [0275], [0306]-[0358], [0429], [0470]-[0475] | 16<br>1-15 |
| X<br>A | WO 2020/095983 A1 (CHUGAI SEIYAKU KABUSHIKI KAISHA) 14 May 2020 (2020-05-14)<br>claims, paragraphs [0162], [0208] | 16<br>1-15 |
| X<br>A | JP 57-159747 A (OKAWA, Kenji) 01 October 1982 (1982-10-01)<br>claims, p. 2, upper left column, example 9(1) | 16<br>1-15 |
| A | WO 2010/053050 A1 (KANEKA CORP) 14 May 2010 (2010-05-14)<br>claims, examples | 1-16 |
| P, X | WO 2023/023631 A1 (PARDES BIOSCIENCES, INC.) 23 February 2023 (2023-02-23)<br>p. 276 (example 50) | 16 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 September 2023** | **10 October 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/027836**

| Patent document cited in search report | Publication date (day/month/year) | Patent family member(s) | Publication date (day/month/year) |
|---|---|---|---|
| WO 2021/090855 A1 | 14 May 2021 | JP 2021-119176 A<br>US 2023/0151060 A1<br>claims, paragraph [0298], pp. 260, 269<br>EP 4043478 A1<br>KR 10-2021-0064234 A<br>KR 10-2022-0081327 A<br>CN 114729006 A | |
| WO 2020/095983 A1 | 14 May 2020 | US 2022/0017456 A1<br>claims, examples 2, 3<br>EP 3878836 A1<br>CN 112969683 A<br>KR 10-2021-0088619 A | |
| JP 57-159747 A | 01 October 1982 | (Family: none) | |
| WO 2010/053050 A1 | 14 May 2010 | (Family: none) | |
| WO 2023/023631 A1 | 23 February 2023 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 57159747 A
- WO 2010053050 A
- WO 2020095983 A
- WO 2021132545 A
- WO 2013100132 A

### Non-patent literature cited in the description


- *Future Med. Chem.*, 2009, vol. 1, 1289-1310
- *J. Med. Chem.*, 2010, vol. 53 (15), 5716-5726
- *Tetrahedron Letters*, 2012, vol. 53, 3225-3229
- *ioorganic & Medicinal Chemistry Letters*, 1992, vol. 2, 579-582
- *Journal of the American Chemical Society*, 2014, vol. 136, 12469-12478
- **FREIDINGER et al.** *J. Org. Chem.*, 1983, vol. 48 (1), 77-81